(19) Europäisches Patentamt
European Patent Office
Office européen des brevets

(11) **EP 4 310 848 A1**

(12) **EUROPEAN PATENT APPLICATION**

(43) Date of publication:
**24.01.2024 Bulletin 2024/04**

(21) Application number: **22290048.2**

(22) Date of filing: **21.07.2022**

(51) International Patent Classification (IPC):
**G16B 25/10** (2019.01)     **G16B 35/20** (2019.01)
**G16B 40/30** (2019.01)

(52) Cooperative Patent Classification (CPC):
**G16B 25/10; G16B 35/20; G16B 40/30**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Sartorius Stedim Data Analytics AB**
**903 33 Umeå (SE)**
• **Deutsthes Forachungszentrur Für Künstliche
Intelligenz GmbH (DFKI)**
**67663 Kaiserslautern (DE)**

(72) Inventors:
• **Asim, Muhammad Nabeel**
**67663 Kaiserslautern (DE)**
• **Ahmed, Sheraz**
**67663 Kaiserslautern (DE)**
• **Zehe, Christoph**
**89081 Ulm (DE)**
• **Trygg, Johan**
**903 33 Urneà (SE)**
• **Cloarec, Olivier**
**13781 AUBAGNE CEDEX (FR)**

(74) Representative: **Müller-Boré & Partner
Patentanwälte PartG mbB
Friedenheimer Brücke 21
80639 München (DE)**

(54) **METHOD, COMPUTER PROGRAM PRODUCT AND SYSTEM FOR OPTIMIZING PROTEIN EXPRESSION**

(57)     A method is provided for optimizing protein expression. The method comprises: obtaining, by a processor (102), a plurality of amino acid sequences and corresponding known efficiency values, each known efficiency value indicating efficiency of expressing a protein having a corresponding one of the plurality of amino acid sequences; for each one of a plurality of prediction algorithms, obtaining, by the processor (102), a prediction function according to the one of the plurality of prediction algorithms, wherein the prediction function outputs a predicted efficiency value for expressing a protein having an amino acid sequence corresponding to an input numerical vector; evaluating, by the processor (102), the obtained prediction function by comparing predicted efficiency values output by the obtained prediction function with the known efficiency values; selecting, by the processor (102), at least one prediction algorithm from among the plurality of prediction algorithms based on said evaluating; predicting, by the processor (102), using the at least one prediction algorithm and the prediction function obtained with the at least one prediction algorithm, one or more efficiency values for expressing one or more proteins respectively having one or more specified amino acid sequences; and outputting, by the processor (102), the one or more specified amino acid sequences and the one or more efficiency values predicted for the one or more specified amino acid sequences.

FIG 4

**Description**

The application relates to a computer-implemented method, a computer program product and a system for optimizing protein expression.

**BACKGROUND**

Protein expression analysis is usually performed through wet lab experiments under controlled environments. For example, with the influx of high-throughput sequencing and genetic engineering technologies, analyses of recombinant antibody sequence parameters and their impact on protein expression may be performed using experimental approaches. Major bottlenecks in the wide adoption of such experimental approaches may include high cost, time, and/or need of controlled environments.

**SUMMARY**

According to an aspect, the problem relates to optimizing protein expression with improved efficiency. The problem is solved by the features disclosed by the independent claims. Further exemplary embodiments are defined by the dependent claims.

According to an aspect, a method for optimizing protein expression is provided. In the method, each one of a plurality of prediction algorithms includes a combination of an encoding algorithm, a dimensionality reduction algorithm and a regression algorithm, the combination being different from a combination included in any other one of the plurality of prediction algorithms. Further, the method comprises:

obtaining, by a processor, a plurality of amino acid sequences and corresponding known efficiency values, each known efficiency value indicating efficiency of expressing a protein having a corresponding one of the plurality of amino acid sequences;

for each one of the plurality of prediction algorithms,

for each one of a plurality of amino acid sequences, generating, by the processor, a first numerical vector corresponding to the one of the plurality of amino acid sequences by encoding at least part of the one of the plurality of amino acid sequences according to the encoding algorithm included in the one of the plurality of prediction algorithms,

generating, by the processor, second numerical vectors corresponding to the plurality of amino acid sequences by applying the dimensionality reduction algorithm included in the one of the plurality of prediction algorithms to the first numerical vectors corresponding to the plurality of amino acid sequences, wherein a dimension of the second numerical vectors is smaller than a dimension of the first numerical vectors,

obtaining, by the processor, a prediction function by processing, according to the regression algorithm included in the one of the plurality of prediction algorithms, at least part of the second numerical vectors and the known efficiency values for the amino acid sequences corresponding to the at least part of the second numerical vectors, wherein the prediction function outputs a predicted efficiency value for expressing a protein having an amino acid sequence corresponding to an input numerical vector, and

evaluating, by the processor, the obtained prediction function by comparing predicted efficiency values output by the obtained prediction function for at least part of the second numerical vectors with the known efficiency values for the amino acid sequences corresponding to the at least part of the second numerical vectors;

selecting, by the processor, at least one prediction algorithm from among the plurality of prediction algorithms based on said evaluating;

predicting, by the processor, using the at least one prediction algorithm and the prediction function obtained with the at least one prediction algorithm, one or more efficiency values for expressing one or more proteins respectively having one or more specified amino acid sequences; and

outputting, by the processor, the one or more specified amino acid sequences and the one or more efficiency values predicted for the one or more specified amino acid sequences.

In various aspects and embodiments described herein, an "amino acid sequence" preferably includes a sequence of amino acids in the one-letter notation. In some exemplary embodiments, however, an "amino acid sequence" may include a sequence of amino acids in the three-letter notation.

In the present disclosure, the "efficiency" of expressing a protein may indicate how well a protein having a particular amino acid sequence is expressed. Further, in the present disclosure, the "efficiency" of expressing a protein may also be referred to as "performance" of the protein expression.

In various aspects and embodiments described herein, a "known efficiency value" corresponding to "one of the plurality of amino acid sequences" may be determined by expressing a protein having the one of the plurality of amino acid sequences in wet lab experiments using a particular type of host cells and a particular type of expression vector. For example, the "known efficiency value" may be an average efficiency of protein expression of the protein having the corresponding amino acid sequence.

In various aspects and embodiments described herein, the "encoding algorithm" may be an algorithm for encoding amino acid sequences into a sequence of numerical values. The sequence of numerical values may be considered as a numerical vector (e.g., the "first numerical vector"). A dimension of the numerical vector may be reduced according to the "dimensionality reduction algorithm" (e.g., resulting in the "second numerical vector"). The "regression algorithm" may be an algorithm to estimate the relationships between the "second numerical vectors" (as independent variables) and an efficiency value for a protein expression (as a dependent variable).

In various aspects and embodiments described herein, different prediction algorithms include different combinations of the encoding algorithm, the dimensionality reduction algorithm and the regression algorithm. For example, a plurality of encoding algorithms, a plurality of dimensionality reduction algorithms and a plurality of regression algorithms may be available and a combination of algorithms included in a prediction algorithm may be determined by selecting one of the plurality of encoding algorithms, one of the plurality of dimensionality reduction algorithms and one of the plurality of regression algorithms. In some exemplary embodiments, the plurality of prediction algorithms may comprise all possible combinations of algorithms obtained from the available encoding, dimensionality reduction and regression algorithms.

Further, in various aspects and embodiments described herein, at least some of the "one or more specified amino acid sequences" may be obtained from a user via an input device connected to the processor. Additionally or alternatively, the "one or more specified amino acid sequences" may be obtained from a computer connected (e.g., via a network) to the processor.

In various aspects and embodiments described herein, the one or more efficiency values predicted for expressing one or more proteins respectively having one or more specified amino acid sequences may indicate predicted efficiency value(s) for expressing the respective proteins using the particular type of host cells and the particular type of expression vector used for obtaining the known efficiency values.

Further, in various aspects and embodiments described herein, the "outputting" of the "one or more specified amino acid sequences" and the "one or more efficiency values predicted for the one or more specified amino acid sequences" may cause a display device connected to the processor to display information indicating the "one or more specified amino acid sequences" and the "one or more efficiency values predicted for the one or more specified amino acid sequences". Additionally or alternatively, the "outputting" may include transmitting the information indicating the "one or more specified amino acid sequences" and the "one or more efficiency values predicted for the one or more specified amino acid sequences" to a computer connected (e.g., via a network) to the processor.

Such a computer receiving the information from the processor may be, for example, comprised in an automated cell culture system that is configured to perform protein expression. The computer comprised in the automated cell culture system may, for example, display the received information so that proteins respectively having the one or more specified amino acid sequences may be expressed in the automated cell culture system. Further, in some circumstances, the computer comprised in the automated cell culture system may control the automated cell culture system to provide controlled environment (e.g., temperature, humidity, agitation etc.) appropriate for expressing the proteins respectively having the one or more specified amino acid sequences.

The method according to the above-stated aspect or any one of various embodiments thereof can facilitate protein expression with improved efficiency.

The method according to the above-stated aspect may further comprise:

identifying, by the processor, one of the one or more specified amino acid sequences for which a highest efficiency value is predicted; and
outputting, by the processor, information indicating that the identified one of the one or more specified amino acid sequences has the highest predicted efficiency value.

In various aspects and embodiments described herein, the plurality of amino acid sequences may be antibody sequences or recombinant antibody sequences.

Recombinant antibodies may be generated, for example, using synthetic genes. Different biotechnological approaches may be used to express antibody sequences in form of proteins, tweak different fragments to enhance affinity, spatial orientation, stability of antigen-binding sites, modify immunogenicity, analyze sequence parameters, expression and suppression mechanisms, and many other parameters. Considering the therapeutic potential of recombinant antibodies, pharmaceutical industries are investing trillions to generate accurate recombinant antibody sequences and perform an in-depth fault-less analysis of recombinant antibodies. Accordingly, any one of embodiments where the plurality of amino acid sequences are recombinant antibody sequences, can largely reduce the humongous recombinant antibody sequence analysis cost incurred by pharmaceutical industries without losing the promise of accurate findings. Further, any one of embodiments where the plurality of amino acid sequences are recombinant antibody sequences can be adopted by a large number of pharmaceutical industries to accurately infer the expression of novel recombinant antibodies, for example.

In the embodiments where the plurality of amino acid sequences are antibody sequences or recombinant antibody sequences, the plurality of amino acid sequences may be light chain sequences, heavy chain sequences or light-heavy chain sequences of antibodies or recombinant antibodies.

In some circumstances, for example, different prediction functions with different evaluation results may be obtained for different types of sequences being used as the plurality of amino acid sequences, even when the prediction functions are obtained according to the same prediction algorithm. Accordingly, the use of the light chain sequences, the heavy chain sequences and/or the light-heavy chain sequences may enable quantifying performance potential of full length sequences of each type of sequence and/or major bottlenecks at the level of amino acid distribution which may be hindering the prediction function to provide accurate predictions of efficiency.

Further, in various aspects and embodiments described herein, the at least part of the one of the plurality of amino acid sequences may include:

> a first x % of the one of the plurality of amino acid sequences; and/or
> a last y % of the one of the plurality of amino acid sequences,
> wherein each of x and y is a value greater than 0 and less than or equal to 50,
> wherein x is preferably 25 or 50 and y is preferably 25 or 50.

Using a sub-sequence, e.g., the first x % and/or the last y % of the one of the plurality of amino acid sequences, to be encoded for generating the first numerical vector may allow identification of the most discriminative distribution of amino acids by retaining variable and/or informative regions and discarding constant regions which remain similar across different sequences.

Further, for example, using different sub-sequences (e.g., the first x % and/or the last y %) of different types of sequences (e.g., light chain, heavy chain and/or light-heavy chain) to be encoded for generating the first numerical vectors may facilitate investigation on which sequence type and kind of sub-sequences can lead to a prediction function that provide more accurate prediction of efficiency.

In various aspects and embodiments described herein, each known efficiency value may indicate a titer for expressing the protein having the corresponding one of the plurality of amino acid sequences and the predicted efficiency value output by the prediction function may indicate a predicted titer. Alternatively or additionally, cell specific productivity (expressed in e.g., pg/cell/day) may be used as the known efficiency value and predicted cell specific productivity may be output by the prediction function as the predicted efficiency value. Further, in some exemplary embodiments, the known efficiency value and the predicted efficiency value may relate to product quality attributes such as aggregation, folding, glycosylation, etc.

In various aspects and embodiments described herein, said generating of the second numerical vectors may comprise:

> generating, by the processor, a plurality of sets of the second numerical vectors using the dimensionality reduction algorithm,
> wherein the second numerical vectors included in a same set of the plurality of sets of the second numerical vectors have a same dimension,
> wherein the second numerical vectors included in different sets of the plurality of sets of the second numerical vectors have different dimensions, and
> wherein said obtaining the prediction function and said evaluating the prediction function are performed for each one of the plurality of sets of the second numerical vectors.

The use of the plurality of sets of the second numerical vectors as stated above may facilitate determination of the dimension of the second numerical vectors leading to a more accurate prediction function for predicting efficiency of protein expression.

In various aspects and embodiments described herein, said evaluating of the obtained prediction function may comprise:

determining, by the processor, one or more of the following performance metrics for the obtained prediction function:

> accuracy,

precision,
recall,
F1-score.

The one or more performance metrics as stated above may indicate how well the prediction function can predict efficiency values of input amino acid sequences.

Further, in various aspects and embodiments described herein, the combination of the encoding algorithm, the dimensionality reduction algorithm and the regression algorithm may be a combination of the following:

as the encoding algorithm, a k-mer based encoding algorithm, a counting based encoding algorithm, a K-gap based encoding algorithm, a window-based encoding algorithm, a group-based encoding algorithm, a physico-chemical property based encoding algorithm or a word embedding based encoding algorithm;
as the dimensionality reduction algorithm, principal component analysis, PCA, K-means, t-distributed stochastic neighbor embedding, TSNE, kernel-PCA, locally-linear embedding, LLE, tensor singular value decomposition, T-SVD, non-negative matrix factorization, NMF, multi-dimensional scaling, MDS, factor analysis, agglomerate feature, Gaussian random projection, sparse random projection or fast independent component analysis, fast-ICA; and
as the regression algorithm, linear regression, non-linear regression, penalized linear regression, penalized non-linear regression, naive Bayes, bagging regression, random forest regressor, boosting regression, partial least square regression or support vector machine.

In the present disclosure, "k-mer" may be understood as a substring of length k contained within a biological sequence, e.g. an amino acid sequence.

In various aspects and embodiments described herein, said encoding of the at least part of the one of the plurality of amino acid sequences according to the physico-chemical property based algorithm as the encoding algorithm may comprise:

assigning, by the processor, weight values to individual amino acids based on one or more physico-chemical properties of proteins; and
generating, by the processor, the first numerical vector including the weights corresponding to the amino acids included in the at least part of the one of the plurality of amino acid sequences in an order in the at least part of the one or the plurality of amino acid sequences,
wherein the one or more physicochemical properties include one or more of the following:

dissociation,
solubility,
hydration,
polarity,
charge,
hydrophobicity,
molecular weight,
size.

In various aspects and embodiments described herein, said encoding of the at least part of the one of the plurality of amino acid sequences according to the k-mer based encoding algorithm as the encoding algorithm may comprise:

determining, by the processor, k-mers of the at least part of the one of the plurality of amino acid sequences;
determining, by the processor, a weight for each one of the k-mers based at least on:

how many times the one of the k-mers appears in the at least part of the one of the plurality of amino acid sequences, and
how many times the one of the k-mers appears in the at least part of the plurality of amino acid sequences;

generating, by the processor, the first numerical vector including the weights of the k-mers in an order of the k-mers appearing in the at least part of the one of the plurality of amino acid sequences.

A specific, but non-limiting, example of the k-mer based encoding algorithm involves Okapi-BM25 that is a ranking function used in information retrieval and that can provide a score indicating relevance of a document against a certain query based on frequencies of terms included in the query appearing in the document.

Further, the method according to any one of the above-stated aspect and various embodiments thereof may further comprise:

using, in an automated cell culture system, one or more nucleic acids that respectively encode the one or more specified amino acid sequences to express one or more proteins respectively including the one or more specified amino acid sequences.

When using the one or more nucleic acids that respectively encode the one or more specified amino acid sequences in the automated cell culture system, the particular type of host cells and the particular type of expression vector used for obtaining the known efficiency values may be used.

In the present disclosure, the "automated cell culture system" may be understood as a system that is configured to and/or capable of expressing one or more proteins.

According to another aspect, a computer program product is provided. The computer program product comprises computer-readable instructions that, when loaded and run on a computer, cause the computer to perform the method according to any one of the above-stated aspect and various embodiments thereof.

According to yet another aspect, a prediction system is provided for predicting efficiency of protein expression. The prediction system comprises:

a processor configured to perform the method according to any one of the above-stated aspect and various embodiments thereof; and

a storage medium that is in communication with the processor and that is configured to store the plurality of amino acid sequences and the corresponding known efficiency values.

According to yet another aspect, a system is provided for optimizing protein expression. The system comprises:

the prediction system according to the above-stated aspect; and
an automated cell culture system that is capable of expressing the one or more proteins.

According to any one of various aspects and embodiments described herein, inference of protein expression can be made by developing a computational framework that evaluates the effectiveness of a wide range of inherent relationships of sequence residues and machine learning regressions, without extensive wet lab experiments. In other words, wet lab experiments can be replaced with computation using artificial intelligence for protein expression analysis.

Developing a computational framework capable of accurately infer expression of a protein according to any one of various aspects and embodiments described herein can facilitate expressing a target protein. For example, different amino acid sequences of a same protein class (e.g., antibodies) may show different efficiencies in the resulting expression. Thus, obtaining a prediction function that can provide accurate efficiency prediction based on an input amino acid sequence as in any one of various aspects and embodiments described herein may lead to better understanding of how a sequence should be adapted to improve the efficiency of the target protein expression, which can facilitate the protein expression.

Further, the computational framework provided by any one of various aspects and embodiments described herein may be with lower cost, more adaptable, scalable and/or robust as compared to wet lab experimental approaches for analysis of amino acid sequences and inferring their expression.

The subject matter described in the application can be implemented as a method or as a system, possibly in the form of one or more computer program products. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. In some examples, the system may be a general purpose computer system. In other examples, the system may be a special purpose computer system including an embedded system.

## BRIEF DESCRIPTION OF THE DRAWINGS

Details of one or more implementations are set forth in the exemplary drawings and description below. Other features will be apparent from the description, the drawings, and from the claims. It should be understood, however, that even though embodiments are separately described, single features of different embodiments may be combined to further embodiments.

**Fig. 1** shows an exemplary development process for the generation of protein expressing cell lines.

**Fig. 2** shows examples of product titers obtained by exemplary protein expression for a same class of proteins (e.g. antibodies) with different amino acid sequences.

**Fig. 3** shows a functional block diagram of an exemplary system according to the present disclosure.

**Fig. 4** shows a flowchart of an exemplary process performed by an exemplary prediction system according to the present disclosure.

**Fig. 5** shows an exemplary workflow for estimating and evaluating prediction functions according to a plurality of prediction algorithms.

**Fig. 6** shows examples of different types of sub-sequences of antibodies or recombinant antibodies.

**Fig. 7A** shows an exemplary encoding algorithm that may be used in the present disclosure.

**Fig. 7B** shows another exemplary encoding algorithm that may be used in the present disclosure.

**Fig. 8** shows a specific example of encoding an amino acid sequence to generate a first numerical vector.

**Fig. 9** shows an example of a graph generated in an exemplary word embedding based encoding algorithm.

**Fig. 10** shows a specific example of dimensionality reduction of a first numerical vector to generate a second numerical vector.

**Fig. 11** shows a specific example of estimating a prediction function according to a regression algorithm.

**Fig. 12** shows a specific example of classifying outputs of the prediction function for evaluating the prediction function.

**Fig. 13** shows an exemplary hardware configuration of a computer that may be used to implement at least a part of a system according to the present disclosure.

## DETAILED DESCRIPTION OF EMBODIMENTS

In the following text, a detailed description of examples will be given with reference to the drawings. It should be understood that various modifications to the examples may be made. In particular, one or more elements of one example may be combined and used in other examples to form new examples.

## Protein Expression

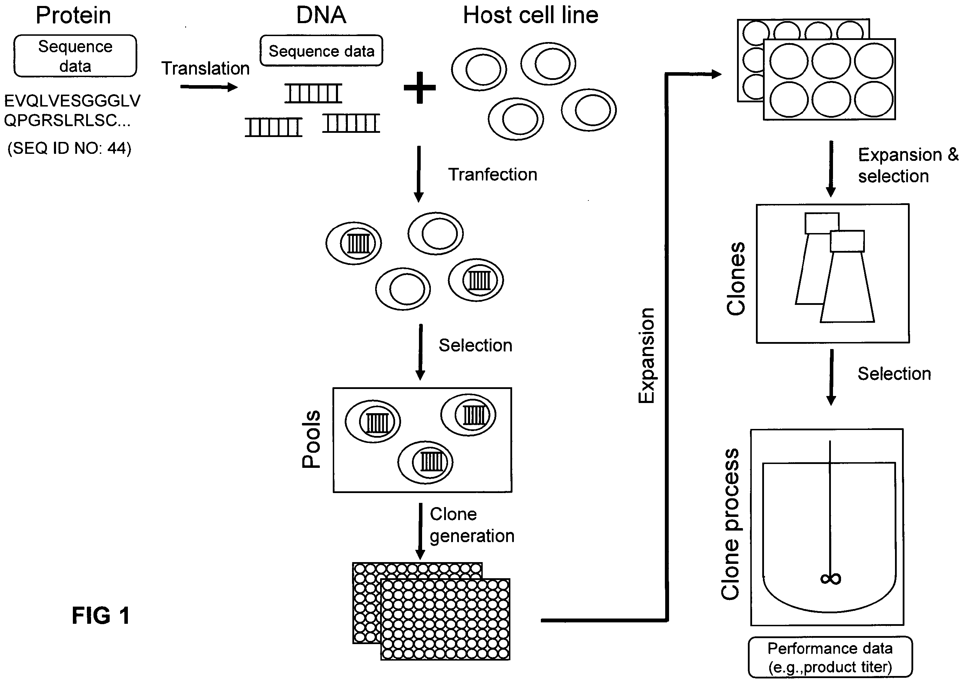

**Fig. 1** shows an exemplary development process for generation of protein expressing cell lines. As shown in Fig. 1, a target protein may be represented by sequence data including an amino acid sequence of the protein. The protein sequence data, e.g., the amino acid sequence, may be reverse-translated into DNA sequence data. Once the DNA sequence data is obtained, transfection of the DNA sequence into a host cell line may be performed. After the transfection, the cells may be subjected to a selection process where pools of cells carrying the DNA sequence may be obtained. Clone generation may then be performed using the pools and expansion of the clones may be made. Clones of the cells may be subjected to yet further selection process. A titer of the target protein may be measured, for example, in the end of the protein expression process. Additionally or alternatively, the titer of the target protein may be measured when the pools of cells are obtained, during the expansion and selection and/or after the expansion and selection before the further selection process. The titer, also referred to as "product titer", may be understood as indicating efficiency or performance of the protein expression.

Details of at least a part of the exemplary process shown in Fig. 1 can be found in Lai et al., "Advances in Mammalian Cell Line Development Technologies for Recombinant Protein Production", Pharmaceuticals 2013, 6(5), p. 579-603, doi: 10.3390/ph6050579.

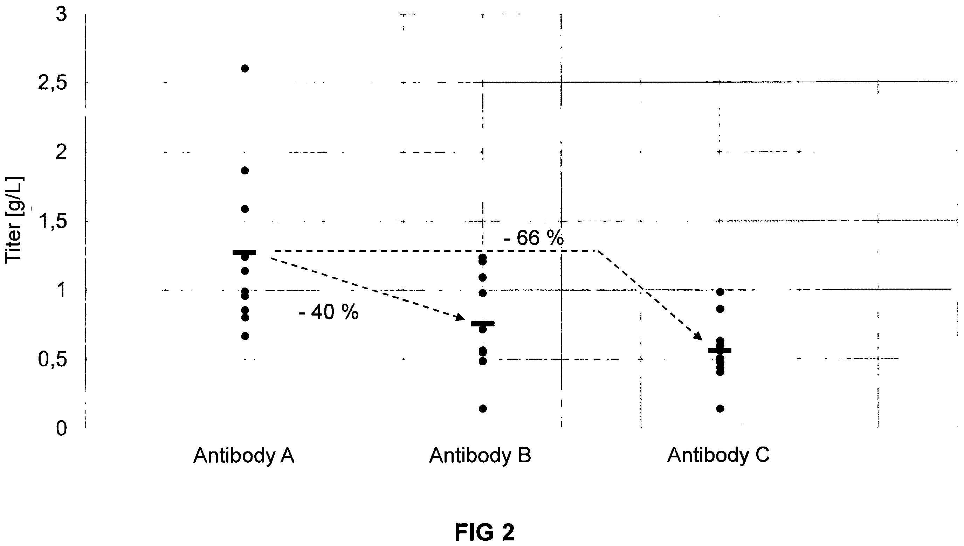

**Fig. 2** shows examples of product titers obtained as results of exemplary protein expression for a same kind of antibodies with different amino acid sequences. Antibodies A, B and C referred to in Fig. 2 were expressed using the exemplary automated cell culture system shown in Fig. 1. Further, antibodies A, B and C referred to in Fig. 2 are the same kind of antibodies but have different amino acid sequences. Fig. 2 shows that an average titer for antibody B was 40 % less than that of antibody A and that an average titer for antibody C was 66 % less than that of antibody A.

As can be seen from Fig. 2, within a class of proteins (e.g., antibody), different amino acid sequences can and most often will result in different efficiencies (e.g., product titers) when expressing the protein. It is often difficult to identify and/or predict a correlation between the amino acid sequence of the protein and the efficiency of the protein expression. The present disclosure is directed to optimizing protein expression with improved efficiency by enabling prediction of efficiency from an amino acid sequence.

## System Configuration

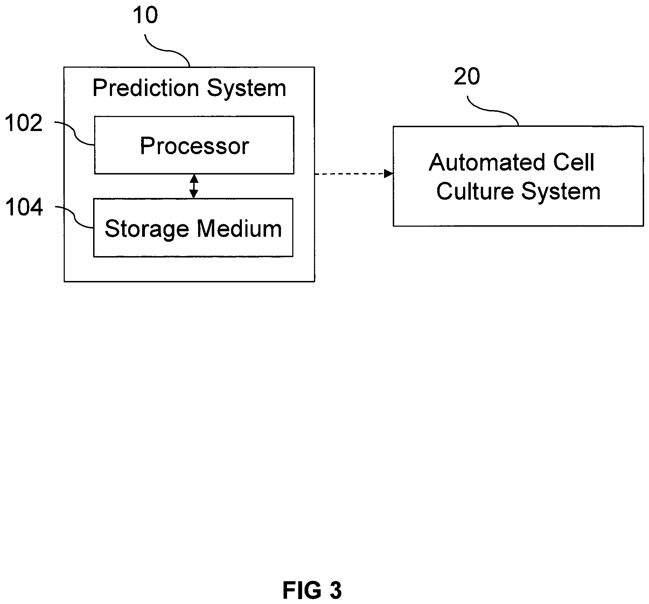

**Fig. 3** shows a functional block diagram of an exemplary system according to the present disclosure. The exemplary system shown in Fig. 3 comprises a prediction system 10 and an automated cell culture system 20.

As shown in Fig. 3, the prediction system 10 may comprise a processor 102 and a storage medium 104.

The processor 102 may be configured to predict efficiency of protein expression from an amino acid sequence. For the prediction, a plurality of prediction algorithms may be made available. Each prediction algorithm includes a combination of an encoding algorithm, a dimensionality reduction algorithm and a regression algorithm. Different prediction algorithms may include different combinations of those algorithms.

For example, the processor 102 may be configured to obtain a plurality of amino acid sequences and corresponding known efficiency values, each known efficiency value indicating efficiency of expressing a protein having a corresponding one of the plurality of amino acid sequences. The processor 102 may be configured to estimate, using the plurality of amino acid sequences and the known efficiency values according to each one of the prediction algorithms, a prediction function that takes a numerical vector corresponding to an amino acid sequence as an input and that outputs a predicted efficiency value for expressing a protein having the amino acid sequence. The processor 102 may be further configured to evaluate prediction functions that are estimated according to the plurality of prediction algorithms and select at least one prediction algorithm based on the evaluation. The processor 102 can then predict one or more efficiency values for expressing one or more proteins respectively having one or more specified amino acid sequences, using the at least one prediction algorithm and the prediction function obtained with the at least one prediction algorithm. The processor 102 may be further configured to output the one or more specified amino acid sequences and the one or more efficiency values predicted for the one or more specified amino acid sequences. Further details of a prediction process performed by the processor 102 will be described later below.

The storage medium 104 may be configured to store information that is necessary for the processor 102 to predict efficiency of the protein expression. For example, the storage medium 104 may be configured to store the plurality of amino acid sequences and the corresponding known efficiency values used for the prediction as stated above. Further, for example, the storage medium 104 may be configured to store information concerning the plurality of prediction algorithms. Further, for example, the storage medium 104 may be configured to store program code that enables the processor 102 to perform the prediction process as stated above. In addition, the storage medium 104 may be configured to store information that is obtained during and/or as a result of the prediction process by the processor 102 as stated above.

The prediction system 10 may be connected to the automated cell culture system 20 via one or more wired and/or wireless communication networks.

The automated cell culture system 20 may be a system that is capable of controlling expression of one or more proteins. As a specific example, the automated cell culture system 20 may be configured to perform the exemplary development process as stated above with reference to Fig. 1. For instance, the automated cell culture system 20 may comprise one or more holders of one or more receptacles in which cloning, expansion and/or selection of the cells may be performed. Further, the automated cell culture system 20 may comprise at least one container that is configured to provide an enclosed space for the receptacles and the respective holders to be accommodated. The enclosed space may provide a controlled environment (e.g., temperature, humidity, agitation, etc.) for cloning, expansion and/or selection of the cells. In addition, the automated cell culture system 20 may comprise means for agitation of the cells, e.g. a shaker, a stirrer or the like. A system to be used as the automated cell culture system 20 may comprise the Ambr 15 (Sartorius) in combination with the ALS CellCelector (Sartorius), and/or the Octet (Sartorius) and/or a liquid handler and/or a robotic arm and/or a cell culture incubator.

In some exemplary embodiments, the automated cell culture system 20 may further comprise a computer (not shown) that is configured to control operation of the automated cell culture system 20. For example, the computer of the automated cell culture system 20 may control one or more parameters (e.g., temperature, humidity, agitation, etc.) for the protein expression. The computer comprised in the automated cell culture system 20 may be further configured to receive the one or more specified amino acid sequences and the respective predicted efficiency value(s) from the processor 102 of the prediction system 10 so that one or more proteins respectively having the one or more specified amino acid sequences may be expressed in the automated cell culture system 20.

In some exemplary embodiments, the system does not necessarily comprise the automated cell culture system 20. In such exemplary embodiments, the output(s) from the prediction system 10 may be used in manual cell culture workflows, for example.

**Efficiency Prediction**

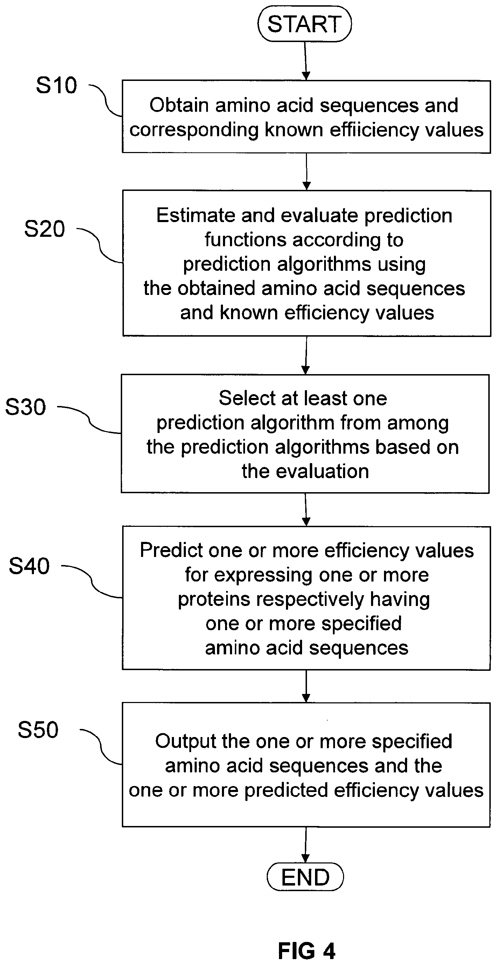

**Fig. 4** shows a flowchart of an exemplary process performed by an exemplary prediction system according to the present disclosure. For example, the processor 102 of the prediction system 10 shown in Fig. 3 may perform the exemplary process shown in Fig. 4. The exemplary process shown in Fig. 4 may be considered as an example of the prediction process performed by the processor 102 as stated above with reference to Fig. 3.

The exemplary process shown in Fig. 4 may start when a user input instructing the start of the exemplary process is received via an input device (not shown) by the processor 102 of the prediction system 10, for example.

At step S10, the processor 102 may obtain a plurality of amino acid sequences and corresponding known efficiency values. In some exemplary embodiments, each amino acid sequence may include a sequence of amino acids with the one-letter notation. The plurality of amino acid sequences and the corresponding known efficiency values may be received, for example, from the storage medium 104 and/or from a computer (e.g., database server) other than the prediction system 10. The known efficiency values may have been determined by expressing the proteins having the corresponding amino acid sequences with protein expression using a particular type of host cells and a particular type of expression vector (cf. Fig. 1). Information indicating the particular type of host cells and the particular type of expression vector may also be obtained at step S10 and stored in the storage medium 104. In some exemplary embodiments, the known efficiency values may be titers for expressing proteins respectively having the corresponding amino acid sequences.

In some exemplary embodiments, the plurality of amino acid sequences obtained at step S10 may be of antibodies or recombinant antibodies. Further, in some cases where the plurality of amino acid sequences are of antibodies or recombinant antibodies, the plurality of amino acid sequences may include light chain sequences of the antibodies or recombinant antibodies, heavy chain sequences of the antibodies or recombinant antibodies or light-heavy chain sequences of the antibodies or recombinant antibodies.

The exemplary process of Fig. 4 may proceed to step S20 after step S10.

At step S20, the processor 102 may estimate and evaluate prediction functions according to available prediction algorithms using the plurality of amino acid sequences and the corresponding known efficiency values obtained at step S10. Each of the available prediction algorithms may include a combination of an encoding algorithm, a dimensionality reduction algorithm and a regression algorithm. With the encoding algorithm, amino acid sequences may be encoded to numerical vectors. A dimension of the numerical vectors may be reduced with the dimensionality algorithm. Further, a prediction function may be estimated according to the regression algorithm. As also stated above, the prediction function takes a numerical vector corresponding to an amino acid sequence as an input and outputs a predicted efficiency value for expressing a protein having the amino acid sequence. In the exemplary embodiments where the known efficiency values are titers as stated above, the predicted efficiency value may also be a predicted titer for expressing the protein having the amino acid sequence.

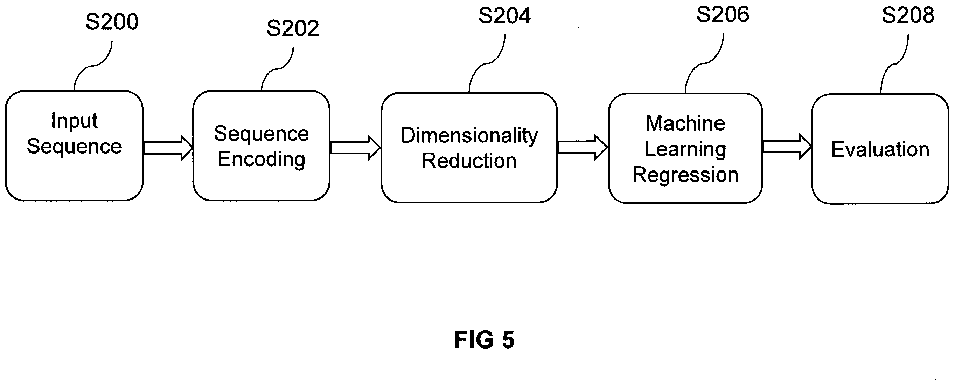

**Fig. 5** shows an exemplary workflow for estimating and evaluating prediction functions according to a plurality of prediction algorithms. The estimation and evaluation at step S20 of the exemplary process shown in Fig. 4 may be performed according to the exemplary workflow shown in Fig. 5. As shown in Fig. 5, the exemplary workflow comprises input sequence (step S200), sequence encoding (step S202), dimensionality reduction (step S204), machine learning regression (step S206) and evaluation (step S208). The processor 102 may implement process modules respectively corresponding to the steps S200 to S208 in order to perform step S20 of the exemplary process shown in Fig. 4.

At step S200 of the exemplary workflow shown in Fig. 5, the processor 102 may pre-process, if necessary, the plurality of amino acid sequences obtained at step S10 of Fig. 4. For example, the processor 102 may extract at least part of each one of the plurality of amino acid sequences. For example, the at least part of each amino acid sequence may include a first x % of the amino acid sequence and/or a last y % of the amino acid sequence, where each of x and y may be a value greater than 0 and less than or equal to 50. The at least part of each amino acid sequence may also be referred to as a "sub-sequence".

By extracting sub-sequences of the plurality of amino acid sequences, regions of sequences having most informative and discriminative patterns can be retained for further processing. In other words, the idea of generating sub-sequences may be understood as to skip highly similar regions of sequences for raising the level of uniqueness.

The regression algorithms used later in step S206 for estimating the prediction functions may learn discriminative patterns in a data-driven fashion which helps the regression algorithms to predict an appropriate group of amino acid sequences unknown to the regression algorithms. In some exemplary embodiments, to explore which particular sequence type has more potential to support the regression algorithms in predicting the efficiency of the protein expression, different types of amino acid sequences may be input to the exemplary workflow. For example, in case the plurality of amino acid sequences are of antibodies or recombinant antibodies, the following three types of sequences may be taken as inputs: light chain sequences, heavy chain sequences or light-heavy chain sequences. The light-heavy chain sequences may be generated by combining the light chain sequences and the heavy chain sequences.

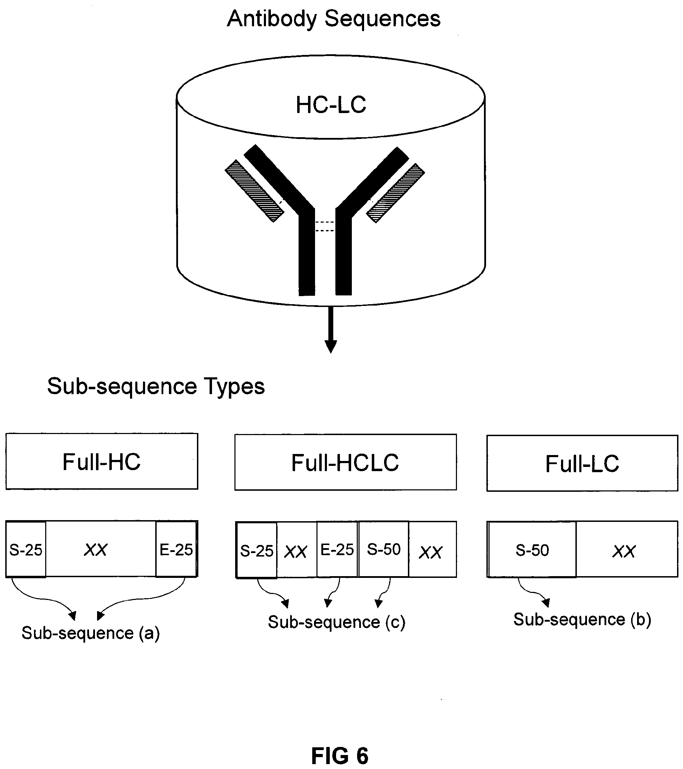

**Fig. 6** shows specific examples of different types of sub-sequences of antibodies or recombinant antibodies. In Fig. 6, "Full-HC", "Full-HCLC" and "Full-LC" respectively indicate a heavy chain sequence, a light-heavy chain sequence and a heavy chain sequence in their full lengths. Further, in Fig. 6, "S-25" and "S-50" indicate the first 25 % and the first 50 % of the corresponding sequences, respectively. Similarly, in Fig. 6, "E-25" indicates the last 25 % of the corresponding sequences. An exemplary sub-sequence (a) shown in Fig. 6 includes the first 25 % and the last 25 % of the heavy chain sequence. An exemplary sub-sequence (b) shown in Fig. 6 includes the first 50 % of the light chain sequence. An exemplary sub-sequence (c) shown in Fig. 6 includes the combination of the sub-sequence (a) and the sub-sequence (b), in other words, the first 25 % and the last 25 % of the heavy chain sequence as well as the first 50 % of the light chain sequence. It has been found that the exemplary sub-sequences (a), (b) and (c) shown in Fig. 6 can represent the most discriminative parts of the light chain, heavy chain and light-heavy chain sequence types, respectively, after extensive experimentation with the three types of amino acid sequences, heavy chain, light chain and light-heavy chain. In the experimentation, the exemplary workflow shown in Fig. 5 was performed with following types of amino acid sequences as inputs:

- the first 25 % of each type of sequences (heavy chain, light chain, light-heavy chain);
- the first 50 % of each type of sequences (heavy chain, light chain, light-heavy chain);
- the last 25 % of each type of sequences (heavy chain, light chain, light-heavy chain);
- the last 50 % of each type of sequences (heavy chain, light chain, light-heavy chain);
- the first 25 % and the last 25 % of each type of sequences (heavy chain, light chain, light-heavy chain); and
- the first 50 % and the last 50 % of each type of sequences (heavy chain, light chain, light-heavy chain), in other words, each type of sequences in the full length.

In the experimentation, the exemplary sub-sequence (c) shown in Fig. 6 has shown the best performance.

Referring again to Fig. 5, in some circumstances, for example, in case of processing the plurality of amino acid sequences in their full length, the pre-processing of step S200 may be skipped. For specific example, in case the light chain sequences, heavy chain sequences or light-heavy chain sequences of antibodies or recombinant antibodies are obtained at step S10 of Fig. 4 and if the full length of those sequences are to be processed, step S200 of Fig. 5 may be skipped.

At step S202, the processor 102 may encode the plurality of amino acid sequences that are pre-processed at step S200, according to a plurality of encoding algorithms to generate numerical vectors corresponding to the plurality of amino acid sequences. The numerical vectors generated at step S202 may be referred to as "first numerical vectors".

As also stated above, each amino acid sequence may include a sequence of amino acids in the one-letter notation. Thus, each amino acid sequence may be a sequence of alphabetic characters. Machine learning algorithms used in step S206 of the exemplary workflow shown in Fig. 5, on the other hand, may require statistical representation, e.g., numerical representation, of amino acid sequences for predictive modeling. Accordingly, the plurality of amino acid sequences may be encoded at step S202 to generate first numerical vectors corresponding to the plurality of amino acid sequences. In some exemplary embodiments, a plurality of encoding algorithms may be available for performing the encoding at step S202 of Fig. 5.

Thus, at step S202, for example, the processor 102 may, for each one of the plurality of amino acid sequences, encode at least part of the one of the plurality of amino acid sequences, (e.g., the sub-sequences obtained at step S200) according to one of the plurality of encoding algorithms to generate a first numerical vector. This encoding step may be repeated with different ones of the plurality of encoding algorithms to generate a plurality of sets of first numerical vectors.

Examples of the plurality of encoding algorithms used in step S202 of Fig. 5 may include, but are not limited to, three categories of algorithms: statistical, physico-chemical and word embedding. Statistical encoding algorithms (also referred to as "statistical encoders") may capture distributional information of amino acids. An example of a statistical encoder may be based on okapi-BM25, which will be described later below in detail.

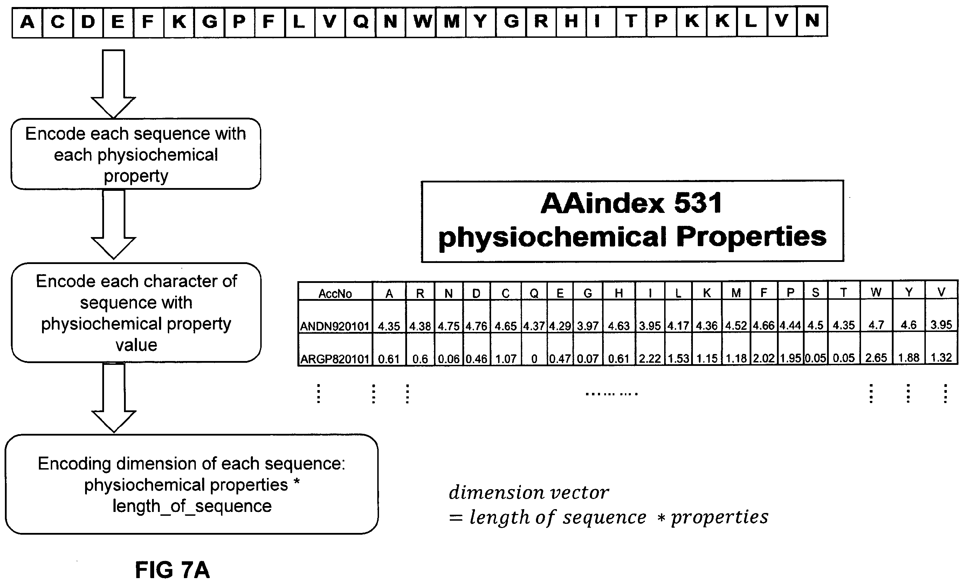

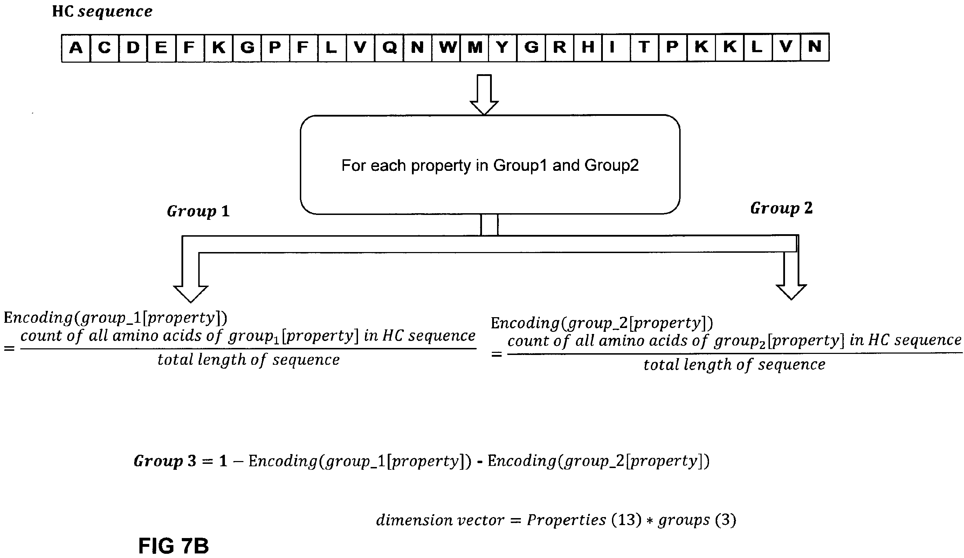

Physico-chemical properties based encoding algorithms (also referred to as "physico-chemical properties based encoders") may make use of pre-computed values of amino acids. In this category, some algorithms such as AAINDEX may replace amino acids with their respective values (see **Fig. 7A** for a specific, detailed example) and some algorithms such as CTDC (Composition / Transition / Distribution) may make use of physico-chemical properties along with some statistical procedures (see **Fig. 7B** for a specific, detailed example). With regards to the specific example of CTDC show in Fig. 7B, Table 1 below summarizes exemplary properties and groups that may be used in the specific example.

Table 1

| Properties | group1 | group2 | group3 |
|---|---|---|---|
| hydrophobicity_PRAM900101 | RKEDQN (SEQ ID NO: 1) | GASTPHY (SEQ ID NO: 2) | CLVIMFW (SEQ ID NO: 3) |
| hydrophobicity_ARGP820101 | QSTNGDE (SEQ ID NO: 4) | RAHCKMV (SEQ ID NO: 5) | LYPFIW (SEQ ID NO: 6) |
| hydrophobicity_ZIMJ680101 | QNGSWTDERA (SEQ ID NO: 7) | HMCKV (SEQ ID NO: 8) | LPFYI (SEQ ID NO: 9) |
| hydrophobicity_PONP930101 | KPDESNQT (SEQ ID NO: 10) | GRHA (SEQ ID NO: 11) | YMFWLCVI (SEQ ID NO: 12) |
| hydrophobicity_CASG920101 | KDEQPSRNTG (SEQ ID NO: 13) | AHYMLV (SEQ ID NO: 14) | FIWC (SEQ ID NO: 15) |
| hydrophobicity_ENGD860101 | RDKENQHYP (SEQ ID NO: 16) | SGTAW (SEQ ID NO: 17) | CVLIMF (SEQ ID NO: 18) |
| hydrophobicity_FASG890101 | KERSQD (SEQ ID NO: 19) | NTPG (SEQ ID NO: 20) | A YHWVMFLIC (SEQ ID NO: 21) |
| Normwaalsvolume | GASTPDC (SEQ ID NO: 22) | NVEQIL (SEQ ID NO: 23) | MHKFRYW (SEQ ID NO: 24) |
| Polarity | LIFWCMVY (SEQ ID NO: 25) | PATGS (SEQ ID NO: 26) | HQRKNED (SEQ ID NO: 27) |
| Polarizability | GASDT | CPNVEQIL | KMHFRYW |
| | (SEQ ID NO: 28) | (SEQ ID NO: 29) | (SEQ ID NO: 30) |
| Charge | KR | ANCQGHILMFPSTWYV (SEQ ID NO: 31) | DE |
| Secondarystruct | EALMQKRH (SEQ ID NO: 32) | VIYCWFT (SEQ ID NO: 33) | GNPSD (SEQ ID NO: 34) |
| solventaccess | ALFCGIVW (SEQ ID NO: 35) | RKQEND (SEQ ID NO: 36) | MSPTHY (SEQ ID NO: 37) |

Further, the following equations may be used in the specific example of CTDC shown in Fig. 7B.

$$Encoding\ (group_i[property])$$
$$= \frac{count\ of\ all\ amino\ acids\ of\ group_i[property]\ in\ HC\ sequence}{total\ length\ of\ sequence}$$

$$amino\ acids \in HC\ sequence$$

$$Encoding\ (group1[hydrophobicity\_PRAM900101])$$
$$= \frac{(count(R) + count(K) + count(E) + count(D) + count(Q) + count(N))}{total\ length\ of\ sequence}$$

Word embedding based encoding algorithms (also referred to as "word embedding based encoders") may make use of deep learning model and learn distribution of amino acids in an unsupervised manner.

As also stated above, in some exemplary embodiments, the plurality of encoding algorithms may include one or more algorithms based on physico-chemical properties of proteins. In a physico-chemical property based encoding algorithm, different weight values may be assigned to individual amino acids on the basis of physical and chemical characteristics of a protein, such as dissociation, solubility, hydration, polarity, charge, hydrophobicity, molecular weight, size, etc. The weight values may be computed through rich wet lab experiments performed by domain experts, for example. As a specific example, Table 2 below shows weight values (referred to as "feature value" in Table 2) assigned to the 20 kinds of amino acids based on physico-chemical properties.

Table 2

|  | One-letter code | Feature value |
|---|---|---|
| 1 | A | 0.008 |
| 2 | R | 0.171 |
| 3 | N | 0.255 |
| 4 | D | 0.303 |
| 5 | C | -0.132 |
| 6 | Q | 0.149 |
| 7 | E | 0.221 |
| 8 | G | 0.218 |
| 9 | H | 0.023 |
| 10 | I | -0.353 |
| 11 | L | -0.267 |
| 12 | K | 0.243 |
| 13 | M | -0.239 |
| 14 | F | -0.329 |
| 15 | P | 0.173 |
| 16 | S | 0.199 |
| 17 | T | 0.068 |
| 18 | W | -0.296 |
| 19 | Y | -0.141 |
| 20 | V | -0.274 |

Taking the following amino acid sequence as an exemplary hypothetical sequence of a protein:
(SEQ ID NO: 38) M L K K R F L G A L A ...,
a numerical vector (e.g., first numerical vector) generated using the physico-chemical property based weights shown in Table 2 may include the following:

$$[-0.239\ -0.267\ 0.243\ 0.243\ 0.171\ -0.329\ -0.267\ 0.218\ 0.008\ -0.267\ 0.008\ \ldots].$$

In the exemplary embodiments where the plurality of encoding algorithms include one or more physico-chemical property based encoding algorithms, the weight values such as the ones shown in Table 2 may be stored in the storage medium 104 of the prediction system 10 shown in Fig. 3. The processor 102 may reference the weight values stored in the storage medium 104 when encoding an amino acid sequence to generate a first numerical vector at step S202 of Fig. 5.

Further, in some exemplary embodiments, the plurality of encoding algorithms may include one or more algorithms based on k-mers of amino acid sequences. The "k-mers" of an amino acid sequence may be understood as substrings of length k contained within the amino acid sequence. In k-mer based encoding algorithms, k-mers of the at least part of the plurality of amino acid sequences may be first generated by rotating a window of size k with a stride size of S. The frequencies of each generated k-mer are used for encoding the amino acid sequence that contains the k-mers.

As a specific example of k-mer based encoding algorithm, Okapi-BM25 may be used. Okapi-BM25 is a ranking function used in information retrieval and can provide a score indicating relevance of a document against a certain query based on frequencies of terms included in the query appearing in the document.

In the encoding algorithm involving Okapi-BM25, all k-mers may be treated as queries and all sequences to be encoded may be treated as documents. In order to generate the encoding of amino acid sequences, the first step may be to generate the k-mers of the amino acid sequences which are generated by rotating a window of size k with the stride size of S. To illustrate better, consider an exemplary hypothetical corpus containing 2 sequences where 2-mers are generated by rotating a window of size 2 with the stride size of 1 over the sequences.

## Sequence 1: ACGTTGACTT (SEQ ID NO: 39; 2-mers=AC CG GT TT TG GA AC

## CT TT)

## Sequence 2: AGCTGTACTT (SEQ ID NO: 40; 2-mers=AG GC CT TG GT TA AC

## CT TT)

After the generation of k-mers, the second step may be to find unique k-mers vocabulary (e.g., V = {AC, CG, GT, TT, TG, GA, AC, CT, TT, AG, GC, TA}). The processor 102 may then compute elements of the numerical vectors (e.g., first numerical vectors) for each k-mer present in a sequence. Specifically, for example, using the k-mers vocabulary, the processor 102 may compute a k-mer frequency (KF) in the amino acid sequence *Seq*, the number of amino acid sequences in which k-mer appears represented as the sequence frequency (SF), and a k-mer inverse sequence frequency (ISF) to compute k-mer BM25 score and/or Okapi BM25 score using following equations:

$$ISF(q_i) = log\frac{number\ of\ sequences\ in\ corpus\ (S) - SF_{q_i} + 0.5}{\left(SF_{q_i} + 0.5\right)} \quad (1)$$

$$BM25Score(q_i, Seq) = \frac{KF(q_i, Seq) \times (k_1 + 1)}{KF(q_i, Seq) + k_1 \times \left(1 - b + b \times \frac{|Seq|}{S_{avg}}\right)} \quad (2)$$

$$OkapiBM25Score(q_i, Seq) = BM25Score(q_i, Seq) \times ISF(q_i) \quad (3)$$

In the above equations, $q_i$ may represent a particular k-mer taken from unique k-mers vocabulary *V*, *|Seq|* may denote the number of k-mers present in the amino acid sequence *Seq* and $S_{avg}$ may represent an average number of k-mers present in the corpus of amino acid sequences. Further, $k_1$ and *b* may be hyperparameters values which may fall in a range of $k_1$ = [1.2, 2.0] and *b* = 0.75, for example.

Using the above equations, assuming $k_1$ = 1.2, b = 0.75, |Seq| = 9, $S_{avg}$ = 9, for kmer (AC) that has occurred twice in sequence 1, *KF* will be 2, ISF(AC) will be -0.6989, BM25 Score (AC, sequence 1) will be 1.375 and Okapi BM25 Score (AC, sequence 1) will be -0.96. The value of the BM25 Score (AC, sequence 1) or of the Okapi BM25 Score (AC, sequence 1) may be considered as a weight for the kmer (AC) in sequence 1. Similarly, weights of other kmers (e.g., values of respective BM25 Scores or of respective Okapi BM25 Scores) can be computed to generate a numerical vector for sequence 1. A numerical vector for sequence 2 and all other amino acid sequences present in the corpus may also be computed in an analogous manner.

As also mentioned above, either values of the BM25 Scores (Equation (2)) or values of the Okapi BM25 Scores (Equation (3)) may be used as weights to be assigned to the kmers. In case the values of the Okapi BM25 Scores are used as the weights, all the Equations (1) to (3) above may be used for the computation. In case the values of BM25 Scores are used as the weights, the Equation (2) above may be used for the computation and the Equations (1) and (3) are not necessary for the computation.

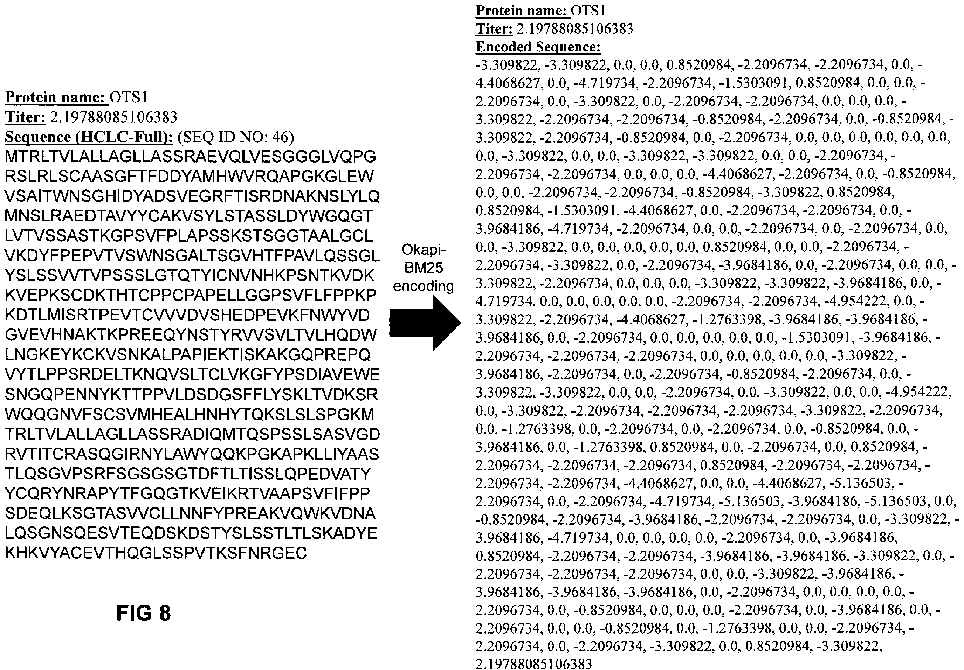

**Fig. 8** shows a specific example of encoding an amino acid sequence to generate a first numerical vector according to a k-mer based encoding algorithm using Okapi-BM25. In the specific example of Fig. 8, a light-heavy chain sequence with the full length is encoded to a numerical vector.

As also stated above, in some exemplary embodiments, the plurality of encoding algorithms may include one or more word embedding based encoding algorithms. A practical example of a word embedding based encoding algorithm involving Graph Representation based statistical representation generation will be described below. To make the example simple, rather than taking 20 different amino acids, only four different amino acids are taken in the following example.

Graph Representation Embedding

**Sequence Data:**

GCCGAGACTA (SEQ ID NO: 41)
GAGTCACATC (SEQ ID NO: 42)
AGTCCTGACA (SEQ ID NO: 43)
AGTCCTGACA (SEQ ID NO: 43)
AGTCCTGACA (SEQ ID NO: 43)

**Step 1: Generate bimers of each sample**

GCCGAGACTA: ['GC', 'CC', 'CG', 'GA', 'AG', 'GA', 'AC', 'CT', 'TA']
GAGTCACATC: ['GA', 'AG', 'GT', 'TC', 'CA', 'AC', 'CA', 'AT', 'TC']
AGTCCTGACA: ['AG', 'GT', 'TC', 'CC', 'CT', 'TG', 'GA', 'AC', 'CA']
AGTCCTGACA: ['AG', 'GT', 'TC', 'CC', 'CT', 'TG', 'GA', 'AC', 'CA']
AGTCCTGACA: ['AG', 'GT', 'TC', 'CC', 'CT', 'TG', 'GA', 'AC', 'CA']

**Step 2: Make a list of bimers by concatenating all the samples**

['GC', 'CC', 'CG', 'GA', 'AG', 'GA', 'AC', 'CT', 'TA', 'GA', 'AG', 'GT', 'TC', 'CA', 'AC', 'CA', 'AT', 'TC', 'AG', 'GT', 'TC', 'CC', 'CT', 'TG', 'GA', 'AC', 'CA', 'AG', 'GT', 'TC', 'CC', 'CT', 'TG', 'GA', 'AC', 'CA', 'AG', 'GT', 'TC', 'CC', 'CT', 'TG', 'GA', 'AC', 'CA']

**Step 3: make pairs of bimers**

[('GC', 'CC'), ('CC', 'CG'), ('CG', 'GA'), ('GA', 'AG'), ('AG', 'GA'), ('GA', 'AC'), ('AC', 'CT'), ('CT', 'TA'), ('TA', 'GA'), ('GA', 'AG'), ('AG', 'GT'), ('GT', 'TC'), ('TC', 'CA'), ('CA', 'AC'), ('AC', 'CA'), ('CA', 'AT'), ('AT', 'TC'), ('TC', 'AG'), ('AG', 'GT'), ('GT', 'TC'), ('TC', 'CC'), ('CC', 'CT'), ('CT', 'TG'), ('TG', 'GA'), ('GA', 'AC'), ('AC', 'CA'), ('CA', 'AG'), ('AG', 'GT'), ('GT', 'TC'), ('TC', 'CC'), ('CC', 'CT'), ('CT', 'TG'), ('TG', 'GA'), ('GA', 'AC'), ('AC', 'CA'), ('CA', 'AG'), ('AG', 'GT'), ('GT', 'TC'), ('TC', 'CC'), ('CC', 'CT'), ('CT', 'TG'), ('TG', 'GA'), ('GA', 'AC'), ('AC', 'CA')]

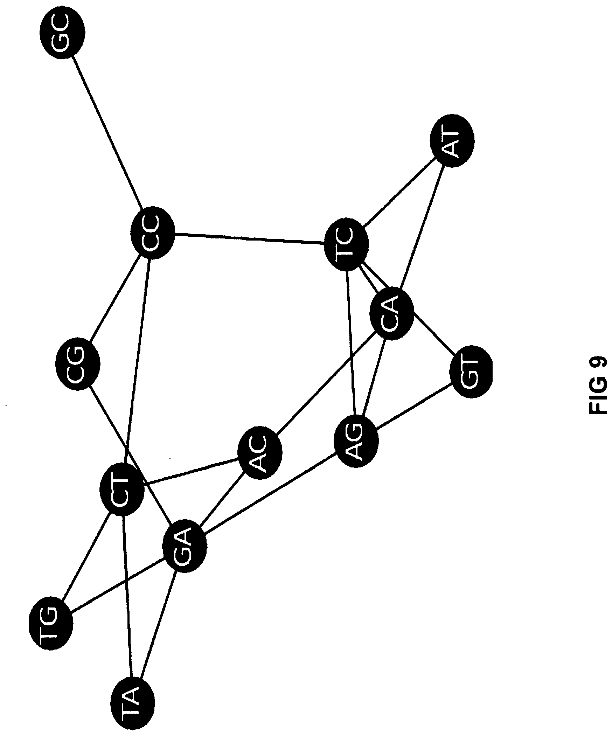

**Step 4: Generate Graph by taking unique bimers as nodes and edges by taking bimers pairs relations**
['GC', 'CC', 'CG', 'GA', 'AG', 'AC', 'CT', 'TA', 'GT', 'TC', 'CA', 'AT', 'TG']
The above bimers are unique bimers and each bimer can be represented with an integer. An example of a generated graph is shown in **Fig. 9.**
**Step 5: Create adjacency matrix from graph:**

1. read graph and get all nodes

2. create adjacency matrix by getting the edges from graph.

3. And the matrix as array may be:

[[0. 1. 0. 0. 0. 0. 0. 0. 0. 0. 0. 0. 0.]

[1. 0. 1. 0. 0. 0. 1. 0. 0. 1. 0. 0. 0.]

[0. 1. 0. 1. 0. 0. 0. 0. 0. 0. 0. 0. 0.]

[0. 0. 1. 0. 1. 1. 0. 1. 0. 0. 0. 0. 1.]

[0. 0. 0. 1. 0. 0. 0. 0. 1. 1. 1. 0. 0.]

[0. 0. 0. 1. 0. 0. 1. 0. 0. 0. 1. 0. 0.]

[0. 1. 0. 0. 0. 1. 0. 1. 0. 0. 0. 0. 1.]

[0. 0. 0. 1. 0. 0. 1. 0. 0. 0. 0. 0. 0.]

[0. 0. 0. 0. 1. 0. 0. 0. 0. 1. 0. 0. 0.]

[0. 1. 0. 0. 1. 0. 0. 0. 1. 0. 1. 1. 0.]

[0. 0. 0. 0. 1. 1. 0. 0. 0. 1. 0. 1. 0.]

[0. 0. 0. 0. 0. 0. 0. 0. 0. 1. 1. 0. 0.]

[0. 0. 0. 1. 0. 0. 1. 0. 0. 0. 0. 0. 0.]]

**Step 6:** Now, the algorithm may read the matrix and sum values of each array and create matrix of size (1,13) which may be:

[[1. 4. 2. 5. 4. 3. 4. 2. 2. 5. 4. 2. 2.]]

**Step 7:** Subsequently, the algorithm may repeat the above matrix with node size 13 and create a matrix such as:

[[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]

[1. 4. 2. 5. 4. 3. 4. 2. 2. 5. 4. 2. 2.]]

**Step 8:** The algorithm may then take the log of adjacency matrix/repeated matrix and minus the log of 1/node_size.
node_size = maximum number of nodes

$$\text{matrix} = \log(\text{adjacency matrix/repeated matrix}) - \log(1/\text{node\_size})$$

And the matrix may be:

[[0.   1.178655   0.   0.   0.   0., 0.   0.   0.   0.   0.   0., 0.   ], [2.56494936 0.   1.87180218 0.   0.   0., 1.178655   0.   0.   0.95551145 0.   0., 0.   ], [0.   1.178655   0.   0.95551145 0.   0., 0.   0.   0.   0.   0.   0., 0.   ], [0.   0.   1.87180218 0.   1.178655   1.46633707, 0.   1.87180218 0.   0.   0.   0., 1.87180218], [0.   0.   0.   0.95551145 0.   0., 0.   0.   1.87180218 0.95551145 1.178655   0., 0.   ], [0.   0.   0.   0.95551145 0.   0., 1.178655   0.   0.   0.   1.178655   0., 0.   ], [0.   1.178655   0.   0.   0.   1.46633707, 0.   1.87180218 0.   0.   0.   0., 1.87180218], [0.   0.   0.   0.95551145 0.   0., 1.178655   0.   0.   0.   0.   0., 0.   ], [0.   0.   0.   0.   1.178655   0., 0.   0.   0.   0.95551145 0.   0., 0.   ], [0.   1.178655   0.   0.   1.178655   0., 0.   0.   1.87180218 0.   1.178655   1.87180218, 0.   ], [0.   0.   0.   0.   1.178655   1.46633707, 0.   0.   0.   0.95551145 0.   1.87180218, 0.   ], [0.   0.   0.   0.   0.   0., 0.   0.   0.95551145 1.178655   0.   0., 0.   ], [0.   0.   0.   0.95551145 0.   0., 1.178655   0.   0.   0.   0.   0., 0.   ]]

**Step 9:** Now above matrix with dimension size may be passed to the SVD (singular value decomposition) function which returns three values such as:

**U:** matrix of eigenvectors of (dot product of matrix transpose and matrix) and return the matrix of dimension (13,2) which may be:

[[-0.03699832   0.05194064]

[-0.03454252  0.24204397]

[-0.08904815  0.06422915]

[ 0.36782685  0.65536502]

[-0.46348411  0.141408   ]

[-0.21221736  0.06567255]

[ 0.30954136  0.50359704]

[-0.08760413  0.03300486]

[-0.10556369  0.11058263]

[-0.62260098  0.31725057]

[-0.2275661   0.32846482]

[-0.18064115  0.06499917]

[-0.08760413  0.03300486]]

**Sigma:** singular value of matrix of eigenvectors of (dot product of matrix transpose and matrix) which may be:

[4.06050863 5.00618494]

**VT:** transpose of U with dimension (2,13) which may be:

[[-0.02181988    -0.12746053        0.1536365        -0.22118917    -0.17065249    0.16243322

   -0.12248585        0.31225135    -0.50066054    -0.23809435    -0.42929702    -0.3919077

   0.31225135]

  [  0.1240127        0.22061115        0.33553936        0.06438287        0.33236161    0.43567491

   0.08799002        0.43333351        0.17149149        0.16939347        0.13875178    0.24143164

   0.43333351]]

The sigma vector may be reshaped into (2,2) which may be:

[[4.06050863 0.          ], [0.          5.00618494]]

**Step 10:** Subsequently, the **U** may be multiplied with power of alpha value 0.5 of **sigma** and a vector of shape (13,2) may be returned.

**Step 11:** Further, the transpose of **V** may be multiplied with power of multiplication of **sigma** with alpha value 0.5 and a vector of shape (13,2) may be returned.

**Step 12:** The above two vectors from steps 10 and 11 may then be added and a vector of dimension (13,2) may be returned.

**Step 13:** The above vector from step 12 may be normalized and a vector of shape (13,2) may be returned:

$$[[-0.28827767 \quad 0.95754686]$$
$$[-0.30075636 \quad 0.95370101]$$
$$[\ 0.14399019 \quad 0.98957911]$$
$$[\ 0.18047275 \quad 0.98357998]$$
$$[-0.76964777 \quad 0.63846872]$$
$$[-0.08907568 \quad 0.99602486]$$
$$[\ 0.2738781 \quad\ 0.96176441]$$
$$[\ 0.39800417 \quad 0.91738361]$$
$$[-0.88843335 \quad 0.45900564]$$
$$[-0.84692865 \quad 0.53170656]$$
$$[-0.78476551 \quad 0.61979279]$$
$$[-0.8596588 \quad\ 0.51086863]$$
$$[\ 0.39800417 \quad 0.91738361]]$$

**Step 14:** the vector may be reshaped into (13,4) and the embedding may be returned, e.g., by getting the embedding of each node from a lookup table:

| | | | |
|---|---|---|---|
| GC | -0.28827766755584794 | 0.9575468585863358 | -0.571154913256859 |
| | 0.8208422900061557 | | |
| CC | -0.3007563592459236 | 0.9537010078494922 | 0.5310487207066668 |
| | 0.847341286752754 | | |
| CG | 0.14399019303043473 | 0.9895791147306304 | 0.09140321374020337 |
| | 0.9958139648136908 | | |
| GA | 0.18047274984044 | 0.9835799848334806 | -0.8187068949159197 |
| | 0.5742116510635545 | | |
| AG | -0.7696477736771328 | 0.6384687184771335 | 0.4719490055031393 |
| | 0.8816258481944583 | | |
| AC | -0.08907567614296603 | 0.9960248610951804 | 0.7380156803219812 |
| | 0.6747835620396242 | | |
| CT | 0.27387810042293925 | 0.9617644129976544 | -0.77747062504106 |
| | 0.6289192533213332 | | |
| TA | 0.3980041721080683 | 0.9173836051426748 | 0.7474766785590197 |
| | 0.6642880512325778 | | |
| GT | -0.8884333530344746 | 0.45900563963411234 | -0.48781506399409386 |
| | 0.8729469991588482 | | |
| TC | -0.8469286500794678 | 0.531706555982311 | -0.4149359398848531 |
| | 0.9098506282856947 | | |
| CA | -0.7847655072557946 | 0.6197927868421471 | -0.68745956575406 |
| | 0.7262226555631816 | | |
| AT | -0.8596587968915401 | 0.5108686258980776 | 0.24633479010427825 |
| | 0.9691847972313026 | | |
| TG | 0.39800417210806815 | 0.9173836051426748 | 0.7474766785590196 |

(continued)

0.6642880512325778

Various examples of the encoding algorithms stated above are mere examples and do not limit the encoding algorithms. In other words, the plurality of encoding algorithms may include algorithms other than the exemplary algorithms described above. Further non-limiting examples of the encoding algorithms are summarized below:

- Word Embedding based Encoders

  - Word2vec [1]
  - FastText [4][5]
  - DeepWalk [12]
  - Graph Auto Encoder (GAE) [13]
  - Graph Factorization (GF) [14]
  - Graph Representation (GraRep) [18]
  - Large scale Information Network Embedding (LINE) [19]
  - High-Order Proximity preserved Embedding (HOPE) [21]
  - Laplacian [23]
  - Structural Deep Network Embedding (SDNE) [25]
  - Singular Value Decomposition (SVD) [27]
  - Node2Vec [32]

- Statistical Encoders

  - Composition of k-spaced Amino Acid Pairs (CKSAAP) [2]
  - MonoMonoKGap [6]
  - MonoDiKGap [6]
  - MonoTriKGap [6]
  - DiMonoKGap [6]
  - DiDiKGap [6]
  - DiTriKGap [6]
  - TriMonoKGap [6]
  - TriDiKGap [6]
  - Accumulated Nucleotide Frequency ANF [45]
  - K-mer [28] [29]
  - EAAC [33] [2]
  - TFIDF [35] [36]
  - One-Hot Vector Encoding [35]
  - Cross-Covariance [38] [39] [40]
  - Okapi-BM25 [41]
  - GAAC [33] [2]
  - WSRC-Local [42]
  - WSRC-Global [42]
  - EGAAC [33] [2]
  - GTPC [33] [2]
  - Ctriad [43]
  - KSCTriad [33] [2]
  - CKSAAGP [33] [2]
  - PseKRAAC (type 1 to type 16) [44]
  - KSCTriad [33] [2]

- Physico-Chemical Property based Encoders

  - AAIndex [3]
  - CTDC [7][8][9][10][11]
  - CTDT [7][8][9][10][11]
  - CTDD [7][8][9][10][11]

- Soc-Number [15][16][17]
- QSOrder [15][16][17]
- PAAC [20]
- APAAC [22]
- BLOSUM62 [24]
- Z-Scale [26]
- Moran [30][31]
- Geary [34]
- NMBroto [37]
- Auto-covariance [38] [39] [40]
- Auto-Cross-Covariance [38] [39] [40]

The reference numbers indicated in [brackets] in the lists above respectively represent the following reference documents:

[1] Kenneth Ward Church. Word2vec. Natural Language Engineering, 23(1):155 - 162, 2017.

[2] Zhen Chen, Pei Zhao, Fuyi Li, Tatiana T Marquez-Lago, Andre' Leier, Jerico Revote, Yan Zhu, David R Powell, Tatsuya Akutsu, Geoffrey I Webb, et al. ilearn: an integrated platform and meta-learner for feature engineering, machine-learning analysis and modeling of dna, rna and protein sequence data. Briefings in bioinformatics, 21(3):1047-1057, 2020.

[3] Chun-Wei Tung and Shinn-Ying Ho. Computational identification of ubiquitylation sites from protein sequences. BMC bioinformatics, 9(1):1-15, 2008.

[4] Michal Busta, Lukas Neumann, and Jiri Matas. Fastext: Efficient unconstrained scene text detector. In Proceedings of the IEEE International Conference on Computer Vision, pages 1206-1214, 2015.

[5] Alexander Filonenko, Konstantin Gudkov, Aleksei Lebedev, Ivan Zagaynov, and Nikita Orlov. Fastext: Fast and small text extractor. In 2019 International Conference on Document Analysis and Recognition Workshops (ICDARW), volume 4, pages 49-54. IEEE, 2019.

[6] Rafsanjani Muhammod, Sajid Ahmed, Dewan Md Farid, Swakkhar Shatabda, Alok Sharma, and Abdollah Dehzangi. Pyfeat: a python-based effective feature generation tool for dna, rna and protein sequences. Bioinformatics, 35(19):3831-3833, 2019.

[7] CZ Cai, LY Han, Zhi Liang Ji, X Chen, and Yu Zong Chen. Svm-prot: web-based support vector machine software for functional classification of a protein from its primary sequence. Nucleic acids research, 31(13):3692-3697, 2003.

[8] CZ Cai, LY Han, ZL Ji, and YZ Chen. Enzyme family classification by support vector machines. Proteins: Structure, Function, and Bioinformatics, 55(1):66-76, 2004.

[9] Inna Dubchak, Ilya Muchnik, Stephen R Holbrook, and Sung-Hou Kim. Prediction of protein folding class using global description of amino acid sequence. Proceedings of the National Academy of Sciences, 92(19):8700-8704, 1995.

[10] Inna Dubchak, Ilya Muchnik, Christopher Mayor, Igor Dralyuk, and Sung-Hou Kim. Recognition of a protein fold in the context of the scop classification. Proteins: Structure, Function, and Bioinformatics, 35(4):401-407, 1999.

[11] Lian Yi Han, Cong Zhong Cai, Siew Lin Lo, Maxey CM Chung, and Yu Zong Chen. Prediction of rna-binding proteins from primary sequence by a support vector machine approach. Rna, 10(3):355-368, 2004.

[12] Bryan Perozzi, Rami Al-Rfou, and Steven Skiena. Deepwalk: Online learning of social representations. In Proceedings of the 20th ACM SIGKDD international conference on Knowledge discovery and data mining, pages 701-710, 2014.

[13] Thomas N Kipf and Max Welling. Variational graph auto-encoders. arXiv preprint arXiv:1611.07308, 2016.

[14] Palash Goyal and Emilio Ferrara. Graph embedding techniques, applications, and performance: A survey. Knowledge-Based Systems, 151:78-94, 2018.

[15] Kuo-Chen Chou. Prediction of protein subcellular locations by incorporating quasi-sequence-order effect. Biochemical and biophysical research communications, 278(2):477-483, 2000.

[16] Kuo-Chen Chou and Yu-Dong Cai. Prediction of protein subcellular locations by go-fund-pseaa predictor. Biochemical and Biophysical Research Communications, 320(4):1236-1239, 2004.

[17] Gisbert Schneider and Paul Wrede. The rational design of amino acid sequences by artificial neural networks and simulated molecular evolution: de novo design of an idealized leader peptidase cleavage site. Biophysical Journal, 66(2):335-344, 1994.

[18] Shaosheng Cao, Wei Lu, and Qiongkai Xu. Grarep: Learning graph representations with global structural information. In Proceedings of the 24th ACM international on conference on information and knowledge management, pages 891-900, 2015.

[19] Jian Tang, Meng Qu, Mingzhe Wang, Ming Zhang, Jun Yan, and Qiaozhu Mei. Line: Large-scale information network embedding. In Proceedings of the 24th international conference on world wide web, pages 1067-1077, 2015.

[20] Kuo-Chen Chou. Prediction of protein cellular attributes using pseudo-amino acid composition. Proteins: Structure, Function, and Bioinformatics, 43(3):246-255, 2001.

[21] Dingyuan Zhu, Peng Cui, Ziwei Zhang, Jian Pei, and Wenwu Zhu. High-order proximity preserved embedding for dynamic networks. IEEE Transactions on Knowledge and Data Engineering, 30(11):2134-2144, 2018.

[22] Kuo-Chen Chou. Using amphiphilic pseudo amino acid composition to predict enzyme subfamily classes. Bioinformatics, 21(1):10-19, 2005.

[23] Russell Merris. Laplacian matrices of graphs: a survey. Linear algebra and its applications, 197:143-176, 1994.

[24] Tzong-Yi Lee, Shu-An Chen, Hsin-Yi Hung, and Yu-Yen Ou. Incorporating distant sequence features and radial basis function networks to identify ubiquitin conjugation sites. PloS one, 6(3):e17331, 2011.

[25] Daixin Wang, Peng Cui, and Wenwu Zhu. Structural deep network embedding. In Proceedings of the 22nd ACM SIGKDD international conference on Knowledge discovery and data mining, pages 1225-1234, 2016.

[26] Yong-Zi Chen, Zhen Chen, Yu-Ai Gong, and Guoguang Ying. Sumohydro: a novel method for the prediction of sumoylation sites based on hydrophobic properties. PloS one, 7(6):e39195, 2012.

[27] Hervé Abdi. Singular value decomposition (svd) and generalized singular value decomposition. Encyclopedia of measurement and statistics, pages 907-912, 2007.

[28] William Stafford Noble, Scott Kuehn, Robert Thurman, Man Yu, and John Stamatoyannopoulos. Predicting the in vivo signature of human gene regulatory sequences. Bioinformatics, 21(suppl 1):i338-i343, 2005.

[29] Shobhit Gupta, Jonathan Dennis, Robert E Thurman, Robert Kingston, John A Stamatoyannopoulos, and William Stafford Noble. Predicting human nucleosome occupancy from primary sequence. PLoS computational biology, 4(8):e1000134, 2008.

[30] Zhi-Ping Feng and Chun-Ting Zhang. Prediction of membrane protein types based on the hydrophobic index of amino acids. Journal of protein chemistry, 19(4):269-275, 2000.

[31] Zong Lin and Xian-Ming Pan. Accurate prediction of protein secondary structural content. Journal of Protein Chemistry, 20(3):217-220, 2001.

[32] Aditya Grover and Jure Leskovec. node2vec: Scalable feature learning for networks. In Proceedings of the 22nd ACM SIGKDD international conference on Knowledge discovery and data mining, pages 855-864, 2016.

[33] Chao Zhou, Changshi Wang, Hongbo Liu, Qiangwei Zhou, Qian Liu, Yan Guo, Ting Peng, Jiaming Song, Jianwei Zhang, Lingling Chen, et al. Identification and analysis of adenine n6-methylation sites in the rice genome. Nature plants, 4(8):554-563, 2018.

[34] Robert R Sokal and Barbara A Thomson. Population structure inferred by local spatial autocorrelation: an example from an amerindian tribal population. American Journal of Physical Anthropology: The Official Publication of the American Association of Physical Anthropologists, 129(1):121-131, 2006.

[35] Juan Ramos et al. Using tf-idf to determine word relevance in document queries. In Proceedings of the first instructional conference on machine learning, volume 242, pages 29-48. Citeseer, 2003.

[36] Ari Aulia Hakim, Alva Erwin, Kho I Eng, Maulahikmah Galinium, and Wahyu Muliady. Automated document classification for news article in bahasa Indonesia based on term frequency inverse document frequency (tf-idf) approach. In 2014 6th international conference on information technology and electrical engineering (ICITEE), pages 1-4. IEEE, 2014.

[37] David S Home. Prediction of protein helix content from an autocorrelation analysis of sequence hydrophobicities. Biopolymers: Original Research on Biomolecules, 27(3):451-477, 1988.

[38] Bin Liu, Fule Liu, Longyun Fang, Xiaolong Wang, and Kuo-Chen Chou. repdna: a python package to generate various modes of feature vectors for dna sequences by incorporating user-defined physicochemical properties and sequence-order effects. Bioinformatics, 31(8):1307-1309, 2015.

[39] Qiwen Dong, Shuigeng Zhou, and Jihong Guan. A new taxonomy-based protein fold recognition approach based on autocross-covariance transformation. Bioinformatics, 25(20):2655-2662, 2009.

[40] Yanzhi Guo, Lezheng Yu, Zhining Wen, and Menglong Li. Using support vector machine combined with auto covariance to predict protein-protein interactions from protein sequences. Nucleic acids research, 36(9):3025-3030, 2008.

[41] John S Whissell and Charles LA Clarke. Improving document clustering using okapi bm25 feature weighting. Information retrieval, 14(5):466-487, 2011.

[42] Meng Kong, Yusen Zhang, Da Xu, Wei Chen, and Matthias Dehmer. Fctp-wsrc: protein-protein interactions prediction via weighted sparse representation based classification. Frontiers in genetics, 11:18, 2020.

[43] Juwen Shen, Jian Zhang, Xiaomin Luo, Weiliang Zhu, Kunqian Yu, Kaixian Chen, Yixue Li, and Hualiang Jiang. Predicting protein-protein interactions based only on sequences information. Proceedings of the National Academy of Sciences, 104(11):4337-4341, 2007.

[44] Yongchun Zuo, Yuan Li, Yingli Chen, Guangpeng Li, Zhenhe Yan, and Lei Yang. Psekraac: a flexible web server for generating pseudo k-tuple reduced amino acids composition. Bioinformatics, 33(1):122-124, 2017.

[45] Wei Chen, Hong Tran, Zhiyong Liang, Hao Lin, and Liqing Zhang. Identification and analysis of the n6-methyladenosine in the saccharomyces cerevisiae transcriptome. Scientific reports, 5(1):1-8, 2015.

Referring again to Fig. 5, after encoding the amino acid sequences at step S202, the processor 102 may perform dimensionality reduction at step S204. The dimensionality reduction may be performed, for each set of encoded sequences (e.g., each set of first numerical vectors generated according to one of the plurality of encoding algorithms), according to a plurality of dimensionality reduction algorithms. As a result of dimensionality reduction, further sets of numerical vectors may be generated, where the dimension of the further sets of numerical vectors is smaller than that of the encoded sequences. The further sets of numerical vectors generated at step S204 may be referred to as "second numerical vectors".

The dimensionality reduction may be considered as investigating whether the first numerical vectors contain redundant and repetitive patterns. In some exemplary embodiments, the plurality of dimensionality reduction algorithms used in step S204 may include at least some of the following:

- principal component analysis (PCA) in which principal components of a dataset are computed and a change of basis is performed on the dataset using the principal components;
- K-means in which $n$ observations in a dataset are partitioned into $k$ clusters, where each observation belongs to the cluster with the nearest mean (e.g., cluster center) and the nearest means of the $k$ clusters are used for the dimensionality reduction;
- t-distributed stochastic neighbor embedding (TSNE) which is a statistical method for visualizing high-dimensional data by giving each datapoint a location in a two or three-dimensional map;
- kernel-PCA which is an extension of PCA using techniques of kernel methods;
- locally-linear embedding (LLE) which provides a lower-dimensional projection of a dataset which preserves distances within local neighborhoods;
- tensor singular value decomposition (T-SVD) which computes the orthonormal subspaces associated with the different factors appearing in the tensor product of vector spaces in which the tensor lives;
- non-negative matrix factorization (NMF) which is a group of algorithms in multivariate analysis and linear algebra where a matrix V may be factorized into two or more matrices W and H, ..., with the property that all the matrices V, W, H, ... have no negative elements;
- multi-dimensional scaling (MDS) which is a means of visualizing the level of similarity of individual cases of a dataset ;
- factor analysis which is a statistical method used to describe variability among observed, correlated variables in terms of a potentially lower number of unobserved variables called factors;
- agglomerate feature which uses hierarchical clustering to group together features that behave similarly;
- Gaussian random projection which reduces the dimensionality by projecting the original input space on a random matrix generated using a Gaussian distribution;
- sparse random projection which projects the original input space using a sparse random matrix to reduce dimensionality;
- fast independent component analysis (fast-ICA) which is a kind of independent component analysis that is a computational method for separating a multivariate signal into additive subcomponents;
- t-distributed stochastic neighbor embedding (TSNE) which is a statistical method for visualizing high-dimensional data by giving each datapoint a location in a two or three-dimensional map ;
- kernel-PCA is an extension of principal component analysis (PCA) using techniques of kernel methods that are a class of algorithms for pattern analysis.

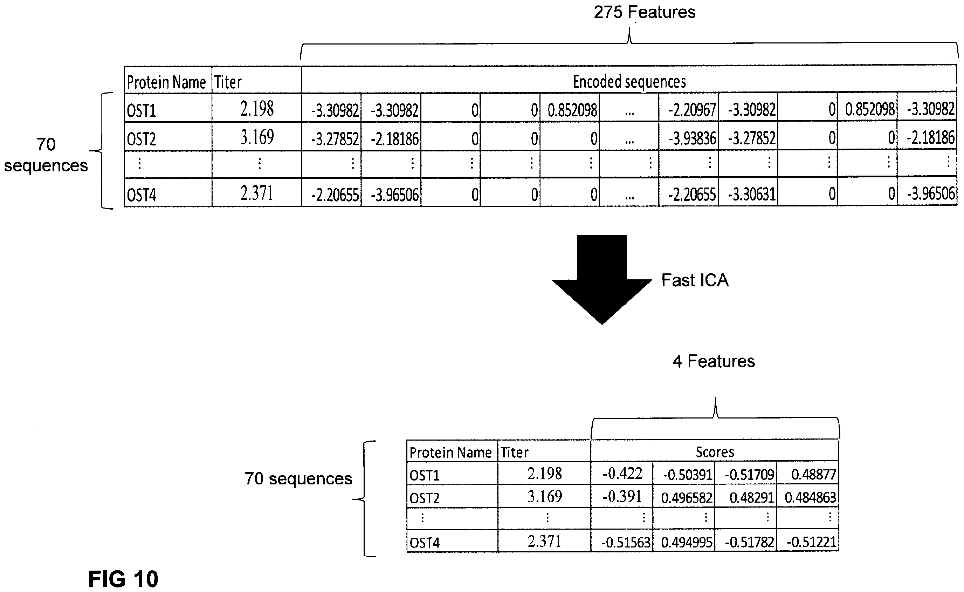

**Fig. 10** shows a specific example of dimensionality reduction of a first numerical vector to generate a second numerical vector. In the specific example shown in Fig. 10, 70 first numerical vectors having a dimension of 275 are processed according to the first-ICA and 70 second numerical vectors having a dimension of 4 are generated.

In the dimensionality reduction, it should be noted that, while transforming higher dimensional space to lower dimensional space, there is often a strong likelihood of loosing important information. Therefore, determining upto what minimum number of components can capture the most information of higher dimensional space may not be straightforward.

Accordingly, in some exemplary embodiments, an incremental method may be adopted for determining an optimal (reduced) dimensionality. In the incremental method, higher dimensional space may be mapped to K possible lower dimensional spaces before determining the optimal dimensionality through empirical evaluation.

For example, in the incremental method, in order to reduce the original sequence vector dimensions, an L (e.g., L = 275 in the specific example shown in Fig. 10) dimensional feature space may be mapped to multiple lower dimensional feature spaces and the one which captures the essence of high dimensional space with minimal dimensions may be selected. The incremental method may start by mapping L dimensional feature space to lower dimensional space by selecting N components of dimensionality reduction algorithms equal to xo % (e.g., xo = 5) of original feature vectors (e.g., first numerical vectors) and evaluate its efficacy for protein expression prediction. Details of this evaluation will be described below with regards to step S208 of Fig. 5. Subsequently, N components equal to (xo + n) % (e.g., n = 5) of the original feature vectors may be selected and its efficacy for protein expression may be evaluated. The percentage may be incremented by the interval of n % until a threshold percentage and evaluate the effectiveness of obtained N components for hand on task. For example, in case xo = 5, n = 5, and the threshold percentage is 95 %, the percentages of N components to be evaluated will be 5 %, 10 %, 15 %, 20 %, 25 %, 30 %, ..., 95%. In this manner, through empirical evaluation, different target dimensions can be found for different encoding algorithms. For instance, some encoding algorithms may perform better when their high dimensional feature vectors are reduced to 5% dimensions, some with 10% reduced dimensions, and so on. Optimal reduced dimension of the original feature vectors for a variety of encoding algorithms may be considered as a hyperparameter. To compute this hyperparameter for each one of the plurality of encoding algorithms, different reduced dimensions falling in the range of xo % (e.g., 5 %) to the threshold percentage (e.g., 95 %) with a step size of n % (e.g., 5 %) may be iteratively evaluated. Accordingly, in the incremental method, a plurality of sets of second numerical vectors may be generated using a single dimensionality reduction algorithm, with different reduced dimensionalities.

Referring again to Fig. 5, the processor 102 may perform machine learning regression at step S206, using the second numerical vectors generated by the dimensionality reduction at step S204. For example, at step S206, the processor 102 may obtain prediction functions by processing at least part of the second numerical vectors and the known efficiency values (e.g., obtained at step S10 of Fig. 4), according to a plurality of regression algorithms. In some exemplary embodiments, a prediction function according to one of the plurality of regression algorithms may be obtained for each set of second numerical vectors generated using one of the plurality of encoding algorithms and one of the plurality of dimensionality reduction algorithms with one of the reduced dimensionalities. In other words, a prediction function may be obtained for each prediction algorithm that is a combination of an encoding algorithm, a regression algorithm and a regression algorithm. The obtained prediction functions may be used to infer efficiency values for expressing proteins respectively having novel amino acid sequences.

As the available plurality of regression algorithms, algorithms that are capable of handling complex non-linear data distribution may be adopted. Examples of the plurality of regression algorithms may include, but are not limited to, linear regression, non-linear regression, penalized linear regression, penalized non-linear regression, naive Bayes, bagging regression, random forest regressor, boosting regression, partial least square regression, support vector machine, etc.

Linear regression is a linear approach for modelling the relationship between a scalar response (also referred to as dependent variable) and one or more explanatory variables (also referred to as independent variables). In non-linear regression, on the other hand, the relationship between the dependent variable and the independent variables may be modeled as a non-linear function.

Penalized linear regression / penalized non-linear regression may be understood as linear / non-linear regression that penalizes certain variables to avoid overfitting.

Naive Bayes may be understood as a conditional probability model based on applying Bayes' theorem with strong (in other word, "naïve") independence assumptions between the features.

Bagging regression is a kind of regression algorithms involving a machine learning ensemble meta-algorithm designed to improve the stability and accuracy of the regression algorithm.

Random forest regressor may be understood as an ensemble learning method for regression tasks, which operates by constructing a multitude of decision trees at training time, where the mean or average prediction of the individual trees is returned for the regression.

Boosting regression may be understood as a machine learning regression algorithm that gives a prediction model in the form of an ensemble of weak prediction models, which are typically decision trees.

In partial least square regression, a linear regression model can be found by projecting the predicted variables and the observable variables to a new space.

Support vector machine may be a supervised learning model that can assign new examples to one category or the other and that can be used for analyzing data for regression.

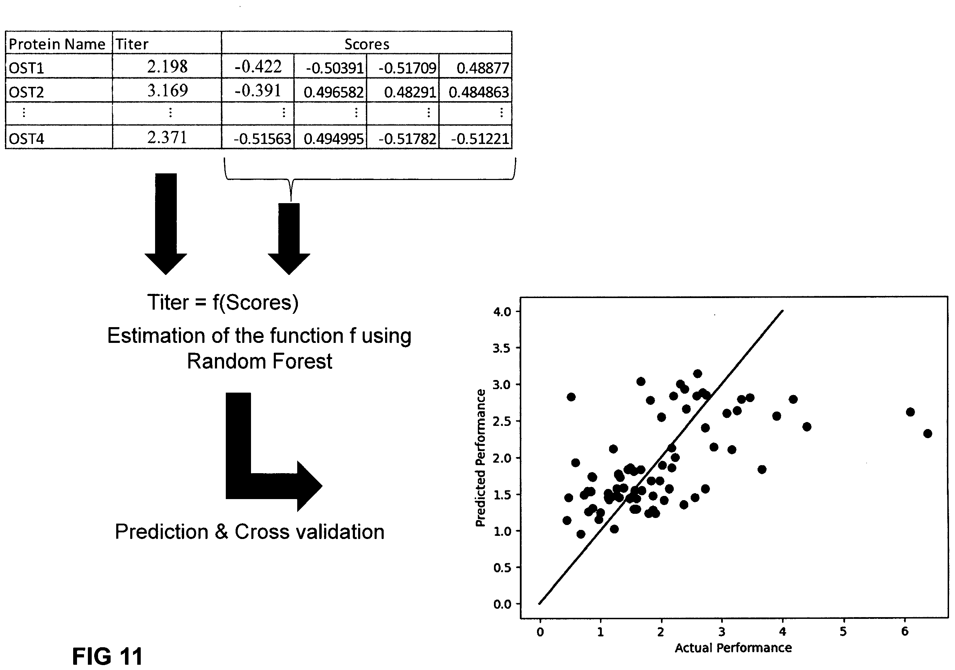

**Fig. 11** shows a specific example of estimating a prediction function according to a regression algorithm. In the specific example shown in Fig. 11, a prediction function f which returns a titer with an input including scores of a second numerical vector is estimated according to the random forest regression algorithm.

Referring again to Fig. 5, at step S208, the processor 102 may evaluate the prediction functions estimated at step S206. For example, the processor 102 may evaluate each prediction function by comparing predicted efficiency values output by the prediction function for at least part of the second numerical vectors with the known efficiency values for the amino acid sequences corresponding to the at least part of the second numerical vectors. More specifically, for example, the processor 102 may determine one or more of the following performance metrics for each prediction function: accuracy, precision, recall, F1-score.

For determining the performance metrics of accuracy, precision, recall and/or F1-score, for example, more than one second numerical vectors may be input to the prediction function and the predicted efficiency values output by the prediction function may be classified into two categories: high efficiency and low efficiency. For instance, the predicted efficiency values below a specified threshold value may be classified as low efficiency and the predicted efficiency values equal to or above the specified threshold value may be classified as high efficiency. The specified threshold value may vary depending on context (e.g., production objectives) of the desired analysis. The predicted efficiency values may then be compared to corresponding known efficiency values and each efficiency values may be labeled as true positive, false positive, true negative or false negative. True positive may be understood as both the predicted efficiency value and the corresponding known efficiency value being equal to or above the specified threshold value. False positive may be understood as the predicted efficiency value being equal to or above the specified threshold value while the corresponding known efficiency value being below the specified threshold value. True negative may be understood as both the predicted efficiency value and the corresponding known efficiency value being below the specified threshold value. False negative may be understood as the predicted efficiency value being below the specified threshold value while the corresponding known efficiency value being equal to or above the specified threshold value.

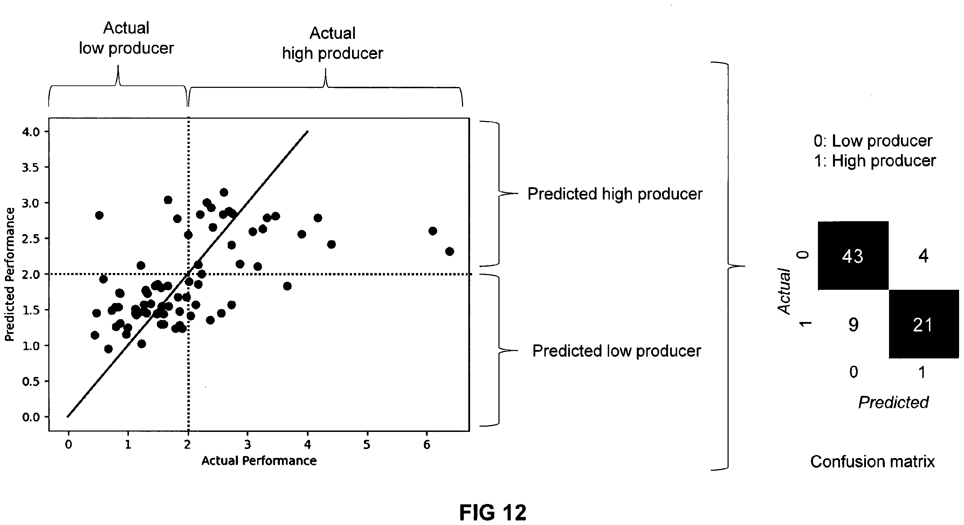

**Fig. 12** shows a specific example of classifying outputs of the prediction function for evaluating the prediction function. In the specific example of Fig. 12, 70 second numerical vectors (corresponding to amino acid sequences) as inputs to the prediction function are classified into high producer (e.g., with high efficiency equal to or above a threshold value (which is 2.0 in the specific example of Fig. 12)) and low producer (e.g., with low efficiency below the threshold value). The plot on the left of Fig. 12 shows the know efficiency values (referred to as "Actual Performance" in Fig. 12) against the predicted efficiency values (referred to as "Predicted Performance" in Fig. 12) for the 70 inputs. The numbers of inputs labeled as true positive, false positive, true negative and false negative are shown in the confusion matrix on the right in Fig. 12. As can be seen from the confusion matrix in Fig. 12, in this specific example, the number of true positives is 21, the number of false positives is 4, the number of true negatives is 43 and the number of false negatives is 9.

The performance metrics as stated above, accuracy, precision, recall and F1-score may be determined using the numbers of inputs labeled as true positive, false positive, true negative and false negative.

For example, accuracy may be computed as follows:

$$Accuracy = \frac{TruePositives + TrueNegatives}{TruePositives + TrueNegatives + FalsePositives + FalseNegatives} \ .$$

In case of the specific example shown in Fig. 12, accuracy will be 0.91 (= (21 + 43) / (21 + 43 + 4 + 9)).

Further, precision may be computed as follows:

$$Precision = \frac{TruePositives}{TruePositives + FalsePositives} \ .$$

In case of the specific example shown in Fig. 12, the precision will be 0.84 (= 21 / (21 + 4)).

Further, recall may be computed as follows:

$$Recall = \frac{TruePositives}{TruePositives + FalseNegatives} \ .$$

In case of the specific example shown in Fig. 12, recall will be 0.7 (= 21 / (21 + 9)).

Further, F1-score may be computed as follows:

$$F1score = 2 \times \frac{Precision \times Recall}{Precision + Recall} \ .$$

In case of the specific example shown in Fig. 12, F1-score will be 0.76 (0.84 * 0.7 / (0.84 + 0.7)).

Referring again to Fig. 5, the exemplary workflow of Fig. 5 may end after the evaluation at step S208.

In the exemplary embodiments description above with reference to Figs. 5 to 11, the processor 102 performs each of steps S202, S204 and S206 according to a plurality of encoding algorithms, a plurality of dimensionality reduction algorithms and a plurality of regression algorithms, respectively, before proceeding to the following step. In some exemplary embodiments, however, the processor 102 may first choose a combination of a single encoding algorithm, a single dimensionality reduction algorithm and a single regression algorithm and perform steps S202, S204 and S206 to estimate a prediction function for the combination of the algorithms, evaluate the prediction function at step S208 and then repeat the same steps S202 to S208 for another combination of algorithms until all possible combinations are processed.

Referring again to Fig. 4, after evaluating the prediction functions, step S20 may end. The exemplary process may then proceed to step S30.

At step S30, the processor 102 may select at least one prediction algorithm from among the available prediction algorithms based on the evaluation of the prediction functions performed at step S20 (more specifically, step S208 of Fig. 5). For example, the processor 102 may select at least one prediction algorithm which has the highest value(s) for one or more of the performance metrics determined at step S208 of Fig. 5. After step S30, the exemplary process may proceed to step S40.

At step S40, the processor 102 may predict one or more efficiency values for expressing one or more proteins respectively having one or more specified amino acid sequences, using the at least one prediction algorithm determined at step S30 and the corresponding prediction function. The efficiency value(s) predicted at step S40 may indicate efficiency of expressing respective proteins using the particular type of host cells and the particular type of expression vector used for determining the known efficiency values obtained at step S10. In some exemplary embodiments, the one or more specified amino acid sequences may be input by a user via an input device connected to the processor 102. Additionally or alternatively, the processor 102 may receive the one or more specified amino acid sequences from a computer (e.g., a server computer, a client computer, etc.) other than the prediction system 10. In some exemplary embodiments, the one or more specified amino acid sequences may include one or more amino acid sequences that are not included in the plurality of amino acid sequences obtained at step S10. Additionally or alternatively, the one or more specified amino acid sequences may include one or more of the plurality of amino acid sequences obtained at step S10. The exemplary process may proceed to step S50 after step S40.

At step S50, the processor 102 may output the one or more specified amino acid sequences and the one or more predicted efficiency values. In some exemplary embodiments, the one or more specified amino acid sequences and the one or more predicted efficiency values may be displayed on a display device (not shown) connected to the processor 102. Additionally or alternatively, the processor 102 may transmit information indicating the one or more specified amino acid sequences and the one or more predicted efficiency values to the automated cell culture system 20. In some exemplary embodiments, the particular type of host cells and the particular type of expression vector used for determining the known efficiency values obtained at step S10 may also be output at step S50.

In some exemplary embodiments, the processor 102 may further identify one of the one or more specified amino acid sequences for which a highest efficiency value is predicted and further output, at step S50, information indicating that the identified one of the one or more specified amino acid sequences has the highest predicted efficiency value.

The exemplary process of Fig. 4 may end after step S50.

The computational framework provided by any one of the exemplary embodiments described above can in-depth explore which antibody sequence type as well which region of sequence contain rich residue relationships for protein expression analysis and which statistical representation learning scheme effectively captures biological characteristics of residues. Further, the computational framework provided by any one of the exemplary embodiments described above can investigate whether generated statistical representation needs to be reduced to mitigate curse of dimensionality and which machine learning regressor performs better for hand on task. In a complete end-to-end predictive modelling workflow, the computational framework provided by any one of the exemplary embodiments described above can find the best combination of algorithms at different levels of predictive pipeline, e.g., which sequence encoding scheme performs better with what dimensionality reduction algorithm and which encoder-dimensionality reduction pair works better with what machine learning regressor. The robustness, transparency and effectiveness of the computational framework provided by any one of the exemplary embodiments described above can serve as a great alternative to highly time consuming and costly wet lab experiments for protein expression analysis, e.g., recombinant antibody sequence expression analysis.

### Application of Efficiency Prediction to Protein Expression

The results of the efficiency prediction as described above with reference to Figs. 4 to 12 may be used in protein expression performed at the automated cell culture system 20.

For example, the automated cell culture system 20 may use the one or more specified amino acid sequences output at step S50 of Fig. 4 to express one or more target proteins respectively including the one or more specified amino acid sequences. Specifically, for example, in the automated cell culture system 20, one or more nucleic acids that respectively encode the one or more specified acid sequences may be used to express the one or more target proteins, where the particular type of host cells and the particular type of expression vector used for determining the known efficiency values obtained at step S10 of Fig. 4 may be used.

Further, according to the exemplary process shown in Fig. 4, for example, an optimal predictive pipeline (e.g., the selected prediction algorithm(s) at step S30 of Fig. 4) can be found based on sequence encoding, dimensionality reduction, and machine learning regressor by validating different combination of algorithms on validation data (see also, e.g., Fig. 5). The best performing pipeline (e.g., the selected prediction algorithm(s)) may be saved and used whenever a target expression value of test amino acid sequences (e.g., recombinant antibody sequences) needs to be obtained.

Further, for example, the exemplary process shown in Fig. 4 may be used to generate accurate amino acid sequences of target proteins (e.g., recombinant antibodies) and perform an in-depth fault-less analysis of the target proteins. For instance, the selected prediction algorithm(s) may be used to predict efficiency values of expressing the target proteins respectively including different amino acid sequences. One or more amino acid sequences that may show high efficiency can then be identified for use in the protein expression in wet lab experiments.

## Hardware Configuration

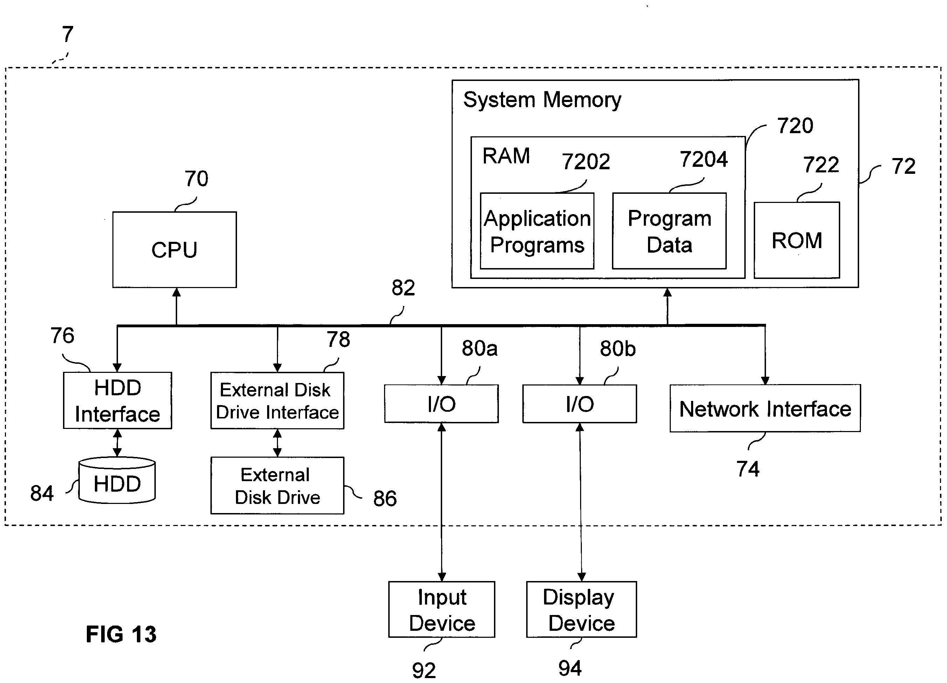

**Fig. 13** shows an exemplary hardware configuration of a computer that may be used to implement at least a part of the system as described above. For example, at least a part of the prediction system 10 and/or the automated cell culture system 20 shown in Fig. 3 may be implemented with the computer 7 shown in Fig. 13. The computer 7 shown in Fig. 13 includes a central processing unit (CPU) 70, a system memory 72, a network interface 74, a hard disk drive (HDD) interface 76, an external disk drive interface 78 and input/output (I/O) interfaces 80. These components of the computer are coupled to each other via a system bus 82. The CPU 70 may perform arithmetic, logic and/or control operations by accessing the system memory 72. The system memory 72 may store information and/or instructions for use in combination with the CPU 70. The system memory 72 may include volatile and non-volatile memory, such as a random access memory (RAM) 720 and a read only memory (ROM) 722. A basic input/output system (BIOS) containing the routines that helps to transfer information between elements within the computer 7, such as during start-up, may be stored in the ROM 722. The system bus 82 may be any of several types of bus structures including a memory bus or memory controller, a peripheral bus, and a local bus using any of a variety of bus architectures.

The computer may include a network interface 74 for communicating with other computers and/or devices via a network.

Further, the computer may include a hard disk drive (HDD) 84 for reading from and writing to a hard disk (not shown), and an external disk drive 86 for reading from or writing to a removable disk (not shown). The removable disk may be a magnetic disk for a magnetic disk drive or an optical disk such as a CD ROM for an optical disk drive. The HDD 84 and the external disk drive 86 are connected to the system bus 82 by a HDD interface 76 and an external disk drive interface 78, respectively. The drives and their associated computer-readable media provide non-volatile storage of computer-readable instructions, data structures, program modules and other data for the general purpose computer. The data structures may include relevant data for the implementation of the exemplary method and its variations as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 722 or RAM 720, including an operating system (not shown), one or more application programs 7202, other program modules (not shown), and program data 7204. The application programs may include at least a part of the functionality as described above.

The computer 7 may be connected to an input device 92 such as mouse and/or keyboard and a display device 94 such as liquid crystal display, via corresponding I/O interfaces 80a and 80b as well as the system bus 82. In case the computer 7 is implemented as a tablet computer, for example, a touch panel that displays information and that receives input may be connected to the computer 7 via a corresponding I/O interface and the system bus 82. Further, in some examples, although not shown in Fig. 13, the computer 7 may further be connected to a printer and/or an imaging device such as a camera, via corresponding I/O interfaces and the system bus 82.

In addition or as an alternative to an implementation using a computer 7 as shown in Fig. 13, a part or all of the functionality of the exemplary embodiments described herein may be implemented as one or more hardware circuits. Examples of such hardware circuits may include but are not limited to: Large Scale Integration (LSI), Reduced Instruction Set Circuits (RISC), Application Specific Integrated Circuit (ASIC) and Field Programmable Gate Array (FPGA).

Sequence Listing Information:
    DTD Version: V1_3
    File Name: S15547EU – SeqL ST.26 120722.xml
    Software Name: WIPO Sequence
    Software Version: 2.1.0
    Production Date: 2022-07-12
General Information:
    Current application / IP Office: EP
    Current application / Applicant file reference:
S15547EU – hb
    Applicant name: Sartorius Stedim Data Analytics AB
    Applicant name / Language: en
    Invention title: METHOD, COMPUTER PROGRAM PRODUCT AND
SYSTEM FOR OPTIMIZING PROTEIN EXPRESSION ( en )
    Sequence Total Quantity: 46
Sequences:
    Sequence Number (ID): 1
    Length: 6
    Molecule Type: AA
    Features Location/Qualifiers:
        - source, 1..6
            > mol_type, protein
            > organism, synthetic construct
    Residues:
    RKEDQN
6


    Sequence Number (ID): 2
    Length: 7
    Molecule Type: AA
    Features Location/Qualifiers:
        - source, 1..7
            > mol_type, protein
            > organism, synthetic construct
    Residues:
    GASTPHY
7


    Sequence Number (ID): 3
    Length: 7
    Molecule Type: AA
    Features Location/Qualifiers:
        - source, 1..7
            > mol_type, protein
            > organism, synthetic construct
    Residues:
    CLVIMFW
7


    Sequence Number (ID): 4
    Length: 7
    Molecule Type: AA
    Features Location/Qualifiers:
        - source, 1..7
            > mol_type, protein
            > organism, synthetic construct
    Residues:

```
QSTNGDE
```

```
Sequence Number (ID): 5
Length: 7
Molecule Type: AA
Features Location/Qualifiers:
      - source, 1..7
            > mol_type, protein
            > organism, synthetic construct
Residues:
RAHCKMV
```

```
Sequence Number (ID): 6
Length: 6
Molecule Type: AA
Features Location/Qualifiers:
      - source, 1..6
            > mol_type, protein
            > organism, synthetic construct
Residues:
LYPFIW
```

```
Sequence Number (ID): 7
Length: 10
Molecule Type: AA
Features Location/Qualifiers:
      - source, 1..10
            > mol_type, protein
            > organism, synthetic construct
Residues:
QNGSWTDERA
```

```
Sequence Number (ID): 8
Length: 5
Molecule Type: AA
Features Location/Qualifiers:
      - source, 1..5
            > mol_type, protein
            > organism, synthetic construct
Residues:
HMCKV
```

```
Sequence Number (ID): 9
Length: 5
Molecule Type: AA
Features Location/Qualifiers:
      - source, 1..5
            > mol_type, protein
            > organism, synthetic construct
Residues:
LPFYI
```

Sequence Number (ID): 10
Length: 8
Molecule Type: AA
Features Location/Qualifiers:
- source, 1..8
> mol_type, protein
> organism, synthetic construct
Residues:
KPDESNQT
8

Sequence Number (ID): 11
Length: 4
Molecule Type: AA
Features Location/Qualifiers:
- source, 1..4
> mol_type, protein
> organism, synthetic construct
Residues:
GRHA
4

Sequence Number (ID): 12
Length: 8
Molecule Type: AA
Features Location/Qualifiers:
- source, 1..8
> mol_type, protein
> organism, synthetic construct
Residues:
YMFWLCVI
8

Sequence Number (ID): 13
Length: 10
Molecule Type: AA
Features Location/Qualifiers:
- source, 1..10
> mol_type, protein
> organism, synthetic construct
Residues:
KDEQPSRNTG
10

Sequence Number (ID): 14
Length: 6
Molecule Type: AA
Features Location/Qualifiers:
- source, 1..6
> mol_type, protein
> organism, synthetic construct
Residues:
AHYMLV
6

Sequence Number (ID): 15

Length: 4
Molecule Type: AA
Features Location/Qualifiers:
        - source, 1..4
                > mol_type, protein
                > organism, synthetic construct
Residues:
FIWC
4


Sequence Number (ID): 16
Length: 9
Molecule Type: AA
Features Location/Qualifiers:
        - source, 1..9
                > mol_type, protein
                > organism, synthetic construct
Residues:
RDKENQHYP
9


Sequence Number (ID): 17
Length: 5
Molecule Type: AA
Features Location/Qualifiers:
        - source, 1..5
                > mol_type, protein
                > organism, synthetic construct
Residues:
SGTAW
5


Sequence Number (ID): 18
Length: 6
Molecule Type: AA
Features Location/Qualifiers:
        · - source, 1..6
                > mol_type, protein
                > organism, synthetic construct
Residues:
CVLIMF
6


Sequence Number (ID): 19
Length: 6
Molecule Type: AA
Features Location/Qualifiers:
        - source, 1..6
                > mol_type, protein
                > organism, synthetic construct
Residues:
KERSQD
6


Sequence Number (ID): 20
Length: 4
Molecule Type: AA

```
Features Location/Qualifiers:
      - source, 1..4
            > mol_type, protein
            > organism, synthetic construct
Residues:
NTPG
```
4

```
Sequence Number (ID): 21
Length: 10
Molecule Type: AA
Features Location/Qualifiers:
      - source, 1..10
            > mol_type, protein
            > organism, synthetic construct
Residues:
AYHWVMFLIC
```
10

```
Sequence Number (ID): 22
Length: 7
Molecule Type: AA
Features Location/Qualifiers:
      - source, 1..7
            > mol_type, protein
            > organism, synthetic construct
Residues:
GASTPDC
```
7

```
Sequence Number (ID): 23
Length: 6
Molecule Type: AA
Features Location/Qualifiers:
      - source, 1..6
            > mol_type, protein
            > organism, synthetic construct
Residues:
NVEQIL
```
6

```
Sequence Number (ID): 24
Length: 7
Molecule Type: AA
Features Location/Qualifiers:
      - source, 1..7
            > mol_type, protein
            > organism, synthetic construct
Residues:
MHKFRYW
```
7

```
Sequence Number (ID): 25
Length: 8
Molecule Type: AA
Features Location/Qualifiers:
      - source, 1..8
```

```
                      > mol_type, protein
                      > organism, synthetic construct
          Residues:
          LIFWCMVY
8


          Sequence Number (ID): 26
          Length: 5
          Molecule Type: AA
          Features Location/Qualifiers:
               - source, 1..5
                      > mol_type, protein
                      > organism, synthetic construct
          Residues:
          PATGS
5


          Sequence Number (ID): 27
          Length: 7
          Molecule Type: AA
          Features Location/Qualifiers:
               - source, 1..7
                      > mol_type, protein
                      > organism, synthetic construct
          Residues:
          HQRKNED
7


          Sequence Number (ID): 28
          Length: 5
          Molecule Type: AA
          Features Location/Qualifiers:
               - source, 1..5
                      > mol_type, protein
                      > organism, synthetic construct
          Residues:
          GASDT
5


          Sequence Number (ID): 29
          Length: 8
          Molecule Type: AA
          Features Location/Qualifiers:
               - source, 1..8
                      > mol_type, protein
                      > organism, synthetic construct
          Residues:
          CPNVEQIL
8


          Sequence Number (ID): 30
          Length: 7
          Molecule Type: AA
          Features Location/Qualifiers:
               - source, 1..7
                      > mol_type, protein
                      > organism, synthetic construct
```

Residues:
KMHFRYW

Sequence Number (ID): 31
Length: 16
Molecule Type: AA
Features Location/Qualifiers:
- source, 1..16
> mol_type, protein
> organism, synthetic construct
Residues:
ANCQGHILMF  PSTWYV

Sequence Number (ID): 32
Length: 8
Molecule Type: AA
Features Location/Qualifiers:
- source, 1..8
> mol_type, protein
> organism, synthetic construct
Residues:
EALMQKRH

Sequence Number (ID): 33
Length: 7
Molecule Type: AA
Features Location/Qualifiers:
- source, 1..7
> mol_type, protein
> organism, synthetic construct
Residues:
VIYCWFT

Sequence Number (ID): 34
Length: 5
Molecule Type: AA
Features Location/Qualifiers:
- source, 1..5
> mol_type, protein
> organism, synthetic construct
Residues:
GNPSD

Sequence Number (ID): 35
Length: 8
Molecule Type: AA
Features Location/Qualifiers:
- source, 1..8
> mol_type, protein
> organism, synthetic construct
Residues:
ALFCGIVW

8

```
Sequence Number (ID): 36
Length: 6
Molecule Type: AA
Features Location/Qualifiers:
     - source, 1..6
          > mol_type, protein
          > organism, synthetic construct
Residues:
RKQEND
```

6

```
Sequence Number (ID): 37
Length: 6
Molecule Type: AA
Features Location/Qualifiers:
     - source, 1..6
          > mol_type, protein
          > organism, synthetic construct
Residues:
MSPTHY
```

6

```
Sequence Number (ID): 38
Length: 11
Molecule Type: AA
Features Location/Qualifiers:
     - source, 1..11
          > mol_type, protein
          > organism, synthetic construct
Residues:
MLKKRFLGAL   A
```

11

```
Sequence Number (ID): 39
Length: 10
Molecule Type: DNA
Features Location/Qualifiers:
     - source, 1..10
          > mol_type, other DNA
          > organism, synthetic construct
Residues:
acgttgactt
```

10

```
Sequence Number (ID): 40
Length: 10
Molecule Type: DNA
Features Location/Qualifiers:
     - source, 1..10
          > mol_type, other DNA
          > organism, synthetic construct
Residues:
agctgtactt
```

10

Sequence Number (ID): 41
Length: 10
Molecule Type: DNA
Features Location/Qualifiers:
        - source, 1..10
                > mol_type, other DNA
                > organism, synthetic construct
Residues:
gccgagacta


Sequence Number (ID): 42
Length: 10
Molecule Type: DNA
Features Location/Qualifiers:
        - source, 1..10
                > mol_type, other DNA
                > organism, synthetic construct
Residues:
gagtcacatc


Sequence Number (ID): 43
Length: 10
Molecule Type: DNA
Features Location/Qualifiers:
        - source, 1..10
                > mol_type, other DNA
                > organism, synthetic construct
Residues:
agtcctgaca


Sequence Number (ID): 44
Length: 22
Molecule Type: AA
Features Location/Qualifiers:
        - source, 1..22
                > mol_type, protein
                > organism, synthetic construct
Residues:
EVQLVESGGG  LVQPGRSLRL  SC


Sequence Number (ID): 45
Length: 27
Molecule Type: AA
Features Location/Qualifiers:
        - source, 1..27
                > mol_type, protein
                > organism, synthetic construct
Residues:
ACDEFKGPFL  VQNWMYGRHI  TPKKLVN


Sequence Number (ID): 46
Length: 703

Molecule Type: AA
Features Location/Qualifiers:
  - source, 1..703
    > mol_type, protein
    > organism, synthetic construct
Residues:

| | | | |
|---|---|---|---|
| MTRLTVLALL | AGLLASSRAE | VQLVESGGGL | VQPGRSLRLS |
| CAASGFTFDD | YAMHWVRQAP | 60 | |
| GKGLEWVSAI | TWNSGHIDYA | DSVEGRFTIS | RDNAKNSLYL |
| QMNSLRAEDT | AVYYCAKVSY | 120 | |
| LSTASSLDYW | GQGTLVTVSS | ASTKGPSVFP | LAPSSKSTSG |
| GTAALGCLVK | DYFPEPVTVS | 180 | |
| WNSGALTSGV | HTFPAVLQSS | GLYSLSSVVT | VPSSSLGTQT |
| YICNVNHKPS | NTKVDKKVEP | 240 | |
| KSCDKTHTCP | PCPAPELLGG | PSVFLFPPKP | KDTLMISRTP |
| EVTCVVVDVS | HEDPEVKFNW | 300 | |
| YVDGVEVHNA | KTKPREEQYN | STYRVVSVLT | VLHQDWLNGK |
| EYKCKVSNKA | LPAPIEKTIS | 360 | |
| KAKGQPREPQ | VYTLPPSRDE | LTKNQVSLTC | LVKGFYPSDI |
| AVEWESNGQP | ENNYKTTPPV | 420 | |
| LDSDGSFFLY | SKLTVDKSRW | QQGNVFSCSV | MHEALHNHYT |
| QKSLSLSPGK | MTRLTVLALL | 480 | |
| AGLLASSRAD | IQMTQSPSSL | SASVGDRVTI | TCRASQGIRN |
| YLAWYQQKPG | KAPKLLIYAA | 540 | |
| STLQSGVPSR | FSGSGSGTDF | TLTISSLQPE | DVATYYCQRY |
| NRAPYTFGQG | TKVEIKRTVA | 600 | |
| APSVFIFPPS | DEQLKSGTAS | VVCLLNNFYP | REAKVQWKVD |
| NALQSGNSQE | SVTEQDSKDS | 660 | |
| TYSLSSTLTL | SKADYEKHKV | YACEVTHQGL | SSPVTKSFNR | GEC |
| 703 | | | |

END

```
<?xml version="1.0" encoding="UTF-8"?>
<!DOCTYPE ST26SequenceListing PUBLIC "-//WIPO//DTD Sequence
Listing 1.3//EN" "ST26SequenceListing_V1_3.dtd">
<ST26SequenceListing   dtdVersion="V1_3" fileName="S15547EU -
SeqL ST.26 120722.xml" softwareName="WIPO Sequence"
softwareVersion="2.1.0" productionDate="2022-07-12">
        <ApplicationIdentification>
                <IPOfficeCode>EP</IPOfficeCode>
                <ApplicationNumberText></ApplicationNumberText>
                <FilingDate></FilingDate>
        </ApplicationIdentification>
        <ApplicantFileReference>S15547EU -
hb</ApplicantFileReference>
        <ApplicantName languageCode="en">Sartorius Stedim Data
Analytics AB</ApplicantName>
        <InventionTitle languageCode="en">METHOD, COMPUTER
PROGRAM PRODUCT AND SYSTEM FOR OPTIMIZING PROTEIN
EXPRESSION</InventionTitle>
        <SequenceTotalQuantity>46</SequenceTotalQuantity>
        <SequenceData sequenceIDNumber="1">
                <INSDSeq>
                        <INSDSeq_length>6</INSDSeq_length>
                        <INSDSeq_moltype>AA</INSDSeq_moltype>
                        <INSDSeq_division>PAT</INSDSeq_division>
                        <INSDSeq_feature-table>
                                <INSDFeature>
                                        <INSDFeature_key>
source</INSDFeature_key>

                <INSDFeature_location>1..6</INSDFeature_location>
                                        <INSDFeature_quals>
                                                <INSDQualifier>
                                                        <INSDQualifier_name>
mol_type</INSDQualifier_name>
                                                        <INSDQualifier_value>
protein</INSDQualifier_value>
                                                </INSDQualifier>
                                                <INSDQualifier id="q2">
                                                        <INSDQualifier_name>
organism</INSDQualifier_name>
                                                        <INSDQualifier_value>
synthetic construct</INSDQualifier_value>
                                                </INSDQualifier>
                                        </INSDFeature_quals>
                                </INSDFeature>
                        </INSDSeq_feature-table>
                        <INSDSeq_sequence>RKEDQN</INSDSeq_sequence>
                </INSDSeq>
        </SequenceData>
        <SequenceData sequenceIDNumber="2">
                <INSDSeq>
                        <INSDSeq_length>7</INSDSeq_length>
                        <INSDSeq_moltype>AA</INSDSeq_moltype>
                        <INSDSeq_division>PAT</INSDSeq_division>
                        <INSDSeq_feature-table>
                                <INSDFeature>
                                        <INSDFeature_key>
```

```
source</INSDFeature_key>

        <INSDFeature_location>1..7</INSDFeature_location>
                        <INSDFeature_quals>
                                <INSDQualifier>
                                        <INSDQualifier_name>
mol_type</INSDQualifier_name>
                                        <INSDQualifier_value>
protein</INSDQualifier_value>
                                </INSDQualifier>
                                <INSDQualifier id="q3">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
        </INSDSeq_feature-table>
        <INSDSeq_sequence>GASTPHY</INSDSeq_sequence>
    </INSDSeq>
</SequenceData>
<SequenceData sequenceIDNumber="3">
    <INSDSeq>
        <INSDSeq_length>7</INSDSeq_length>
        <INSDSeq_moltype>AA</INSDSeq_moltype>
        <INSDSeq_division>PAT</INSDSeq_division>
        <INSDSeq_feature-table>
            <INSDFeature>
                <INSDFeature_key>
source</INSDFeature_key>

        <INSDFeature_location>1..7</INSDFeature_location>
                        <INSDFeature_quals>
                                <INSDQualifier>
                                        <INSDQualifier_name>
mol_type</INSDQualifier_name>
                                        <INSDQualifier_value>
protein</INSDQualifier_value>
                                </INSDQualifier>
                                <INSDQualifier id="q4">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
        </INSDSeq_feature-table>
        <INSDSeq_sequence>CLVIMFW</INSDSeq_sequence>
    </INSDSeq>
</SequenceData>
<SequenceData sequenceIDNumber="4">
    <INSDSeq>
        <INSDSeq_length>7</INSDSeq_length>
        <INSDSeq_moltype>AA</INSDSeq_moltype>
        <INSDSeq_division>PAT</INSDSeq_division>
```

```
                <INSDSeq_feature-table>
                        <INSDFeature>
                                <INSDFeature_key>
source</INSDFeature_key>

                <INSDFeature_location>1..7</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q5">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>QSTNGDE</INSDSeq_sequence>
            </INSDSeq>
        </SequenceData>
        <SequenceData sequenceIDNumber="5">
            <INSDSeq>
                <INSDSeq_length>7</INSDSeq_length>
                <INSDSeq_moltype>AA</INSDSeq_moltype>
                <INSDSeq_division>PAT</INSDSeq_division>
                <INSDSeq_feature-table>
                        <INSDFeature>
                                <INSDFeature_key>
source</INSDFeature_key>

                <INSDFeature_location>1..7</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q6">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>RAHCKMV</INSDSeq_sequence>
            </INSDSeq>
        </SequenceData>
        <SequenceData sequenceIDNumber="6">
            <INSDSeq>
```

```
                    <INSDSeq_length>6</INSDSeq_length>
                    <INSDSeq_moltype>AA</INSDSeq_moltype>
                    <INSDSeq_division>PAT</INSDSeq_division>
                    <INSDSeq_feature-table>
                            <INSDFeature>
                                    <INSDFeature_key>
source</INSDFeature_key>

            <INSDFeature_location>1..6</INSDFeature_location>
                                    <INSDFeature_quals>
                                            <INSDQualifier>
                                                    <INSDQualifier_name>
mol_type</INSDQualifier_name>
                                                    <INSDQualifier_value>
protein</INSDQualifier_value>
                                            </INSDQualifier>
                                            <INSDQualifier id="q7">
                                                    <INSDQualifier_name>
organism</INSDQualifier_name>
                                                    <INSDQualifier_value>
synthetic construct</INSDQualifier_value>
                                            </INSDQualifier>
                                    </INSDFeature_quals>
                            </INSDFeature>
                    </INSDSeq_feature-table>
                    <INSDSeq_sequence>LYPFIW</INSDSeq_sequence>
            </INSDSeq>
        </SequenceData>
        <SequenceData sequenceIDNumber="7">
                <INSDSeq>
                    <INSDSeq_length>10</INSDSeq_length>
                    <INSDSeq_moltype>AA</INSDSeq_moltype>
                    <INSDSeq_division>PAT</INSDSeq_division>
                    <INSDSeq_feature-table>
                            <INSDFeature>
                                    <INSDFeature_key>
source</INSDFeature_key>

            <INSDFeature_location>1..10</INSDFeature_location>
                                    <INSDFeature_quals>
                                            <INSDQualifier>
                                                    <INSDQualifier_name>
mol_type</INSDQualifier_name>
                                                    <INSDQualifier_value>
protein</INSDQualifier_value>
                                            </INSDQualifier>
                                            <INSDQualifier id="q8">
                                                    <INSDQualifier_name>
organism</INSDQualifier_name>
                                                    <INSDQualifier_value>
synthetic construct</INSDQualifier_value>
                                            </INSDQualifier>
                                    </INSDFeature_quals>
                            </INSDFeature>
                    </INSDSeq_feature-table>
                    <INSDSeq_sequence>
QNGSWTDERA</INSDSeq_sequence>
```

```
                                </INSDSeq>
                        </SequenceData>
                        <SequenceData sequenceIDNumber="8">
                                <INSDSeq>
                                        <INSDSeq_length>5</INSDSeq_length>
                                        <INSDSeq_moltype>AA</INSDSeq_moltype>
                                        <INSDSeq_division>PAT</INSDSeq_division>
                                        <INSDSeq_feature-table>
                                                <INSDFeature>
                                                        <INSDFeature_key>
source</INSDFeature_key>

                <INSDFeature_location>1..5</INSDFeature_location>
                                                        <INSDFeature_quals>
                                                                <INSDQualifier>
                                                                        <INSDQualifier_name>
mol_type</INSDQualifier_name>
                                                                        <INSDQualifier_value>
protein</INSDQualifier_value>
                                                                </INSDQualifier>
                                                                <INSDQualifier id="q9">
                                                                        <INSDQualifier_name>
organism</INSDQualifier_name>
                                                                        <INSDQualifier_value>
synthetic construct</INSDQualifier_value>
                                                                </INSDQualifier>
                                                        </INSDFeature_quals>
                                                </INSDFeature>
                                        </INSDSeq_feature-table>
                                        <INSDSeq_sequence>HMCKV</INSDSeq_sequence>
                                </INSDSeq>
                        </SequenceData>
                        <SequenceData sequenceIDNumber="9">
                                <INSDSeq>
                                        <INSDSeq_length>5</INSDSeq_length>
                                        <INSDSeq_moltype>AA</INSDSeq_moltype>
                                        <INSDSeq_division>PAT</INSDSeq_division>
                                        <INSDSeq_feature-table>
                                                <INSDFeature>
                                                        <INSDFeature_key>
source</INSDFeature_key>

                <INSDFeature_location>1..5</INSDFeature_location>
                                                        <INSDFeature_quals>
                                                                <INSDQualifier>
                                                                        <INSDQualifier_name>
mol_type</INSDQualifier_name>
                                                                        <INSDQualifier_value>
protein</INSDQualifier_value>
                                                                </INSDQualifier>
                                                                <INSDQualifier id="q10">
                                                                        <INSDQualifier_name>
organism</INSDQualifier_name>
                                                                        <INSDQualifier_value>
synthetic construct</INSDQualifier_value>
                                                                </INSDQualifier>
                                                        </INSDFeature_quals>
```

```
                    </INSDFeature>
                </INSDSeq_feature-table>
                <INSDSeq_sequence>LPFYI</INSDSeq_sequence>
            </INSDSeq>
        </SequenceData>
        <SequenceData sequenceIDNumber="10">
            <INSDSeq>
                <INSDSeq_length>8</INSDSeq_length>
                <INSDSeq_moltype>AA</INSDSeq_moltype>
                <INSDSeq_division>PAT</INSDSeq_division>
                <INSDSeq_feature-table>
                    <INSDFeature>
                        <INSDFeature_key>
source</INSDFeature_key>

        <INSDFeature_location>1..8</INSDFeature_location>
                        <INSDFeature_quals>
                            <INSDQualifier>
                                <INSDQualifier_name>
mol_type</INSDQualifier_name>
                                <INSDQualifier_value>
protein</INSDQualifier_value>
                            </INSDQualifier>
                            <INSDQualifier id="q11">
                                <INSDQualifier_name>
organism</INSDQualifier_name>
                                <INSDQualifier_value>
synthetic construct</INSDQualifier_value>
                            </INSDQualifier>
                        </INSDFeature_quals>
                    </INSDFeature>
                </INSDSeq_feature-table>
                <INSDSeq_sequence>KPDESNQT</INSDSeq_sequence>
            </INSDSeq>
        </SequenceData>
        <SequenceData sequenceIDNumber="11">
            <INSDSeq>
                <INSDSeq_length>4</INSDSeq_length>
                <INSDSeq_moltype>AA</INSDSeq_moltype>
                <INSDSeq_division>PAT</INSDSeq_division>
                <INSDSeq_feature-table>
                    <INSDFeature>
                        <INSDFeature_key>
source</INSDFeature_key>

        <INSDFeature_location>1..4</INSDFeature_location>
                        <INSDFeature_quals>
                            <INSDQualifier>
                                <INSDQualifier_name>
mol_type</INSDQualifier_name>
                                <INSDQualifier_value>
protein</INSDQualifier_value>
                            </INSDQualifier>
                            <INSDQualifier id="q12">
                                <INSDQualifier_name>
organism</INSDQualifier_name>
                                <INSDQualifier_value>
```

```
synthetic construct</INSDQualifier_value>
                                    </INSDQualifier>
                                </INSDFeature_quals>
                            </INSDFeature>
                        </INSDSeq_feature-table>
                        <INSDSeq_sequence>GRHA</INSDSeq_sequence>
                    </INSDSeq>
                </SequenceData>
                <SequenceData sequenceIDNumber="12">
                    <INSDSeq>
                        <INSDSeq_length>8</INSDSeq_length>
                        <INSDSeq_moltype>AA</INSDSeq_moltype>
                        <INSDSeq_division>PAT</INSDSeq_division>
                        <INSDSeq_feature-table>
                            <INSDFeature>
                                <INSDFeature_key>
source</INSDFeature_key>

        <INSDFeature_location>1..8</INSDFeature_location>
                                <INSDFeature_quals>
                                    <INSDQualifier>
                                        <INSDQualifier_name>
mol_type</INSDQualifier_name>
                                        <INSDQualifier_value>
protein</INSDQualifier_value>
                                    </INSDQualifier>
                                    <INSDQualifier id="q13">
                                        <INSDQualifier_name>
organism</INSDQualifier_name>
                                        <INSDQualifier_value>
synthetic construct</INSDQualifier_value>
                                    </INSDQualifier>
                                </INSDFeature_quals>
                            </INSDFeature>
                        </INSDSeq_feature-table>
                        <INSDSeq_sequence>YMFWLCVI</INSDSeq_sequence>
                    </INSDSeq>
                </SequenceData>
                <SequenceData sequenceIDNumber="13">
                    <INSDSeq>
                        <INSDSeq_length>10</INSDSeq_length>
                        <INSDSeq_moltype>AA</INSDSeq_moltype>
                        <INSDSeq_division>PAT</INSDSeq_division>
                        <INSDSeq_feature-table>
                            <INSDFeature>
                                <INSDFeature_key>
source</INSDFeature_key>

        <INSDFeature_location>1..10</INSDFeature_location>
                                <INSDFeature_quals>
                                    <INSDQualifier>
                                        <INSDQualifier_name>
mol_type</INSDQualifier_name>
                                        <INSDQualifier_value>
protein</INSDQualifier_value>
                                    </INSDQualifier>
                                    <INSDQualifier id="q14">
```

```
                                           <INSDQualifier_name>
organism</INSDQualifier_name>
                                           <INSDQualifier_value>
synthetic construct</INSDQualifier_value>
                                      </INSDQualifier>
                                  </INSDFeature_quals>
                              </INSDFeature>
                          </INSDSeq_feature-table>
                          <INSDSeq_sequence>
KDEQPSRNTG</INSDSeq_sequence>
                      </INSDSeq>
                  </SequenceData>
                  <SequenceData sequenceIDNumber="14">
                      <INSDSeq>
                          <INSDSeq_length>6</INSDSeq_length>
                          <INSDSeq_moltype>AA</INSDSeq_moltype>
                          <INSDSeq_division>PAT</INSDSeq_division>
                          <INSDSeq_feature-table>
                              <INSDFeature>
                                  <INSDFeature_key>
source</INSDFeature_key>

          <INSDFeature_location>1..6</INSDFeature_location>
                                  <INSDFeature_quals>
                                      <INSDQualifier>
                                           <INSDQualifier_name>
mol_type</INSDQualifier_name>
                                           <INSDQualifier_value>
protein</INSDQualifier_value>
                                      </INSDQualifier>
                                      <INSDQualifier id="q15">
                                           <INSDQualifier_name>
organism</INSDQualifier_name>
                                           <INSDQualifier_value>
synthetic construct</INSDQualifier_value>
                                      </INSDQualifier>
                                  </INSDFeature_quals>
                              </INSDFeature>
                          </INSDSeq_feature-table>
                          <INSDSeq_sequence>AHYMLV</INSDSeq_sequence>
                      </INSDSeq>
                  </SequenceData>
                  <SequenceData sequenceIDNumber="15">
                      <INSDSeq>
                          <INSDSeq_length>4</INSDSeq_length>
                          <INSDSeq_moltype>AA</INSDSeq_moltype>
                          <INSDSeq_division>PAT</INSDSeq_division>
                          <INSDSeq_feature-table>
                              <INSDFeature>
                                  <INSDFeature_key>
source</INSDFeature_key>

          <INSDFeature_location>1..4</INSDFeature_location>
                                  <INSDFeature_quals>
                                      <INSDQualifier>
                                           <INSDQualifier_name>
mol_type</INSDQualifier_name>
```

```
                                        <INSDQualifier_value>
protein</INSDQualifier_value>
                                </INSDQualifier>
                                <INSDQualifier id="q16">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
            </INSDSeq_feature-table>
            <INSDSeq_sequence>FIWC</INSDSeq_sequence>
        </INSDSeq>
    </SequenceData>
    <SequenceData sequenceIDNumber="16">
        <INSDSeq>
            <INSDSeq_length>9</INSDSeq_length>
            <INSDSeq_moltype>AA</INSDSeq_moltype>
            <INSDSeq_division>PAT</INSDSeq_division>
            <INSDSeq_feature-table>
                <INSDFeature>
                    <INSDFeature_key>
source</INSDFeature_key>

                    <INSDFeature_location>1..9</INSDFeature_location>
                    <INSDFeature_quals>
                        <INSDQualifier>
                            <INSDQualifier_name>
mol_type</INSDQualifier_name>
                            <INSDQualifier_value>
protein</INSDQualifier_value>
                        </INSDQualifier>
                        <INSDQualifier id="q17">
                            <INSDQualifier_name>
organism</INSDQualifier_name>
                            <INSDQualifier_value>
synthetic construct</INSDQualifier_value>
                        </INSDQualifier>
                    </INSDFeature_quals>
                </INSDFeature>
            </INSDSeq_feature-table>
            <INSDSeq_sequence>RDKENQHYP</INSDSeq_sequence>
        </INSDSeq>
    </SequenceData>
    <SequenceData sequenceIDNumber="17">
        <INSDSeq>
            <INSDSeq_length>5</INSDSeq_length>
            <INSDSeq_moltype>AA</INSDSeq_moltype>
            <INSDSeq_division>PAT</INSDSeq_division>
            <INSDSeq_feature-table>
                <INSDFeature>
                    <INSDFeature_key>
source</INSDFeature_key>

                    <INSDFeature_location>1..5</INSDFeature_location>
                        <INSDFeature_quals>
```

```
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q18">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>SGTAW</INSDSeq_sequence>
            </INSDSeq>
        </SequenceData>
        <SequenceData sequenceIDNumber="18">
            <INSDSeq>
                <INSDSeq_length>6</INSDSeq_length>
                <INSDSeq_moltype>AA</INSDSeq_moltype>
                <INSDSeq_division>PAT</INSDSeq_division>
                <INSDSeq_feature-table>
                        <INSDFeature>
                                <INSDFeature_key>
source</INSDFeature_key>

                                <INSDFeature_location>1..6</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q19">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>CVLIMF</INSDSeq_sequence>
            </INSDSeq>
        </SequenceData>
        <SequenceData sequenceIDNumber="19">
            <INSDSeq>
                <INSDSeq_length>6</INSDSeq_length>
                <INSDSeq_moltype>AA</INSDSeq_moltype>
                <INSDSeq_division>PAT</INSDSeq_division>
                <INSDSeq_feature-table>
                        <INSDFeature>
                                <INSDFeature_key>
source</INSDFeature_key>
```

```
                <INSDFeature_location>1..6</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q20">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>KERSQD</INSDSeq_sequence>
            </INSDSeq>
        </SequenceData>
        <SequenceData sequenceIDNumber="20">
            <INSDSeq>
                <INSDSeq_length>4</INSDSeq_length>
                <INSDSeq_moltype>AA</INSDSeq_moltype>
                <INSDSeq_division>PAT</INSDSeq_division>
                <INSDSeq_feature-table>
                        <INSDFeature>
                                <INSDFeature_key>
source</INSDFeature_key>

                <INSDFeature_location>1..4</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q21">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>NTPG</INSDSeq_sequence>
            </INSDSeq>
        </SequenceData>
        <SequenceData sequenceIDNumber="21">
            <INSDSeq>
                <INSDSeq_length>10</INSDSeq_length>
                <INSDSeq_moltype>AA</INSDSeq_moltype>
                <INSDSeq_division>PAT</INSDSeq_division>
                <INSDSeq_feature-table>
```

```
                        <INSDFeature>
                                <INSDFeature_key>
source</INSDFeature_key>

                <INSDFeature_location>1..10</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q22">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>
AYHWVMFLIC</INSDSeq_sequence>
        </INSDSeq>
    </SequenceData>
    <SequenceData sequenceIDNumber="22">
        <INSDSeq>
                <INSDSeq_length>7</INSDSeq_length>
                <INSDSeq_moltype>AA</INSDSeq_moltype>
                <INSDSeq_division>PAT</INSDSeq_division>
                <INSDSeq_feature-table>
                        <INSDFeature>
                                <INSDFeature_key>
source</INSDFeature_key>

                <INSDFeature_location>1..7</INSDFeature_location>
                                <INSDFeature_quals>
                                        <INSDQualifier>
                                                <INSDQualifier_name>
mol_type</INSDQualifier_name>
                                                <INSDQualifier_value>
protein</INSDQualifier_value>
                                        </INSDQualifier>
                                        <INSDQualifier id="q23">
                                                <INSDQualifier_name>
organism</INSDQualifier_name>
                                                <INSDQualifier_value>
synthetic construct</INSDQualifier_value>
                                        </INSDQualifier>
                                </INSDFeature_quals>
                        </INSDFeature>
                </INSDSeq_feature-table>
                <INSDSeq_sequence>GASTPDC</INSDSeq_sequence>
        </INSDSeq>
    </SequenceData>
    <SequenceData sequenceIDNumber="23">
        <INSDSeq>
```

```
                    <INSDSeq_length>6</INSDSeq_length>
                    <INSDSeq_moltype>AA</INSDSeq_moltype>
                    <INSDSeq_division>PAT</INSDSeq_division>
                    <INSDSeq_feature-table>
                            <INSDFeature>
                                    <INSDFeature_key>
source</INSDFeature_key>

            <INSDFeature_location>1..6</INSDFeature_location>
                                    <INSDFeature_quals>
                                            <INSDQualifier>
                                                    <INSDQualifier_name>
mol_type</INSDQualifier_name>
                                                    <INSDQualifier_value>
protein</INSDQualifier_value>
                                            </INSDQualifier>
                                            <INSDQualifier id="q24">
                                                    <INSDQualifier_name>
organism</INSDQualifier_name>
                                                    <INSDQualifier_value>
synthetic construct</INSDQualifier_value>
                                            </INSDQualifier>
                                    </INSDFeature_quals>
                            </INSDFeature>
                    </INSDSeq_feature-table>
                    <INSDSeq_sequence>NVEQIL</INSDSeq_sequence>
            </INSDSeq>
        </SequenceData>
        <SequenceData sequenceIDNumber="24">
            <INSDSeq>
                    <INSDSeq_length>7</INSDSeq_length>
                    <INSDSeq_moltype>AA</INSDSeq_moltype>
                    <INSDSeq_division>PAT</INSDSeq_division>
                    <INSDSeq_feature-table>
                            <INSDFeature>
                                    <INSDFeature_key>
source</INSDFeature_key>

            <INSDFeature_location>1..7</INSDFeature_location>
                                    <INSDFeature_quals>
                                            <INSDQualifier>
                                                    <INSDQualifier_name>
mol_type</INSDQualifier_name>
                                                    <INSDQualifier_value>
protein</INSDQualifier_value>
                                            </INSDQualifier>
                                            <INSDQualifier id="q25">
                                                    <INSDQualifier_name>
organism</INSDQualifier_name>
                                                    <INSDQualifier_value>
synthetic construct</INSDQualifier_value>
                                            </INSDQualifier>
                                    </INSDFeature_quals>
                            </INSDFeature>
                    </INSDSeq_feature-table>
                    <INSDSeq_sequence>MHKFRYW</INSDSeq_sequence>
            </INSDSeq>
```

```
        </SequenceData>
        <SequenceData sequenceIDNumber="25">
                <INSDSeq>
                        <INSDSeq_length>8</INSDSeq_length>
                        <INSDSeq_moltype>AA</INSDSeq_moltype>
                        <INSDSeq_division>PAT</INSDSeq_division>
                        <INSDSeq_feature-table>
                                <INSDFeature>
                                        <INSDFeature_key>
source</INSDFeature_key>

        <INSDFeature_location>1..8</INSDFeature_location>
                                        <INSDFeature_quals>
                                                <INSDQualifier>
                                                        <INSDQualifier_name>
mol_type</INSDQualifier_name>
                                                        <INSDQualifier_value>
protein</INSDQualifier_value>
                                                </INSDQualifier>
                                                <INSDQualifier id="q26">
                                                        <INSDQualifier_name>
organism</INSDQualifier_name>
                                                        <INSDQualifier_value>
synthetic construct</INSDQualifier_value>
                                                </INSDQualifier>
                                        </INSDFeature_quals>
                                </INSDFeature>
                        </INSDSeq_feature-table>
                        <INSDSeq_sequence>LIFWCMVY</INSDSeq_sequence>
                </INSDSeq>
        </SequenceData>
        <SequenceData sequenceIDNumber="26">
                <INSDSeq>
                        <INSDSeq_length>5</INSDSeq_length>
                        <INSDSeq_moltype>AA</INSDSeq_moltype>
                        <INSDSeq_division>PAT</INSDSeq_division>
                        <INSDSeq_feature-table>
                                <INSDFeature>
                                        <INSDFeature_key>
source</INSDFeature_key>

        <INSDFeature_location>1..5</INSDFeature_location>
                                        <INSDFeature_quals>
                                                <INSDQualifier>
                                                        <INSDQualifier_name>
mol_type</INSDQualifier_name>
                                                        <INSDQualifier_value>
protein</INSDQualifier_value>
                                                </INSDQualifier>
                                                <INSDQualifier id="q27">
                                                        <INSDQualifier_name>
organism</INSDQualifier_name>
                                                        <INSDQualifier_value>
synthetic construct</INSDQualifier_value>
                                                </INSDQualifier>
                                        </INSDFeature_quals>
                                </INSDFeature>
```

```
                </INSDSeq_feature-table>
                <INSDSeq_sequence>PATGS</INSDSeq_sequence>
            </INSDSeq>
        </SequenceData>
        <SequenceData sequenceIDNumber="27">
            <INSDSeq>
                <INSDSeq_length>7</INSDSeq_length>
                <INSDSeq_moltype>AA</INSDSeq_moltype>
                <INSDSeq_division>PAT</INSDSeq_division>
                <INSDSeq_feature-table>
                    <INSDFeature>
                        <INSDFeature_key>
source</INSDFeature_key>

        <INSDFeature_location>1..7</INSDFeature_location>
                        <INSDFeature_quals>
                            <INSDQualifier>
                                <INSDQualifier_name>
mol_type</INSDQualifier_name>
                                <INSDQualifier_value>
protein</INSDQualifier_value>
                            </INSDQualifier>
                            <INSDQualifier id="q28">
                                <INSDQualifier_name>
organism</INSDQualifier_name>
                                <INSDQualifier_value>
synthetic construct</INSDQualifier_value>
                            </INSDQualifier>
                        </INSDFeature_quals>
                    </INSDFeature>
                </INSDSeq_feature-table>
                <INSDSeq_sequence>HQRKNED</INSDSeq_sequence>
            </INSDSeq>
        </SequenceData>
        <SequenceData sequenceIDNumber="28">
            <INSDSeq>
                <INSDSeq_length>5</INSDSeq_length>
                <INSDSeq_moltype>AA</INSDSeq_moltype>
                <INSDSeq_division>PAT</INSDSeq_division>
                <INSDSeq_feature-table>
                    <INSDFeature>
                        <INSDFeature_key>
source</INSDFeature_key>

        <INSDFeature_location>1..5</INSDFeature_location>
                        <INSDFeature_quals>
                            <INSDQualifier>
                                <INSDQualifier_name>
mol_type</INSDQualifier_name>
                                <INSDQualifier_value>
protein</INSDQualifier_value>
                            </INSDQualifier>
                            <INSDQualifier id="q29">
                                <INSDQualifier_name>
organism</INSDQualifier_name>
                                <INSDQualifier_value>
synthetic construct</INSDQualifier_value>
```

```
                                        </INSDQualifier>
                                    </INSDFeature_quals>
                                </INSDFeature>
                            </INSDSeq_feature-table>
                            <INSDSeq_sequence>GASDT</INSDSeq_sequence>
                        </INSDSeq>
                </SequenceData>
                <SequenceData sequenceIDNumber="29">
                    <INSDSeq>
                            <INSDSeq_length>8</INSDSeq_length>
                            <INSDSeq_moltype>AA</INSDSeq_moltype>
                            <INSDSeq_division>PAT</INSDSeq_division>
                            <INSDSeq_feature-table>
                                <INSDFeature>
                                    <INSDFeature_key>
source</INSDFeature_key>

        <INSDFeature_location>1..8</INSDFeature_location>
                                    <INSDFeature_quals>
                                        <INSDQualifier>
                                            <INSDQualifier_name>
mol_type</INSDQualifier_name>
                                            <INSDQualifier_value>
protein</INSDQualifier_value>
                                        </INSDQualifier>
                                        <INSDQualifier id="q30">
                                            <INSDQualifier_name>
organism</INSDQualifier_name>
                                            <INSDQualifier_value>
synthetic construct</INSDQualifier_value>
                                        </INSDQualifier>
                                    </INSDFeature_quals>
                                </INSDFeature>
                            </INSDSeq_feature-table>
                            <INSDSeq_sequence>CPNVEQIL</INSDSeq_sequence>
                        </INSDSeq>
                </SequenceData>
                <SequenceData sequenceIDNumber="30">
                    <INSDSeq>
                            <INSDSeq_length>7</INSDSeq_length>
                            <INSDSeq_moltype>AA</INSDSeq_moltype>
                            <INSDSeq_division>PAT</INSDSeq_division>
                            <INSDSeq_feature-table>
                                <INSDFeature>
                                    <INSDFeature_key>
source</INSDFeature_key>

        <INSDFeature_location>1..7</INSDFeature_location>
                                    <INSDFeature_quals>
                                        <INSDQualifier>
                                            <INSDQualifier_name>
mol_type</INSDQualifier_name>
                                            <INSDQualifier_value>
protein</INSDQualifier_value>
                                        </INSDQualifier>
                                        <INSDQualifier id="q31">
                                            <INSDQualifier_name>
```

```
organism</INSDQualifier_name>
                                    <INSDQualifier_value>
synthetic construct</INSDQualifier_value>
                                </INSDQualifier>
                            </INSDFeature_quals>
                        </INSDFeature>
                    </INSDSeq_feature-table>
                    <INSDSeq_sequence>KMHFRYW</INSDSeq_sequence>
                </INSDSeq>
            </SequenceData>
            <SequenceData sequenceIDNumber="31">
                <INSDSeq>
                    <INSDSeq_length>16</INSDSeq_length>
                    <INSDSeq_moltype>AA</INSDSeq_moltype>
                    <INSDSeq_division>PAT</INSDSeq_division>
                    <INSDSeq_feature-table>
                        <INSDFeature>
                            <INSDFeature_key>
source</INSDFeature_key>

        <INSDFeature_location>1..16</INSDFeature_location>
                            <INSDFeature_quals>
                                <INSDQualifier>
                                    <INSDQualifier_name>
mol_type</INSDQualifier_name>
                                    <INSDQualifier_value>
protein</INSDQualifier_value>
                                </INSDQualifier>
                                <INSDQualifier id="q32">
                                    <INSDQualifier_name>
organism</INSDQualifier_name>
                                    <INSDQualifier_value>
synthetic construct</INSDQualifier_value>
                                </INSDQualifier>
                            </INSDFeature_quals>
                        </INSDFeature>
                    </INSDSeq_feature-table>
                    <INSDSeq_sequence>
ANCQGHILMFPSTWYV</INSDSeq_sequence>
                </INSDSeq>
            </SequenceData>
            <SequenceData sequenceIDNumber="32">
                <INSDSeq>
                    <INSDSeq_length>8</INSDSeq_length>
                    <INSDSeq_moltype>AA</INSDSeq_moltype>
                    <INSDSeq_division>PAT</INSDSeq_division>
                    <INSDSeq_feature-table>
                        <INSDFeature>
                            <INSDFeature_key>
source</INSDFeature_key>

        <INSDFeature_location>1..8</INSDFeature_location>
                            <INSDFeature_quals>
                                <INSDQualifier>
                                    <INSDQualifier_name>
mol_type</INSDQualifier_name>
                                    <INSDQualifier_value>
```

```
protein</INSDQualifier_value>
                                    </INSDQualifier>
                                    <INSDQualifier id="q33">
                                        <INSDQualifier_name>
organism</INSDQualifier_name>
                                        <INSDQualifier_value>
synthetic construct</INSDQualifier_value>
                                    </INSDQualifier>
                                </INSDFeature_quals>
                            </INSDFeature>
                        </INSDSeq_feature-table>
                        <INSDSeq_sequence>EALMQKRH</INSDSeq_sequence>
                    </INSDSeq>
            </SequenceData>
            <SequenceData sequenceIDNumber="33">
                <INSDSeq>
                        <INSDSeq_length>7</INSDSeq_length>
                        <INSDSeq_moltype>AA</INSDSeq_moltype>
                        <INSDSeq_division>PAT</INSDSeq_division>
                        <INSDSeq_feature-table>
                            <INSDFeature>
                                <INSDFeature_key>
source</INSDFeature_key>

        <INSDFeature_location>1..7</INSDFeature_location>
                                <INSDFeature_quals>
                                    <INSDQualifier>
                                        <INSDQualifier_name>
mol_type</INSDQualifier_name>
                                        <INSDQualifier_value>
protein</INSDQualifier_value>
                                    </INSDQualifier>
                                    <INSDQualifier id="q34">
                                        <INSDQualifier_name>
organism</INSDQualifier_name>
                                        <INSDQualifier_value>
synthetic construct</INSDQualifier_value>
                                    </INSDQualifier>
                                </INSDFeature_quals>
                            </INSDFeature>
                        </INSDSeq_feature-table>
                        <INSDSeq_sequence>VIYCWFT</INSDSeq_sequence>
                    </INSDSeq>
            </SequenceData>
            <SequenceData sequenceIDNumber="34">
                <INSDSeq>
                        <INSDSeq_length>5</INSDSeq_length>
                        <INSDSeq_moltype>AA</INSDSeq_moltype>
                        <INSDSeq_division>PAT</INSDSeq_division>
                        <INSDSeq_feature-table>
                            <INSDFeature>
                                <INSDFeature_key>
source</INSDFeature_key>

        <INSDFeature_location>1..5</INSDFeature_location>
                                <INSDFeature_quals>
                                    <INSDQualifier>
```

```
                                            <INSDQualifier_name>
mol_type</INSDQualifier_name>
                                            <INSDQualifier_value>
protein</INSDQualifier_value>
                                        </INSDQualifier>
                                        <INSDQualifier id="q35">
                                            <INSDQualifier_name>
organism</INSDQualifier_name>
                                            <INSDQualifier_value>
synthetic construct</INSDQualifier_value>
                                        </INSDQualifier>
                                    </INSDFeature_quals>
                                </INSDFeature>
                            </INSDSeq_feature-table>
                            <INSDSeq_sequence>GNPSD</INSDSeq_sequence>
                    </INSDSeq>
                </SequenceData>
                <SequenceData sequenceIDNumber="35">
                    <INSDSeq>
                            <INSDSeq_length>8</INSDSeq_length>
                            <INSDSeq_moltype>AA</INSDSeq_moltype>
                            <INSDSeq_division>PAT</INSDSeq_division>
                            <INSDSeq_feature-table>
                                <INSDFeature>
                                    <INSDFeature_key>
source</INSDFeature_key>

        <INSDFeature_location>1..8</INSDFeature_location>
                                    <INSDFeature_quals>
                                        <INSDQualifier>
                                            <INSDQualifier_name>
mol_type</INSDQualifier_name>
                                            <INSDQualifier_value>
protein</INSDQualifier_value>
                                        </INSDQualifier>
                                        <INSDQualifier id="q36">
                                            <INSDQualifier_name>
organism</INSDQualifier_name>
                                            <INSDQualifier_value>
synthetic construct</INSDQualifier_value>
                                        </INSDQualifier>
                                    </INSDFeature_quals>
                                </INSDFeature>
                            </INSDSeq_feature-table>
                            <INSDSeq_sequence>ALFCGIVW</INSDSeq_sequence>
                    </INSDSeq>
                </SequenceData>
                <SequenceData sequenceIDNumber="36">
                    <INSDSeq>
                            <INSDSeq_length>6</INSDSeq_length>
                            <INSDSeq_moltype>AA</INSDSeq_moltype>
                            <INSDSeq_division>PAT</INSDSeq_division>
                            <INSDSeq_feature-table>
                                <INSDFeature>
                                    <INSDFeature_key>
source</INSDFeature_key>
```

```
<INSDFeature_location>1..6</INSDFeature_location>
                        <INSDFeature_quals>
                                <INSDQualifier>
                                        <INSDQualifier_name>
mol_type</INSDQualifier_name>
                                        <INSDQualifier_value>
protein</INSDQualifier_value>
                                </INSDQualifier>
                                <INSDQualifier id="q37">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
        </INSDSeq_feature-table>
        <INSDSeq_sequence>RKQEND</INSDSeq_sequence>
    </INSDSeq>
</SequenceData>
<SequenceData sequenceIDNumber="37">
    <INSDSeq>
        <INSDSeq_length>6</INSDSeq_length>
        <INSDSeq_moltype>AA</INSDSeq_moltype>
        <INSDSeq_division>PAT</INSDSeq_division>
        <INSDSeq_feature-table>
                <INSDFeature>
                        <INSDFeature_key>
source</INSDFeature_key>

<INSDFeature_location>1..6</INSDFeature_location>
                        <INSDFeature_quals>
                                <INSDQualifier>
                                        <INSDQualifier_name>
mol_type</INSDQualifier_name>
                                        <INSDQualifier_value>
protein</INSDQualifier_value>
                                </INSDQualifier>
                                <INSDQualifier id="q38">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
        </INSDSeq_feature-table>
        <INSDSeq_sequence>MSPTHY</INSDSeq_sequence>
    </INSDSeq>
</SequenceData>
<SequenceData sequenceIDNumber="38">
    <INSDSeq>
        <INSDSeq_length>11</INSDSeq_length>
        <INSDSeq_moltype>AA</INSDSeq_moltype>
        <INSDSeq_division>PAT</INSDSeq_division>
        <INSDSeq_feature-table>
                <INSDFeature>
```

```
                                        <INSDFeature_key>
source</INSDFeature_key>

                    <INSDFeature_location>1..11</INSDFeature_location>
                                        <INSDFeature_quals>
                                            <INSDQualifier>
                                                <INSDQualifier_name>
mol_type</INSDQualifier_name>
                                                <INSDQualifier_value>
protein</INSDQualifier_value>
                                            </INSDQualifier>
                                            <INSDQualifier id="q39">
                                                <INSDQualifier_name>
organism</INSDQualifier_name>
                                                <INSDQualifier_value>
synthetic construct</INSDQualifier_value>
                                            </INSDQualifier>
                                        </INSDFeature_quals>
                                    </INSDFeature>
                                </INSDSeq_feature-table>
                                <INSDSeq_sequence>
MLKKRFLGALA</INSDSeq_sequence>
                            </INSDSeq>
                        </SequenceData>
                        <SequenceData sequenceIDNumber="39">
                            <INSDSeq>
                                <INSDSeq_length>10</INSDSeq_length>
                                <INSDSeq_moltype>DNA</INSDSeq_moltype>
                                <INSDSeq_division>PAT</INSDSeq_division>
                                <INSDSeq_feature-table>
                                    <INSDFeature>
                                        <INSDFeature_key>
source</INSDFeature_key>

                    <INSDFeature_location>1..10</INSDFeature_location>
                                        <INSDFeature_quals>
                                            <INSDQualifier>
                                                <INSDQualifier_name>
mol_type</INSDQualifier_name>
                                                <INSDQualifier_value>
other DNA</INSDQualifier_value>
                                            </INSDQualifier>
                                            <INSDQualifier id="q40">
                                                <INSDQualifier_name>
organism</INSDQualifier_name>
                                                <INSDQualifier_value>
synthetic construct</INSDQualifier_value>
                                            </INSDQualifier>
                                        </INSDFeature_quals>
                                    </INSDFeature>
                                </INSDSeq_feature-table>
                                <INSDSeq_sequence>
acgttgactt</INSDSeq_sequence>
                            </INSDSeq>
                        </SequenceData>
                        <SequenceData sequenceIDNumber="40">
                            <INSDSeq>
```

```
<INSDSeq_length>10</INSDSeq_length>
<INSDSeq_moltype>DNA</INSDSeq_moltype>
<INSDSeq_division>PAT</INSDSeq_division>
<INSDSeq_feature-table>
        <INSDFeature>
                <INSDFeature_key>
source</INSDFeature_key>

        <INSDFeature_location>1..10</INSDFeature_location>
                <INSDFeature_quals>
                        <INSDQualifier>
                                <INSDQualifier_name>
mol_type</INSDQualifier_name>
                                <INSDQualifier_value>
other DNA</INSDQualifier_value>
                        </INSDQualifier>
                        <INSDQualifier id="q41">
                                <INSDQualifier_name>
organism</INSDQualifier_name>
                                <INSDQualifier_value>
synthetic construct</INSDQualifier_value>
                        </INSDQualifier>
                </INSDFeature_quals>
        </INSDFeature>
</INSDSeq_feature-table>
<INSDSeq_sequence>
agctgtactt</INSDSeq_sequence>
        </INSDSeq>
</SequenceData>
<SequenceData sequenceIDNumber="41">
        <INSDSeq>
                <INSDSeq_length>10</INSDSeq_length>
                <INSDSeq_moltype>DNA</INSDSeq_moltype>
                <INSDSeq_division>PAT</INSDSeq_division>
                <INSDSeq_feature-table>
                        <INSDFeature>
                                <INSDFeature_key>
source</INSDFeature_key>

        <INSDFeature_location>1..10</INSDFeature_location>
                        <INSDFeature_quals>
                                <INSDQualifier>
                                        <INSDQualifier_name>
mol_type</INSDQualifier_name>
                                        <INSDQualifier_value>
other DNA</INSDQualifier_value>
                                </INSDQualifier>
                                <INSDQualifier id="q42">
                                        <INSDQualifier_name>
organism</INSDQualifier_name>
                                        <INSDQualifier_value>
synthetic construct</INSDQualifier_value>
                                </INSDQualifier>
                        </INSDFeature_quals>
                </INSDFeature>
        </INSDSeq_feature-table>
        <INSDSeq_sequence>
```

```
gccgagacta</INSDSeq_sequence>
                </INSDSeq>
        </SequenceData>
        <SequenceData sequenceIDNumber="42">
                <INSDSeq>
                        <INSDSeq_length>10</INSDSeq_length>
                        <INSDSeq_moltype>DNA</INSDSeq_moltype>
                        <INSDSeq_division>PAT</INSDSeq_division>
                        <INSDSeq_feature-table>
                                <INSDFeature>
                                        <INSDFeature_key>
source</INSDFeature_key>

        <INSDFeature_location>1..10</INSDFeature_location>
                                        <INSDFeature_quals>
                                                <INSDQualifier>
                                                        <INSDQualifier_name>
mol_type</INSDQualifier_name>
                                                        <INSDQualifier_value>
other DNA</INSDQualifier_value>
                                                </INSDQualifier>
                                                <INSDQualifier id="q43">
                                                        <INSDQualifier_name>
organism</INSDQualifier_name>
                                                        <INSDQualifier_value>
synthetic construct</INSDQualifier_value>
                                                </INSDQualifier>
                                        </INSDFeature_quals>
                                </INSDFeature>
                        </INSDSeq_feature-table>
                        <INSDSeq_sequence>
gagtcacatc</INSDSeq_sequence>
                </INSDSeq>
        </SequenceData>
        <SequenceData sequenceIDNumber="43">
                <INSDSeq>
                        <INSDSeq_length>10</INSDSeq_length>
                        <INSDSeq_moltype>DNA</INSDSeq_moltype>
                        <INSDSeq_division>PAT</INSDSeq_division>
                        <INSDSeq_feature-table>
                                <INSDFeature>
                                        <INSDFeature_key>
source</INSDFeature_key>

        <INSDFeature_location>1..10</INSDFeature_location>
                                        <INSDFeature_quals>
                                                <INSDQualifier>
                                                        <INSDQualifier_name>
mol_type</INSDQualifier_name>
                                                        <INSDQualifier_value>
other DNA</INSDQualifier_value>
                                                </INSDQualifier>
                                                <INSDQualifier id="q44">
                                                        <INSDQualifier_name>
organism</INSDQualifier_name>
                                                        <INSDQualifier_value>
synthetic construct</INSDQualifier_value>
```

```
                                                </INSDQualifier>
                                            </INSDFeature_quals>
                                        </INSDFeature>
                                    </INSDSeq_feature-table>
                                    <INSDSeq_sequence>
agtcctgaca</INSDSeq_sequence>
                            </INSDSeq>
                    </SequenceData>
                    <SequenceData sequenceIDNumber="44">
                            <INSDSeq>
                                    <INSDSeq_length>22</INSDSeq_length>
                                    <INSDSeq_moltype>AA</INSDSeq_moltype>
                                    <INSDSeq_division>PAT</INSDSeq_division>
                                    <INSDSeq_feature-table>
                                        <INSDFeature>
                                            <INSDFeature_key>
source</INSDFeature_key>

                    <INSDFeature_location>1..22</INSDFeature_location>
                                                <INSDFeature_quals>
                                                    <INSDQualifier>
                                                        <INSDQualifier_name>
mol_type</INSDQualifier_name>
                                                            <INSDQualifier_value>
protein</INSDQualifier_value>
                                                    </INSDQualifier>
                                                    <INSDQualifier id="q45">
                                                        <INSDQualifier_name>
organism</INSDQualifier_name>
                                                            <INSDQualifier_value>
synthetic construct</INSDQualifier_value>
                                                    </INSDQualifier>
                                                </INSDFeature_quals>
                                        </INSDFeature>
                                    </INSDSeq_feature-table>
                                    <INSDSeq_sequence>
EVQLVESGGGLVQPGRSLRLSC</INSDSeq_sequence>
                            </INSDSeq>
                    </SequenceData>
                    <SequenceData sequenceIDNumber="45">
                            <INSDSeq>
                                    <INSDSeq_length>27</INSDSeq_length>
                                    <INSDSeq_moltype>AA</INSDSeq_moltype>
                                    <INSDSeq_division>PAT</INSDSeq_division>
                                    <INSDSeq_feature-table>
                                        <INSDFeature>
                                            <INSDFeature_key>
source</INSDFeature_key>

                    <INSDFeature_location>1..27</INSDFeature_location>
                                                <INSDFeature_quals>
                                                    <INSDQualifier>
                                                        <INSDQualifier_name>
mol_type</INSDQualifier_name>
                                                            <INSDQualifier_value>
protein</INSDQualifier_value>
                                                    </INSDQualifier>
```

```
                                        <INSDQualifier id="q46">
                                            <INSDQualifier_name>
organism</INSDQualifier_name>
                                            <INSDQualifier_value>
synthetic construct</INSDQualifier_value>
                                        </INSDQualifier>
                                    </INSDFeature_quals>
                                </INSDFeature>
                        </INSDSeq_feature-table>
                        <INSDSeq_sequence>
ACDEFKGPFLVQNWMYGRHITPKKLVN</INSDSeq_sequence>
                </INSDSeq>
        </SequenceData>
        <SequenceData sequenceIDNumber="46">
                <INSDSeq>
                        <INSDSeq_length>703</INSDSeq_length>
                        <INSDSeq_moltype>AA</INSDSeq_moltype>
                        <INSDSeq_division>PAT</INSDSeq_division>
                        <INSDSeq_feature-table>
                                <INSDFeature>
                                    <INSDFeature_key>
source</INSDFeature_key>

                                    <INSDFeature_location>1..703</INSDFeature_location>
                                    <INSDFeature_quals>
                                        <INSDQualifier>
                                            <INSDQualifier_name>
mol_type</INSDQualifier_name>
                                            <INSDQualifier_value>
protein</INSDQualifier_value>
                                        </INSDQualifier>
                                        <INSDQualifier id="q47">
                                            <INSDQualifier_name>
organism</INSDQualifier_name>
                                            <INSDQualifier_value>
synthetic construct</INSDQualifier_value>
                                        </INSDQualifier>
                                    </INSDFeature_quals>
                                </INSDFeature>
                        </INSDSeq_feature-table>
                        <INSDSeq_sequence>
MTRLTVLALLAGLLASSRAEVQLVESGGGLVQPGRSLRLSCAASGFTFDDYAMHWVRQAPGK
GLEWVSAITWNSGHIDYADSVEGRFTISRDNAKNSLYLQMNSLRAEDTAVYYCAKVSYLSTA
SSLDYWGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDKTHT
CPPCPAPELLGGPSVFLFPPKPKDTLMISRTPEVTCVVVDVSHEDPEVKFNWYVDGVEVHNA
KTKPREEQYNSTYRVVSVLTVLHQDWLNGKEYKCKVSNKALPAPIEKTISKAKGQPREPQVY
TLPPSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGKMTRLTVLALLAGLLASSRADIQMTQS
PSSLSASVGDRVTITCRASQGIRNYLAWYQQKPGKAPKLLIYAASTLQSGVPSRFSGSGSGT
DFTLTISSLQPEDVATYYCQRYNRAPYTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYA
CEVTHQGLSSPVTKSFNRGEC</INSDSeq_sequence>
                </INSDSeq>
        </SequenceData>
</ST26SequenceListing>
```

**Claims**

1. A method for optimizing protein expression,

   wherein each one of a plurality of prediction algorithms includes a combination of an encoding algorithm, a dimensionality reduction algorithm and a regression algorithm, the combination being different from a combination included in any other one of the plurality of prediction algorithms,
   wherein the method comprises:

   obtaining, by a processor (102), a plurality of amino acid sequences and corresponding known efficiency values, each known efficiency value indicating efficiency of expressing a protein having a corresponding one of the plurality of amino acid sequences;
   for each one of the plurality of prediction algorithms,

   for each one of a plurality of amino acid sequences, generating, by the processor (102), a first numerical vector corresponding to the one of the plurality of amino acid sequences by encoding at least part of the one of the plurality of amino acid sequences according to the encoding algorithm included in the one of the plurality of prediction algorithms,
   generating, by the processor (102), second numerical vectors corresponding to the plurality of amino acid sequences by applying the dimensionality reduction algorithm included in the one of the plurality of prediction algorithms to the first numerical vectors corresponding to the plurality of amino acid sequences, wherein a dimension of the second numerical vectors is smaller than a dimension of the first numerical vectors,
   obtaining, by the processor (102), a prediction function by processing, according to the regression algorithm included in the one of the plurality of prediction algorithms, at least part of the second numerical vectors and the known efficiency values for the amino acid sequences corresponding to the at least part of the second numerical vectors, wherein the prediction function outputs a predicted efficiency value for expressing a protein having an amino acid sequence corresponding to an input numerical vector, and
   evaluating, by the processor (102), the obtained prediction function by comparing predicted efficiency values output by the obtained prediction function for at least part of the second numerical vectors with the known efficiency values for the amino acid sequences corresponding to the at least part of the second numerical vectors;

   selecting, by the processor (102), at least one prediction algorithm from among the plurality of prediction algorithms based on said evaluating;
   predicting, by the processor (102), using the at least one prediction algorithm and the prediction function obtained with the at least one prediction algorithm, one or more efficiency values for expressing one or more proteins respectively having one or more specified amino acid sequences; and
   outputting, by the processor (102), the one or more specified amino acid sequences and the one or more efficiency values predicted for the one or more specified amino acid sequences.

2. The method according to claim 1, further comprising:

   identifying, by the processor (102), one of the one or more specified amino acid sequences for which a highest efficiency value is predicted; and
   outputting, by the processor (102), information indicating that the identified one of the one or more specified amino acid sequences has the highest predicted efficiency value.

3. The method according to claim 1 or 2, wherein the plurality of amino acid sequences are antibody sequences or recombinant antibody sequences.

4. The method according to claim 3, wherein the plurality of amino acid sequences are light chain sequences, heavy chain sequences or light-heavy chain sequences of antibodies or recombinant antibodies.

5. The method according to any one of claims 1 to 4, wherein the at least part of the one of the plurality of amino acid sequences includes:

a first x % of the one of the plurality of amino acid sequences; and/or
a last y % of the one of the plurality of amino acid sequences,
wherein each of x and y is a value greater than 0 and less than or equal to 50,
wherein x is preferably 25 or 50 and y is preferably 25 or 50.

6. The method according to any one of claims 1 to 5, wherein each known efficiency value indicates a titer for expressing the protein having the corresponding one of the plurality of amino acid sequences; and
wherein the predicted efficiency value output by the prediction function indicates a predicted titer.

7. The method according to any one of claims 1 to 6, wherein said generating of the second numerical vectors comprises:

generating, by the processor (102), a plurality of sets of the second numerical vectors using the dimensionality reduction algorithm,
wherein the second numerical vectors included in a same set of the plurality of sets of the second numerical vectors have a same dimension,
wherein the second numerical vectors included in different sets of the plurality of sets of the second numerical vectors have different dimensions, and
wherein said obtaining the prediction function and said evaluating the prediction function are performed for each one of the plurality of sets of the second numerical vectors.

8. The method according to any one of claims 1 to 7, wherein said evaluating of the obtained prediction function comprises:
determining, by the processor (102), one or more of the following performance metrics for the obtained prediction function:

accuracy,
precision,
recall,
F1-score.

9. The method according to any one of claims 1 to 8, wherein the combination of the encoding algorithm, the dimensionality reduction algorithm and the regression algorithm is a combination of the following:

as the encoding algorithm, a k-mer based encoding algorithm, a counting based encoding algorithm, a K-gap based encoding algorithm, a window-based encoding algorithm, a group-based encoding algorithm, a physico-chemical property based encoding algorithm, a word embedding based encoding algorithm;
as the dimensionality reduction algorithm, principal component analysis, PCA, K-means, t-distributed stochastic neighbor embedding, TSNE, kernel-PCA, locally-linear embedding, LLE, tensor singular value decomposition, T-SVD, non-negative matrix factorization, NMF, multi-dimensional scaling, MDS, factor analysis, agglomerate feature, Gaussian random projection, sparse random projection or fast independent component analysis, fast-ICA; and
as the regression algorithm, linear regression, non-linear regression, penalized linear regression, penalized non-linear regression, naive Bayes, bagging regression, random forest regressor, boosting regression, partial least square regression or support vector machine.

10. The method according to claim 9, wherein said encoding of the at least part of the one of the plurality of amino acid sequences according to the physico-chemical property based algorithm as the encoding algorithm comprises:

assigning, by the processor (102), weight values to individual amino acids based on one or more physico-chemical properties of proteins; and
generating, by the processor (102), the first numerical vector including the weights corresponding to the amino acids included in the at least part of the one of the plurality of amino acid sequences in an order in the at least part of the one or the plurality of amino acid sequences,
wherein the one or more physico-chemical properties include one or more of the following:

dissociation,
solubility,
hydration,

polarity,
charge,
hydrophobicity,
molecular weight,
size.

11. The method according to claim 9, wherein said encoding of the at least part of the one of the plurality of amino acid sequences according to the k-mer based encoding algorithm as the encoding algorithm comprises:

determining, by the processor (102), k-mers of the at least part of the one of the plurality of amino acid sequences;
determining, by the processor (102), a weight for each one of the k-mers based at least on:

how many times the one of the k-mers appears in the at least part of the one of the plurality of amino acid sequences, and
how many times the one of the k-mers appears in the at least part of the plurality of amino acid sequences;

generating, by the processor (102), the first numerical vector including the weights of the k-mers in an order of the k-mers appearing in the at least part of the one of the plurality of amino acid sequences.

12. The method according to any one of claims 1 to 11, further comprising:
using, in an automated cell culture system (20), one or more nucleic acids that respectively encode the one or more specified amino acid sequences to express one or more proteins respectively including the one or more specified amino acid sequences.

13. A computer program product comprising computer-readable instructions that, when loaded and run on a computer, cause the computer to perform the method according to any one of claims 1 to 11.

14. A prediction system (10) for predicting efficiency of protein expression, the prediction system comprising:

a processor (102) configured to perform the method according to any one of claims 1 to 11;
a storage medium (104) that is in communication with the processor and that is configured to store the plurality of amino acid sequences and the corresponding known efficiency values.

15. A system for optimizing protein expression, the system comprising:

the prediction system (10) according to claim 14; and
an automated cell culture system (20) that is capable of expressing the one or more proteins.

FIG 1

**FIG 2**

EP 4 310 848 A1

10

Prediction System

102

Processor

104

Storage Medium

20

Automated Cell
Culture System

**FIG 3**

START

S10 — Obtain amino acid sequences and corresponding known effiiciency values

S20 — Estimate and evaluate prediction functions according to prediction algorithms using the obtained amino acid sequences and known efficiency values

S30 — Select at least one prediction algorithm from among the prediction algorithms based on the evaluation

S40 — Predict one or more efficiency values for expressing one or more proteins respectively having one or more specified amino acid sequences

S50 — Output the one or more specified amino acid sequences and the one or more predicted efficiency values

END

**FIG 4**

EP 4 310 848 A1

S200      S202      S204      S206      S208

```
┌──────────┐   ┌──────────┐   ┌───────────────┐   ┌────────────┐   ┌────────────┐
│  Input   │→→ │ Sequence │→→ │ Dimensionality│→→ │  Machine   │→→ │ Evaluation │
│ Sequence │   │ Encoding │   │   Reduction   │   │  Learning  │   │            │
│          │   │          │   │               │   │ Regression │   │            │
└──────────┘   └──────────┘   └───────────────┘   └────────────┘   └────────────┘
```

**FIG 5**

Antibody Sequences

Sub-sequence Types

| Full-HC | Full-HCLC | Full-LC |
|---|---|---|

| S-25 | *XX* | E-25 |
|---|---|---|

Sub-sequence (a)

| S-25 | *XX* | E-25 | S-50 | *XX* |
|---|---|---|---|---|

Sub-sequence (c)

| S-50 | *XX* |
|---|---|

Sub-sequence (b)

**FIG 6**

| A | C | D | E | F | K | G | P | F | L | V | Q | N | W | M | Y | G | R | H | I | T | P | K | K | L | V | N |

Encode each sequence with each physiochemical property

⬇

Encode each character of sequence with physiochemical property value

⬇

Encoding dimension of each sequence: physiochemical properties * length_of_sequence

## AAindex 531 physiochemical Properties

| AccNo | A | R | N | D | C | Q | E | G | H | I | L | K | M | F | P | S | T | W | Y | V |
|---|---|---|---|---|---|---|---|---|---|---|---|---|---|---|---|---|---|---|---|---|
| ANDN920101 | 4.35 | 4.38 | 4.75 | 4.76 | 4.65 | 4.37 | 4.29 | 3.97 | 4.63 | 3.95 | 4.17 | 4.36 | 4.52 | 4.66 | 4.44 | 4.5 | 4.35 | 4.7 | 4.6 | 3.95 |
| ARGP820101 | 0.61 | 0.6 | 0.06 | 0.46 | 1.07 | 0 | 0.47 | 0.07 | 0.61 | 2.22 | 1.53 | 1.15 | 1.18 | 2.02 | 1.95 | 0.05 | 0.05 | 2.65 | 1.88 | 1.32 |

*dimension vector = length of sequence \* properties*

**FIG 7A**

**HC** *sequence*

| A | C | D | E | F | K | G | P | F | L | V | Q | N | W | M | Y | G | R | H | I | T | P | K | K | L | V | N |
|---|---|---|---|---|---|---|---|---|---|---|---|---|---|---|---|---|---|---|---|---|---|---|---|---|---|---|

For each property in Group1 and Group2

*Group* 1

*Group* 2

$$Encoding(group\_1[property])$$
$$= \frac{count\ of\ all\ amino\ acids\ of\ group_1[property]\ in\ HC\ sequence}{total\ length\ of\ sequence}$$

$$Encoding(group\_2[property])$$
$$= \frac{count\ of\ all\ amino\ acids\ of\ group_2[property]\ in\ HC\ sequence}{total\ length\ of\ sequence}$$

$$\textbf{\textit{Group} 3} = 1 - Encoding(group\_1[property]) - Encoding(group\_2[property])$$

$$dimension\ vector = Properties\ (13) * groups\ (3)$$

## FIG 7B

EP 4 310 848 A1

## Protein name: OTS1

**Titer:** 2.19788085106383

**Sequence (HCLC-Full):** (SEQ ID NO: 46)
MTRLTVLALLAGLLASSRAEVQLVESGGGLVQPG
RSLRLSCAASGFTFDDYAMHWVRQAPGKGLEW
VSAITWNSGHIDYADSVEGRFTISRDNAKNSLYLQ
MNSLRAEDTAVYYCAKVSYLSTASSLDYWGQGT
LVTVSSASTKGPSVFPLAPSSKSTSGGTAALGCL
VKDYFPEPVTVSWNSGALTSGVHTFPAVLQSSGL
YSLSSVVTVPSSSLGTQTYICNVNHKPSNTKVDK
KVEPKSCDKTHTCPPCPAPELLGGPSVFLFPPKP
KDTLMISRTPEVTCVVVDVSHEDPEVKFNWYVD
GVEVHNAKTKPREEQYNSTYRVVSVLTVLHQDW
LNGKEYKCKVSNKALPAPIEKTISKAKGQPREPQ
VYTLPPSRDELTKNQVSLTCLVKGFYPSDIAVEWE
SNGQPENNYKTTPPVLDSDGSFFLYSKLTVDKSR
WQQGNVFSCSVMHEALHNHYTQKSLSLSPGKM
TRLTVLALLAGLLASSRADIQMTQSPSSLSASVGD
RVTITCRASQGIRNYLAWYQQKPGKAPKLLIYAAS
TLQSGVPSRFSGSGSGTDFTLTISSLQPEDVATY
YCQRYNRAPYTFGQGTKVEIKRTVAAPSVFIFPP
SDEQLKSGTASVVCLLNNFYPREAKVQWKVDNA
LQSGNSQESVTEQDSKDSTYSLSSTLTLSKADYE
KHKVYACEVTHQGLSSPVTKSFNRGEC

**FIG 8**

Okapi-
BM25
encoding

## Protein name: OTS1

**Titer:** 2.19788085106383

**Encoded Sequence:**
-3.309822, -3.309822, 0.0, 0.0, 0.8520984, -2.2096734, -2.2096734, 0.0, -4.4068627, 0.0, -4.719734, -2.2096734, -1.5303091, 0.8520984, 0.0, 0.0, -2.2096734, 0.0, -3.309822, 0.0, -2.2096734, -2.2096734, 0.0, 0.0, 0.0, -3.309822, -2.2096734, -2.2096734, -0.8520984, -2.2096734, 0.0, -0.8520984, -3.309822, -2.2096734, -0.8520984, 0.0, -2.2096734, 0.0, 0.0, 0.0, 0.0, 0.0, 0.0, 0.0, -3.309822, 0.0, 0.0, -3.309822, -3.309822, 0.0, 0.0, -2.2096734, -2.2096734, -2.2096734, 0.0, 0.0, 0.0, -4.4068627, -2.2096734, 0.0, -0.8520984, 0.0, 0.0, -2.2096734, -2.2096734, -0.8520984, -3.309822, 0.8520984, 0.8520984, -1.5303091, -4.4068627, 0.0, -2.2096734, -2.2096734, 0.0, -3.9684186, -4.719734, -2.2096734, 0.0, 0.0, -2.2096734, 0.0, -2.2096734, 0.0, 0.0, -3.309822, 0.0, 0.0, 0.0, 0.0, 0.0, 0.8520984, 0.0, 0.0, -2.2096734, -2.2096734, -3.309822, 0.0, -2.2096734, -3.9684186, 0.0, -2.2096734, 0.0, 0.0, -3.309822, -2.2096734, 0.0, 0.0, 0.0, -3.309822, -3.309822, -3.9684186, 0.0, -4.719734, 0.0, 0.0, 0.0, 0.0, 0.0, -2.2096734, -2.2096734, -4.954222, 0.0, -3.309822, -2.2096734, -4.4068627, -1.2763398, -3.9684186, -3.9684186, -3.9684186, 0.0, -2.2096734, 0.0, 0.0, 0.0, 0.0, 0.0, -1.5303091, -3.9684186, -2.2096734, -2.2096734, -2.2096734, 0.0, 0.0, 0.0, 0.0, 0.0, -3.309822, -3.9684186, -2.2096734, 0.0, -2.2096734, -0.8520984, -2.2096734, 0.0, -3.309822, -3.309822, 0.0, 0.0, -2.2096734, 0.0, -3.309822, 0.0, 0.0, -4.954222, 0.0, -3.309822, -2.2096734, -2.2096734, -2.2096734, -3.309822, -2.2096734, 0.0, -1.2763398, 0.0, -2.2096734, 0.0, -2.2096734, 0.0, -0.8520984, 0.0, -3.9684186, 0.0, -1.2763398, 0.8520984, 0.0, -2.2096734, 0.0, 0.8520984, -2.2096734, -2.2096734, -2.2096734, 0.8520984, -2.2096734, -2.2096734, -2.2096734, -2.2096734, -4.4068627, 0.0, 0.0, -4.4068627, -5.136503, -2.2096734, 0.0, -2.2096734, -4.719734, -5.136503, -3.9684186, -5.136503, 0.0, -0.8520984, -2.2096734, -3.9684186, -2.2096734, -2.2096734, 0.0, -3.309822, -3.9684186, -4.719734, 0.0, 0.0, 0.0, 0.0, -2.2096734, 0.0, -3.9684186, 0.8520984, -2.2096734, -2.2096734, -3.9684186, -3.9684186, -3.309822, 0.0, -2.2096734, -2.2096734, -2.2096734, 0.0, 0.0, -3.309822, -3.9684186, -3.9684186, -3.9684186, -3.9684186, 0.0, -2.2096734, 0.0, 0.0, 0.0, 0.0, -2.2096734, 0.0, -0.8520984, 0.0, 0.0, 0.0, -2.2096734, 0.0, -3.9684186, 0.0, -2.2096734, 0.0, 0.0, -0.8520984, 0.0, -1.2763398, 0.0, 0.0, -2.2096734, -2.2096734, 0.0, -2.2096734, -3.309822, 0.0, 0.8520984, -3.309822, 2.19788085106383

EP 4 310 848 A1

FIG 9

275 Features

| Protein Name | Titer | Encoded sequences | | | | | | | | | | |
|---|---|---|---|---|---|---|---|---|---|---|---|---|
| OST1 | 2.198 | -3.30982 | -3.30982 | 0 | 0 | 0.852098 | ... | -2.20967 | -3.30982 | 0 | 0.852098 | -3.30982 |
| OST2 | 3.169 | -3.27852 | -2.18186 | 0 | 0 | 0 | ... | -3.93836 | -3.27852 | 0 | 0 | -2.18186 |
| ⋮ | ⋮ | ⋮ | ⋮ | ⋮ | ⋮ | ⋮ | ⋮ | ⋮ | ⋮ | ⋮ | ⋮ | ⋮ |
| OST4 | 2.371 | -2.20655 | -3.96506 | 0 | 0 | 0 | ... | -2.20655 | -3.30631 | 0 | 0 | -3.96506 |

70 sequences

Fast ICA

4 Features

| Protein Name | Titer | Scores | | | |
|---|---|---|---|---|---|
| OST1 | 2.198 | -0.422 | -0.50391 | -0.51709 | 0.48877 |
| OST2 | 3.169 | -0.391 | 0.496582 | 0.48291 | 0.484863 |
| ⋮ | ⋮ | ⋮ | ⋮ | ⋮ | ⋮ |
| OST4 | 2.371 | -0.51563 | 0.494995 | -0.51782 | -0.51221 |

70 sequences

**FIG 10**

| Protein Name | Titer | Scores | | | |
|---|---|---|---|---|---|
| OST1 | 2.198 | -0.422 | -0.50391 | -0.51709 | 0.48877 |
| OST2 | 3.169 | -0.391 | 0.496582 | 0.48291 | 0.484863 |
| ⋮ | ⋮ | ⋮ | ⋮ | ⋮ | ⋮ |
| OST4 | 2.371 | -0.51563 | 0.494995 | -0.51782 | -0.51221 |

Titer = f(Scores)

Estimation of the function f using
Random Forest

Prediction & Cross validation

FIG 11

EP 4 310 848 A1

FIG 12

FIG 13

Europäisches
Patentamt

European
Patent Office

Office européen
des brevets

## EUROPEAN SEARCH REPORT

**Application Number**

EP 22 29 0048

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/050923 A1 (UNIV CHICAGO [US]) 18 March 2021 (2021-03-18) * the whole document * * in particular * * paragraph [0054] – paragraph [0193]; figures 3, 4, 7 * ----- | 1-15 | INV. G16B25/10 G16B35/20 G16B40/30 |

TECHNICAL FIELDS
SEARCHED        (IPC)

G16B

The present search report has been drawn up for all claims

| Place of search | Date of completion of the search | Examiner |
|---|---|---|
| Munich | 20 December 2022 | Rákossy, Z |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 22 29 0048

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2022

| Patent document cited in search report | | Publication date | Patent family member(s) | | Publication date |
|---|---|---|---|---|---|
| WO 2021050923 | A1 | 18-03-2021 | AU 2020344624 | A1 | 31-03-2022 |
| | | | BR 112022004539 | A2 | 31-05-2022 |
| | | | CA 3149211 | A1 | 18-03-2021 |
| | | | CN 114651064 | A | 21-06-2022 |
| | | | EP 4004200 | A1 | 01-06-2022 |
| | | | JP 2022548841 | A | 22-11-2022 |
| | | | US 2022348903 | A1 | 03-11-2022 |
| | | | WO 2021050923 | A1 | 18-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LAI et al.** Advances in Mammalian Cell Line Development Technologies for Recombinant Protein Production. *Pharmaceuticals,* 2013, vol. 6 (5), 579-603
- **KENNETH WARD CHURCH.** *Word2vec. Natural Language Engineering,* 2017, vol. 23 (1), 155-162
- **ZHEN CHEN ; PEI ZHAO ; FUYI LI ; TATIANA T MARQUEZ-LAGO ; ANDRE' LEIER ; JERICO REVOTE ; YAN ZHU ; DAVID R POWELL ; TATSUYA AKUTSU ; GEOFFREY I WEBB et al.** ilearn: an integrated platform and meta-learner for feature engineering, machine-learning analysis and modeling of dna, rna and protein sequence data. *Briefings in bioinformatics,* 2020, vol. 21 (3), 1047-1057
- **CHUN-WEI TUNG ; SHINN-YING HO.** Computational identification of ubiquitylation sites from protein sequences. *BMC bioinformatics,* 2008, vol. 9 (1), 1-15
- **MICHAL BUSTA ; LUKAS NEUMANN ; JIRI MATAS.** Fastext: Efficient unconstrained scene text detector. *Proceedings of the IEEE International Conference on Computer Vision,* 2015, 1206-1214
- Fastext: Fast and small text extractor. **ALEXANDER FILONENKO ; KONSTANTIN GUDKOV ; ALEKSEI LEBEDEV ; IVAN ZAGAYNOV ; NIKITA ORLOV.** 2019 International Conference on Document Analysis and Recognition Workshops (ICDARW). IEEE, 2019, vol. 4, 49-54
- **RAFSANJANI MUHAMMOD ; SAJID AHMED ; DEWAN MD FARID ; SWAKKHAR SHATABDA ; ALOK SHARMA ; ABDOLLAH DEHZANGI.** Pyfeat: a python-based effective feature generation tool for dna, rna and protein sequences. *Bioinformatics,* 2019, vol. 35 (19), 3831-3833
- **CZ CAI ; LY HAN ; ZHI LIANG ; JI, X CHEN ; YU ZONG CHEN.** Svm-prot: web-based support vector machine software for functional classification of a protein from its primary sequence. *Nucleic acids research,* 2003, vol. 31 (13), 3692-3697
- **CZ CAI ; LY HAN ; ZL JI ; YZ CHEN.** Enzyme family classification by support vector machines. *Proteins: Structure, Function, and Bioinformatics,* 2004, vol. 55 (1), 66-76
- **INNA DUBCHAK ; ILYA MUCHNIK ; STEPHEN R HOLBROOK ; SUNG-HOU KIM.** Prediction of protein folding class using global description of amino acid sequence. *Proceedings of the National Academy of Sciences,* 1995, vol. 92 (19), 8700-8704

- **INNA DUBCHAK ; ILYA MUCHNIK ; CHRISTOPHER MAYOR ; IGOR DRALYUK ; SUNG-HOU KIM.** Recognition of a protein fold in the context of the scop classification. *Proteins: Structure, Function, and Bioinformatics,* 1999, vol. 35 (4), 401-407
- **LIAN YI HAN ; CONG ZHONG CAI ; SIEW LIN LO ; MAXEY CM CHUNG ; YU ZONG CHEN.** Prediction of rna-binding proteins from primary sequence by a support vector machine approach. *Rna,* 2004, vol. 10 (3), 355-368
- **BRYAN PEROZZI ; RAMI AI-RFOU ; STEVEN SKIENA.** Deepwalk: Online learning of social representations. *Proceedings of the 20th ACM SIGKDD international conference on Knowledge discovery and data mining,* 2014, 701-710
- **THOMAS N KIPF ; MAX WELLING.** Variational graph auto-encoders. *arXiv:1611.07308,* 2016
- **PALASH GOYAL ; EMILIO FERRARA.** Graph embedding techniques, applications, and performance: A survey. *Knowledge-Based Systems,* 2018, vol. 151, 78-94
- **KUO-CHEN CHOU.** Prediction of protein subcellular locations by incorporating quasi-sequence-order effect. *Biochemical and biophysical research communications,* 2000, vol. 278 (2), 477-483
- **KUO-CHEN CHOU ; YU-DONG CAI.** Prediction of protein subcellular locations by go-fund-pseaa predictor. *Biochemical and Biophysical Research Communications,* 2004, vol. 320 (4), 1236-1239
- **GISBERT SCHNEIDER ; PAUL WREDE.** The rational design of amino acid sequences by artificial neural networks and simulated molecular evolution: de novo design of an idealized leader peptidase cleavage site. *Biophysical Journal,* 1994, vol. 66 (2
- **SHAOSHENG CAO ; WEI LU ; QIONGKAI XU.** Grarep: Learning graph representations with global structural information. *Proceedings of the 24th ACM international on conference on information and knowledge management,* 2015, 891-900
- **JIAN TANG ; MENG QU ; MINGZHE WANG ; MING ZHANG ; JUN YAN ; QIAOZHU MEI.** Line: Large-scale information network embedding. *Proceedings of the 24th international conference on world wide web,* 2015, 1067-1077

- **KUO-CHEN CHOU.** Prediction of protein cellular attributes using pseudo-amino acid composition. *Proteins: Structure, Function, and Bioinformatics,* 2001, vol. 43 (3), 246-255
- **DINGYUAN ZHU ; PENG CUI ; ZIWEI ZHANG ; JIAN PEI ; WENWU ZHU.** High-order proximity preserved embedding for dynamic networks. *IEEE Transactions on Knowledge and Data Engineering,* 2018, vol. 30 (11), 2134-2144
- **KUO-CHEN CHOU.** Using amphiphilic pseudo amino acid composition to predict enzyme subfamily classes. *Bioinformatics,* 2005, vol. 21 (1), 10-19
- **RUSSELL MERRIS.** Laplacian matrices of graphs: a survey. *Linear algebra and its applications,* 1994, vol. 197, 143-176
- **TZONG-YI LEE ; SHU-AN CHEN ; HSIN-YI HUNG ; YU-YEN OU.** Incorporating distant sequence features and radial basis function networks to identify ubiquitin conjugation sites. *PloS one,* 2011, vol. 6 (3), e17331
- **DAIXIN WANG ; PENG CUI ; WENWU ZHU.** Structural deep network embedding. *Proceedings of the 22nd ACM SIGKDD international conference on Knowledge discovery and data mining,* 2016, 1225-1234
- **YONG-ZI CHEN ; ZHEN CHEN ; YU-AI GONG ; GUOGUANG YING.** Sumohydro: a novel method for the prediction of sumoylation sites based on hydrophobic properties. *PloS one,* 2012, vol. 7 (6), e39195
- **HERVÉ ABDI.** Singular value decomposition (svd) and generalized singular value decomposition. *Encyclopedia of measurement and statistics,* 2007, 907-912
- **WILLIAM STAFFORD NOBLE ; SCOTT KUEHN ; ROBERT THURMAN ; MAN YU ; JOHN STAMATOYANNOPOULOS.** Predicting the in vivo signature of human gene regulatory sequences. *Bioinformatics,* 2005, vol. 21 (1), i338-i343
- **SHOBHIT GUPTA ; JONATHAN DENNIS ; ROBERT E THURMAN ; ROBERT KINGSTON ; JOHN A STAMATOYANNOPOULOS ; WILLIAM STAFFORD NOBLE.** Predicting human nucleosome occupancy from primary sequence. *PLoS computational biology,* 2008, vol. 4 (8), e1000134
- **ZHI-PING FENG ; CHUN-TING ZHANG.** Prediction of membrane protein types based on the hydrophobic index of amino acids. *Journal of protein chemistry,* 2000, vol. 19 (4), 269-275
- **ZONG LIN ; XIAN-MING PAN.** Accurate prediction of protein secondary structural content. *Journal of Protein Chemistry,* 2001, vol. 20 (3), 217-220
- **ADITYA GROVER ; JURE LESKOVEC.** node2vec: Scalable feature learning for networks. *Proceedings of the 22nd ACM SIGKDD international conference on Knowledge discovery and data mining,* 2016, 855-864
- **CHAO ZHOU ; CHANGSHI WANG ; HONGBO LIU ; QIANGWEI ZHOU ; QIAN LIU ; YAN GUO ; TING PENG ; JIAMING SONG ; JIANWEI ZHANG ; LINGLING CHEN et al.** Identification and analysis of adenine n6-methylation sites in the rice genome. *Nature plants,* 2018, vol. 4 (8), 554-563
- **ROBERT R SOKAL ; BARBARA A THOMSON.** Population structure inferred by local spatial autocorrelation: an example from an amerindian tribal population. *American Journal of Physical Anthropology: The Official Publication of the American Association of Physical Anthropologists,* 2006, vol. 129 (1), 121-131
- **JUAN RAMOS et al.** Using tf-idf to determine word relevance in document queries. *Proceedings of the first instructional conference on machine learning,* 2003, vol. 242, 29-48
- Automated document classification for news article in bahasa Indonesia based on term frequency inverse document frequency (tf-idf) approach. **ARI AULIA HAKIM ; ALVA ERWIN ; KHO I ENG ; MAULAHIKMAH GALINIUM ; WAHYU MULIADY.** 2014 6th international conference on information technology and electrical engineering (ICITEE). IEEE, 2014, 1-4
- **DAVID S HOME.** Prediction of protein helix content from an autocorrelation analysis of sequence hydrophobicities. *Biopolymers: Original Research on Biomolecules,* 1988, vol. 27 (3), 451-477
- **BIN LIU ; FULE LIU ; LONGYUN FANG ; XIAOLONG WANG ; KUO-CHEN CHOU.** repdna: a python package to generate various modes of feature vectors for dna sequences by incorporating user-defined physicochemical properties and sequence-order effects. *Bioinformatics,* 2015, vol. 31 (8), 1307-1309
- **QIWEN DONG ; SHUIGENG ZHOU ; JIHONG GUAN.** A new taxonomy-based protein fold recognition approach based on autocross-covariance transformation. *Bioinformatics,* 2009, vol. 25 (20), 2655-2662
- **YANZHI GUO ; LEZHENG YU ; ZHINING WEN ; MENGLONG LI.** Using support vector machine combined with auto covariance to predict protein-protein interactions from protein sequences. *Nucleic acids research,* 2008, vol. 36 (9), 3025-3030
- **JOHN S WHISSELL ; CHARLES LA CLARKE.** Improving document clustering using okapi bm25 feature weighting. *Information retrieval,* 2011, vol. 14 (5), 466-487
- **MENG KONG ; YUSEN ZHANG ; DA XU ; WEI CHEN ; MATTHIAS DEHMER.** Fctp-wsrc: protein-protein interactions prediction via weighted sparse representation based classification. *Frontiers in genetics,* 2020, vol. 11, 18

- **JUWEN SHEN ; JIAN ZHANG ; XIAOMIN LUO ; WEILIANG ZHU ; KUNQIAN YU ; KAIXIAN CHEN ; YIXUE LI ; HUALIANG JIANG.** Predicting protein-protein interactions based only on sequences information. *Proceedings of the National Academy of Sciences,* 2007, vol. 104 (11), 4337-4341

- **YONGCHUN ZUO ; YUAN LI ; YINGLI CHEN ; GUANGPENG LI ; ZHENHE YAN ; LEI YANG.** Psekraac: a flexible web server for generating pseudo k-tuple reduced amino acids composition. *Bioinformatics,* 2017, vol. 33 (1), 122-124
- **WEI CHEN ; HONG TRAN ; ZHIYONG LIANG ; HAO LIN ; LIQING ZHANG.** Identification and analysis of the n6-methyladenosine in the saccharomyces cerevisiae transcriptome. *Scientific reports,* 2015, vol. 5 (1), 1-8